# EUROPEAN PATENT SPECIFICATION

(11) **EP 3 784 282 B1**
(45) Date of publication and mention of the grant of the patent: **02.07.2025**
(21) Application number: 19720551.1
(22) Date of filing: 26.04.2019
(51) Int. Cl.: A61K 39/395, A61K 40/00, A61P 43/00

(54) **COMBINATION TREATMENT FOR EYE FIBROSIS AND/OR ANGIOGENESIS**
KOMBINATIONSBEHANDLUNG FÜR AUGENFIBROSE UND / ODER ANGIOGENESE
TRAITEMENT D'ASSOCIATION CIBLANT LA FIBROSE ET/OU L'ANGIOGENÈSE OCULAIRES

(30) Priority: 27.04.2018 GB 201806918
(43) Date of publication of application: 03.03.2021
(73) Proprietor: Boehringer Ingelheim International GmbH, 55216 Ingelheim am Rhein (DE)
(72) Inventor: COOK, Stuart Alexander, Singapore 068914 (SG); SCHAEFER, Sebastian, Singapore 068914 (SG)
(74) Representative: Mewburn Ellis LLP
(86) International application number: PCT/EP2019/060772
(87) International publication number: WO 2019/207122

(56) References cited:
- WO-A1-02/07747
- WO-A1-2017/103108
- WO-A1-2017/141032
- US-A1- 2014 193 402
- SEBASTIAN SCHAFER ET AL: "IL11 is a crucial determinant of cardiovascular fibrosis", NATURE, vol. 552, 13 November 2017 (2017-11-13), London, pages 110 - 133, XP055472508, ISSN: 0028-0836, DOI: 10.1038/nature24676

## Description

### Field of the Invention

The present invention relates to the treatment and prophylaxis of fibrosis and/or angiogenesis (in particular in the eye), through antagonism of IL-11 mediated signalling and antagonism of angiogenesis.

### Background to the Invention

Angiogenesis is an essential process that is a key part of the wound healing process. Abnormal angiogenesis frequently results in severe conditions and complications; most diseases that cause catastrophic loss of vision do so as a result of abnormal angiogenesis and wound healing, often in response to tissue ischemia, inflammation or metabolic perturbation.

Anti-angiogenic therapies, such as anti-VEGF therapies, are a growing group of medicines that can reduce new vessel formation under the macula of the retina. Conditions treated by these agents include age-related macular degeneration, diabetic eye disease and some cancers.

Fibrosis is an essential process that is a critical part of wound healing. Excessive fibrosis is common in many rare and common disease conditions and is important in disease pathogenesis. Diseases characterized by excessive fibrosis include but are not restricted to: systemic sclerosis, scleroderma, hypertrophic cardiomyopathy, dilated cardiomyopathy (DCM), atrial fibrillation, ventricular fibrillation, myocarditis, liver cirrhosis, kidney diseases, asthma, diseases of the eye, cystic fibrosis, arthritis and idiopathic pulmonary fibrosis. Despite the large impact on human health, therapeutic and diagnostic approaches to fibrosis are still an unmet medical need.

US 2014/193402 A1 relates to anti-PDGFR antibodies and their use in the treatment of disease. WO 2017/141032 A1 describes the treatment of fibrotic disorders via inhibition of PCSK. Schafer et al., Nature (2017) 552:110-133 relates to the role of IL-11 in fibrosis. WO 2017/103108 A1 discloses the treatment of fibrosis through administering an agent capable of inhibiting the action of IL-11. WO 02/07747 A1 relates to methods for modulating fibrosis and/or angiogenesis by inhibiting components of the VEGF signalling pathway.

### Summary of the Invention

The invention is as defined in the appended claims.

Disclosed herein is a method of treating or preventing fibrosis in a subject, the method comprising administering to a subject a therapeutically or prophylactically effective amount of an antagonist of IL-11 mediated signalling and an antagonist of an angiogenic factor.

Also disclosed is a combination of an antagonist of IL-11 mediated signalling, and an antagonist of an angiogenic factor, for use in a method of treating of preventing fibrosis.

Disclosed herein is the use of an antagonist of IL-11 mediated signalling and an antagonist of an angiogenic factor in the manufacture of a medicament for use in a method of treating or preventing fibrosis.

In any of the aspects disclosed herein, the fibrosis is preferably fibrosis in the eye.

In some embodiments, the fibrosis is selected from Grave's opthalmopathy, epiretinal fibrosis, retinal fibrosis, idiopathic premacular fibrosis, subretinal fibrosis (e.g. associated with retinal detachment or macular degeneration (e.g. wet age-related macular degeneration (AMD)), diabetic retinopathy, glaucoma, geographic atrophy, corneal fibrosis, post-surgical fibrosis (e.g. of the posterior capsule following cataract surgery, or of the bleb following trabeculectomy for glaucoma), conjunctival fibrosis, or subconjunctival fibrosis.

In some embodiments, the fibrosis is retinal fibrosis. In some embodiments, the fibrosis is epiretinal fibrosis. In some embodiments, the fibrosis is subretinal fibrosis.

In some embodiments of the aspects disclosed herein, the fibrosis is fibrosis of the heart, liver, or kidney. In some embodiments, the fibrosis is in the liver and is associated with chronic liver disease or liver cirrhosis. In some embodiments, the fibrosis is in the kidney and is associated with chronic kidney disease. In some embodiments, the fibrosis is in the heart and is associated with dysfunction of the musculature or electrical properties of the heart, or thickening of the walls or valves of the heart.

In some examples disclosed herein, the antagonist of an angiogenic factor is an antagonist of an angiogenic factor selected from vascular endothelial growth factor (VEGF), a fibroblast growth factor (FGF), and platelet-derived growth factor (PDGF).

The angiogenic factor is VEGF. In some embodiments, the VEGF is one or more of VEGF-A, VEGF-B, VEGF-C and/or VEGF-D. In some embodiments, the VEGF is VEGF-A.

In some examples, the antagonist of IL-11 mediated signalling is an agent capable of preventing or reducing the binding of IL-11 to an IL-11 receptor. In some examples, the antagonist of IL-11 mediated signalling is an agent capable of preventing or reducing the binding of IL-11:IL11Rα complex to gp130. In some examples, the antagonist of IL-11 mediated signalling is an agent capable of preventing or reducing the binding of IL-11:IL11Rα *trans*-signalling complex to gp130. In some examples, the antagonist of IL-11 mediated signalling is an agent capable of preventing or reducing the binding of IL11Rα to gp130. In some examples, the antagonist of IL-11 mediated signalling is an agent capable of preventing or reducing the binding of IL-11 to IL-11.

In some examples, the antagonist of IL-11 mediated signalling is an agent capable of binding to IL-11 or a receptor for IL-11. In some examples, the antagonist of IL-11 mediated signalling is selected from the group consisting of: an antibody or an antigen-binding fragment thereof, a polypeptide, a peptide, an oligonucleotide, an aptamer or a small molecule. The antagonist of IL-11 mediated signalling is an anti-IL-11 antibody or an anti-IL-11Rα antibody. In some examples, the antagonist of IL-11 mediated signalling is a decoy IL-11 receptor.

In some examples, the antagonist of IL-11 mediated signalling is capable of reducing the expression of IL-11 or a receptor for IL-11. In some examples, the antagonist of IL-11 mediated signalling is an oligonucleotide or a small molecule.

In some examples the antagonist of an angiogenic factor is an agent capable of preventing or reducing the binding of angiogenic factor to an interaction partner for an angiogenic factor. In some examples the antagonist of an angiogenic factor is an agent capable of binding to an angiogenic factor or an interaction partner for an angiogenic factor.

In some examples, the antagonist of an angiogenic factor is an agent capable of preventing or reducing the binding of angiogenic factor to a receptor for an angiogenic factor.

In some examples, the antagonist of an angiogenic factor is an agent capable of binding to an angiogenic factor or a receptor for an angiogenic factor. In some examples, the antagonist of an angiogenic factor is selected from the group consisting of: an antibody or an antigen-binding fragment thereof, a polypeptide, a peptide, an oligonucleotide, an aptamer or a small molecule. In some examples, the antagonist of an angiogenic factor is an anti-angiogenic factor antibody or an anti-angiogenic factor receptor antibody. In some examples the antagonist of an angiogenic factor is an anti-VEGF antibody or an anti-VEGF receptor antibody. In some examples, the antagonist of an angiogenic factor is a decoy angiogenic factor receptor. The antagonist of an angiogenic factor is a decoy VEGF receptor. In some embodiments, the antagonist of an angiogenic factor is aflibercept (SEQ ID NO:24).

In some examples, the antagonist of an angiogenic factor is capable of reducing the expression of an angiogenic factor or a receptor for an angiogenic factor. In some examples, the antagonist of an angiogenic factor is an oligonucleotide or a small molecule.

In some examples of the methods, the combinations for use, or the uses described here, the method of treating or preventing fibrosis comprises administering an antagonist of IL-11 mediated signalling and an antagonist of an angiogenic factor to a subject in whom expression of IL-11 or a receptor for IL-11 has been determined to be upregulated. In some examples, the expression of an angiogenic factor or a receptor for an angiogenic factor has been determined to be upregulated.

In some examples of the methods, the combinations for use, or the uses described here, the method of treating or preventing comprises determining whether expression of IL-11 or a receptor for IL-11 is upregulated in the subject and administering an antagonist of IL-11 mediated signalling and an antagonist of an angiogenic factor to a subject in which expression of IL-11 or a receptor for IL-11 is upregulated.

Disclosed herein is a method of determining the suitability of a subject for the treatment or prevention of fibrosis with an antagonist of IL-11 mediated signalling and an antagonist of an angiogenic factor. In some examples, the method comprises determining, optionally *in vitro,* whether expression of an Interleukin 11 (IL-11), an Interleukin 11 receptor (IL-11R), an angiogenic factor, or an angiogenic factor receptor is upregulated in the subject. In some examples, the method comprises determining, optionally *in vitro,* whether expression of an angiogenic factor or the receptor of an angiogenic factor is upregulated in the subject.

Also disclosed herein is a method of selecting a subject for the treatment or prevention of fibrosis with an antagonist of IL-11 mediated signalling and an antagonist of an angiogenic factor. In some examples, the method comprises, optionally *in vitro,* whether expression of Interleukin 11 (IL-11), an Interleukin 11 receptor (IL-11R), an angiogenic factor, or an angiogenic factor receptor is upregulated in the subject. In some examples, the method comprises determining, optionally *in vitro,* whether expression of an angiogenic factor or the receptor of an angiogenic factor is upregulated in the subject.

Also disclosed herein is a method of diagnosing fibrosis or a risk of developing fibrosis in a subject. In some examples, the method comprises, optionally *in vitro,* the upregulation of Interleukin 11 (IL-11), an Interleukin 11 receptor (IL-11R), an angiogenic factor, or an angiogenic factor receptor in a sample obtained from the subject. In some examples, the method comprises determining, optionally *in vitro,* whether expression of an angiogenic factor or the receptor of an angiogenic factor is upregulated in the subject. In some examples, the method comprises selecting the subject for treatment with an antagonist of IL-11 mediated signalling and an antagonist of an angiogenic factor.

Also disclosed herein is a method of providing a prognosis for a subject having, or suspected of having fibrosis, and, based on the determination, providing a prognosis for treatment of the subject with an antagonist of IL-11 mediated signalling and an antagonist of an angiogenic factor. In some examples, the method comprising determining, optionally *in vitro,* whether Interleukin 11 (IL-11), an Interleukin 11 receptor (IL-11R), an angiogenic factor, or an angiogenic factor receptor is upregulated in a sample obtained from the subject. In some examples, the method comprises selecting a subject determined to have upregulated expression of Interleukin 11 (IL-11), an Interleukin 11 receptor (IL-11R), an angiogenic factor, or an angiogenic factor receptor for treatment with an antagonist of IL-11 mediated signalling and an antagonist of an angiogenic factor.

Also disclosed herein is a method of diagnosing fibrosis or a risk of developing fibrosis in a subject, the method comprising determining, optionally *in vitro,* one or more genetic factors in the subject and selecting the subject for treatment with an antagonist of IL-11 mediated signalling and an antagonist of an angiogenic factor. In some examples, the one or more genetic factors are predictive of upregulation of Interleukin 11 (IL-11) or an Interleukin 11 receptor (IL-11R) expression, or of upregulation of IL-11 or IL-11R activity. In some examples, the one or more genetic factors are predictive of upregulation of an angiogenic factor or an angiogenic factor receptor expression, or of upregulation of an angiogenic factor or angiogenic factor receptor activity. In some examples, the one or more genetic factors are predictive of upregulation of expression of an angiogenic factor or an angiogenic factor receptor expression, or of upregulation of angiogenic factor or angiogenic factor receptor activity.

The antagonist of IL-11 mediated signalling is an anti-IL-11 antibody and the antagonist of an angiogenic factor is a VEGF decoy receptor. In a preferred embodiment, the VEGF decoy receptor is aflibercept.

Also disclosed herein is a combination comprising an antagonist of IL-11 mediated signalling and an antagonist of an angiogenic factor. Also provided in the disclosure is a pharmaceutical composition comprising an antagonist of IL-11 mediated signalling and an antagonist of an angiogenic factor. Also provided in the disclosure is a kit of parts comprising a predetermined quantity of an antagonist of IL-11 mediated signalling and an antagonist of an angiogenic factor, a combination as described herein, or a pharmaceutical composition as described herein.

In some examples the angiogenic factor is selected from vascular endothelial growth factor (VEGF), a fibroblast growth factor (FGF), and platelet-derived growth factor (PDGF). In some examples the antagonist of IL-11 mediated signalling is an agent capable of preventing or reducing the binding of IL-11 to an IL-11 receptor, optionally wherein the antagonist of IL-11 mediated signalling is an agent capable of binding to IL-11 or a receptor for IL-11, e.g. as described herein. In some examples the antagonist of IL-11 mediated signalling is capable of reducing the expression of IL-11 or a receptor for IL-11, and is e.g. an agent as described herein. In some examples, the antagonist of an angiogenic factor is an agent capable of preventing or reducing the binding of angiogenic factor to an interaction partner for an angiogenic factor. In some examples, the antagonist of an angiogenic factor may be an agent capable of binding to an angiogenic factor or an interaction partner for an angiogenic factor, e.g. as described herein. The antagonist of an angiogenic factor may be aflibercept. In some examples the antagonist of an angiogenic factor may be capable of reducing the expression of an interaction partner for an angiogenic factor, e.g. an antagonist described herein.

### Description

The present invention is based on the unexpected finding that combination therapy with an antagonist of an angiogenic factor and an antagonist of IL-11 mediated signalling provides excellent therapeutic effects in the treatment of fibrosis, and in particular fibrosis in the eye. In addition, demonstrated herein are the unexpected findings that antagonists of IL-11 mediated signalling are able to treat fibrosis in the eye and that antagonism of IL-11 mediated signalling is able to improve the efficacy of antagonism of angiogenesis in a combination treatment. Data also show the surprising treatment effect of antagonism of IL-11 mediated signalling on choroidal neovascularisation (CNV).

### Interleukin 11 and receptors for IL-11

Interleukin 11 (IL-11), also known as adipogenesis inhibitory factor, is a pleiotropic cytokine and a member of the IL-6 family of cytokines that includes IL-6, IL-11, IL-27, IL-31, oncostatin, leukemia inhibitory factor (LIF), cardiotrophin-1 (CT-1), cardiotrophin-like cytokine (CLC), ciliary neurotrophic factor (CNTF) and neuropoetin (NP-1).

Interleukin 11 (IL-11) is expressed in a variety of mesenchymal cell types¹. IL-11 genomic sequences have been mapped onto chromosome 19 and the centromeric region of chromosome 7¹, and is transcribed with a canonical signal peptide that ensures efficient secretion from cells. The activator protein complex of IL-11, cJun/AP-1, located within its promoter sequence is critical for basal transcriptional regulation of IL-11¹. The immature form of human IL-11 is a 199 amino acid polypeptide whereas the mature form of IL-11 encodes a protein of 178 amino acid residues (Garbers and Scheller., Biol. Chem. 2013; 394(9):1145-1161). The human IL-11 amino acid sequence is available under UniProt accession no. P20809 (P20809.1 GI:124294; SEQ ID NO:1). Recombinant human IL-11 (oprelvekin) is also commercially available. IL-11 from other species, including mouse, rat, pig, cow, several species of bony fish and primates, have also been cloned and sequenced.

In this specification "IL-11" refers to an IL-11 from any species and includes isoforms, fragments, variants or homologues of an IL-11 from any species. In preferred embodiments the species is human (Homo sapiens). Isoforms, fragments, variants or homologues of an IL-11 may optionally be characterised as having at least 70%, preferably one of 80%, 85%, 90%, 91%, 92%, 93%, 94%, 95%, 96%, 97%, 98%, 99% or 100% amino acid sequence identity to the amino acid sequence of immature or mature IL-11 from a given species, e.g. human. Isoforms, fragments, variants or homologues of an IL-11 may optionally be characterised by ability to bind IL-11Rα (preferably from the same species) and stimulate signal transduction in cells expressing IL-11Rα and gp130 (e.g. as described in Curtis et al. Blood, 1997, 90(11); or Karpovich et al. Mol. Hum. Reprod. 2003 9(2): 75-80). A fragment of IL-11 may be of any length (by number of amino acids), although may optionally be at least 25% of the length of mature IL-11 and may have a maximum length of one of 50%, 75%, 80%, 85%, 90%, 91%, 92%, 93%, 94%, 95%, 96%, 97%, 98%, or 99% of the length of mature IL-11. A fragment of IL-11 may have a minimum length of 10 amino acids, and a maximum length of one of 15, 20, 25, 30, 40, 50, 100, 110, 120, 130, 140, 150, 160, 170, 180, 190 or 195 amino acids.

IL-11 signals through a homodimer of the ubiquitously expressed glycoprotein 130 (gp130; also known as glycoprotein 130, IL-6ST, IL-6-beta or CD130). Gp130 is a transmembrane protein that forms one subunit of the type I cytokine receptor with the IL-6 receptor family. Specificity is gained through an individual interleukin 11 receptor subunit alpha (IL-11Rα), which does not directly participate in signal transduction, although the initial cytokine binding event to the α-receptor leads to the final complex formation with gp130.

Human gp130 (including the 22 amino acid signal peptide) is a 918 amino acid protein, and the mature form is 866 amino acids, comprising a 597 amino acid extracellular domain, a 22 amino acid transmembrane domain, and a 277 amino acid intracellular domain. The extracellular domain of the protein comprises the cytokine-binding module (CBM) of gp130. The CBM of gp130 comprises the Ig-like domain D1, and the fibronectin-type III domains D2 and D3 of gp130. The amino acid sequence of human gp130 is available under UniProt accession no. P40189-1 (SEQ ID NO:2).

Human IL-11Rα is a 422 amino acid polypeptide (UniProt Q14626; SEQ ID NO:3) and shares ~85% nucleotide and amino acid sequence identity with the murine IL-11Rα (Du and Williams., Blood Vol, 89, No,11, June 1, 1997). Two isoforms of IL-11Rα have been reported, which differ in the cytoplasmic domain (Du and Williams, supra). The IL-11 receptor α-chain (IL-11Rα) shares many structural and functional similarities with the IL-6 receptor α-chain (IL-6Rα). The extracellular domain shows 24% amino acid identity including the characteristic conserved Trp-Ser-X-Trp-Ser (WSXWS) motif. The short cytoplasmic domain (34 amino acids) lacks the Box 1 and 2 regions that are required for activation of the JAK/STAT signalling pathway.

The receptor binding sites on murine IL-11 have been mapped and three sites - sites I, II and III - identified. Binding to gp130 is reduced by substitutions in the site II region and by substitutions in the site III region. Site III mutants show no detectable agonist activity and have IL-11Rα antagonist activity (Cytokine Inhibitors Chapter 8; edited by Gennaro Ciliberto and Rocco Savino, Marcel Dekker, Inc. 2001).

In this specification an IL-11 receptor/receptor for IL-11 (IL-11R) refers to a polypeptide or polypeptide complex capable of binding IL-11. In some embodiments an IL-11 receptor is capable of binding IL-11 and inducing signal transduction in cells expressing the receptor.

An IL-11 receptor may be from any species and includes isoforms, fragments, variants or homologues of an IL-11 receptor from any species. In preferred embodiments the species is human (Homo sapiens).

In some embodiments the IL-11 receptor (IL-11R) may be IL-11Rα. In some embodiments a receptor for IL-11 may be a polypeptide complex comprising IL-11Rα. In some embodiments the IL-11 receptor may be a polypeptide complex comprising IL-11Rα and gp130. In some embodiments the IL-11 receptor may be gp130 or a complex comprising gp130 to which IL-11 binds.

Isoforms, fragments, variants or homologues of an IL-11Rα may optionally be characterised as having at least 70%, preferably one of 80%, 85%, 90%, 91%, 92%, 93%, 94%, 95%, 96%, 97%, 98%, 99% or 100% amino acid sequence identity to the amino acid sequence of IL-11Rα from a given species, e.g. human. Isoforms, fragments, variants or homologues of an IL-11Rα may optionally be characterised by ability to bind IL-11 (preferably from the same species) and stimulate signal transduction in cells expressing the IL-11Rα and gp130 (e.g. as described in Curtis et al. Blood, 1997, 90(11) or Karpovich et al. Mol. Hum. Reprod. 2003 9(2): 75-80). A fragment of an IL-11 receptor may be of any length (by number of amino acids), although may optionally be at least 25% of the length of the mature IL-11Rα and have a maximum length of one of 50%, 75%, 80%, 85%, 90%, 91%, 92%, 93%, 94%, 95%, 96%, 97%, 98%, or 99% of the length of the mature IL-11Rα. A fragment of an IL-11 receptor fragment may have a minimum length of 10 amino acids, and a maximum length of one of 15, 20, 25, 30, 40, 50, 100, 110, 120, 130, 140, 150, 160, 170, 180, 190, 200, 250, 300, 400, or 415 amino acids.

### IL-11 signalling

IL-11 binds to IL-11Rα with low affinity (Kd ~10 nmol/L), and interaction between these binding partners alone is insufficient to transduce a biological signal. The generation of a high affinity receptor (Kd ~400 to 800 pmol/L) capable of signal transduction requires co-expression of the IL-11Rα and gp130 (Curtis et al (Blood 1997 Dec 1;90 (11):4403-12; Hilton et al., EMBO J 13:4765, 1994; Nandurkar et al., Oncogene 12:585, 1996). Binding of IL-11 to cell-surface IL-11Rα induces heterodimerization, tyrosine phosphorylation, activation of gp130 and downstream signalling, predominantly through the mitogen-activated protein kinase (MAPK)-cascade and the Janus kinase/signal transducer and activator of transcription (Jak/STAT) pathway (Garbers and Scheller, supra).

In principle, a soluble IL-11Rα can also form biologically active soluble complexes with IL-11 (Pflanz et al., 1999 FEBS Lett, 450, 117-122) raising the possibility that, similar to IL-6, IL-11 may in some instances bind soluble IL-11Rα prior to binding cell-surface gp130 (Garbers and Scheller, supra). Curtis et al (Blood 1997 Dec 1;90 (11):4403-12) describe expression of a soluble murine IL-11 receptor alpha chain (sIL-11R) and examined signalling in cells expressing gp130. In the presence of gp130 but not transmembrane IL-11R the sIL-11R mediated IL-11 dependent differentiation of M1 leukemic cells and proliferation in Ba/F3 cells and early intracellular events including phosphorylation of gp130, STAT3 and SHP2 similar to signalling through transmembrane IL-11R. Activation of signalling through cell-membrane bound gp130 by IL-11 bound to soluble IL-11Rα has recently been demonstrated (Lokau et al., 2016 Cell Reports 14, 1761-1773). This so-called IL-11 *trans* signalling may be a very important component of IL-11-mediated signalling, and may even be the most common form of IL-11-mediated signalling, because whilst the expression of IL-11Rα is restricted to a relatively small subset of cell types, gp130 is expressed on a wide range of cell types.

As used herein, 'IL-11 *trans* signalling' is used to refer to signalling which is triggered by binding of IL-11 bound to IL-11Rα, to gp130. The IL-11 may be bound to IL-11Rα as a non-covalent complex. The gp130 is membrane-bound and expressed by the cell in which signalling occurs following binding of the IL-11:IL-11Rα complex to gp130. In some embodiments the IL-11Rα may be a soluble IL-11Rα. In some embodiments, the soluble IL-11Rα is a soluble (secreted) isoform of IL-11Rα (e.g. lacking a transmembrane domain). In some embodiments, the soluble IL-11Rα is the liberated product of proteolytic cleavage of the extracellular domain of cell membrane bound IL-11 Rα. In some embodiments, the IL-11Rα may be cell membrane-bound, and signalling through gp130 may be triggered by binding of IL-11 bound to cell-membrane-bound IL-11Rα, termed "IL-11 *cis* signalling".

IL-11-mediated signalling has been shown to stimulate haematopoiesis and thrombopoiesis, stimulate osteoclast activity, stimulate neurogenesis, inhibit adipogenesis, reduce pro inflammatory cytokine expression, modulate extracellular matrix (ECM) metabolism, and mediate normal growth control of gastrointestinal epithelial cells¹.

The physiological role of Interleukin 11 (IL-11) remains unclear. IL-11 has been most strongly linked with activation of haematopoetic cells and with platelet production, but has also been suggested to be found to be pro-inflammatory as well as anti-inflammatory, pro-angiogenic and important for neoplasia. It is known that TGFβ1 or tissue injury can induce IL-11 expression (Zhu, M. et al. PLOS ONE 10, (2015); Yashiro, R. et al. J. Clin. Periodontol. 33, 165-71 (2006); Obana, M. et al. Circulation 121, 684-91 (2010); Tang, W et al. J. Biol. Chem. 273, 5506-13 (1998)).

IL-11 is an important post-transcriptional modulator of TGFβ-mediated signalling. TGFβ1 has been shown to stimulate the AP-1 promoter region of IL-11, and TGFβ-induced secretion of IL-11 has been shown to induce activation of ERK p42/44 and p38 MAP kinases in intestinal myofibroblasts (Bamba et al. Am J Physiol Gastrointest Liver Physiol. (2003)( 285(3):G529-38). MAP kinase inhibitors are able to significantly reduce TGFβ-induced IL-11 secretion, and p38 MAP kinase-mediated stabilization of mRNA has been shown to be critical for TGFβ-induced secretion of IL-11.

As used herein, "IL-11 signalling" and "IL-11-mediated signalling" refers to signalling mediated by binding of IL-11, or a fragment thereof having the function of the mature IL-11 molecule, to a receptor for IL-11.

### Antibodies and antigen-binding fragments

Agents capable of inhibiting IL-11-mediated signalling or capable of inhibiting angiogenic factor-mediated signalling are antibodies, or antigen binding fragments thereof.

An "antibody" is used herein in the broadest sense, and encompasses monoclonal antibodies, polyclonal antibodies, monospecific and multispecific antibodies (e.g., bispecific antibodies), and antibody fragments, as long as they display binding to the relevant target molecule.

In view of today's techniques in relation to monoclonal antibody technology, antibodies can be prepared to most antigens. The antigen-binding portion may be a part of an antibody (for example a Fab fragment) or a synthetic antibody fragment (for example a single chain Fv fragment [ScFv]). Monoclonal antibodies to selected antigens may be prepared by known techniques, for example those disclosed in "Monoclonal Antibodies: A manual of techniques ", H Zola (CRC Press, 1988) and in "Monoclonal Hybridoma Antibodies: Techniques and Applications ", J G R Hurrell (CRC Press, 1982). Chimaeric antibodies are discussed by Neuberger et al (1988, 8th International Biotechnology Symposium Part 2, 792-799). Monoclonal antibodies (mAbs) are particularly useful in the methods of the invention, and are a homogenous population of antibodies specifically targeting a single epitope on an antigen.

Polyclonal antibodies are also useful in the methods of the invention. Monospecific polyclonal antibodies are preferred. Suitable polyclonal antibodies can be prepared using methods well known in the art.

Antigen-binding fragments of antibodies, such as Fab and Fab₂ fragments may also be used/provided as can genetically engineered antibodies and antibody fragments. The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sd. USA 81, 6851-6855).

Antibodies and antigen-binding fragments according to the present disclosure comprise the complementarity-determining regions (CDRs) of an antibody which is capable of binding to the relevant target molecule, i.e. IL-11, an IL-11 containing complex, a receptor for IL-11, an angiogenic factor, an angiogenic factor containing complex or a receptor for an angiogenic factor.

Antibodies generally comprise six CDRs; three in the light chain variable region (VL): LC-CDR1, LC-CDR2, LC-CDR3, and three in the heavy chain variable region (VH): HC-CDR1, HC-CDR2 and HC-CDR3. The six CDRs together define the paratope of the antibody, which is the part of the antibody which binds to the target molecule. There are several different conventions for defining antibody CDRs, such as those described in Kabat et al., Sequences of Proteins of Immunological Interest, 5th Ed. Public Health Service, National Institutes of Health, Bethesda, MD (1991), Chothia et al., J. Mol. Biol. 196:901-917 (1987), and VBASE2, as described in Retter et al., Nucl. Acids Res. (2005) 33 (suppl 1): D671-D674.

Antibodies and antigen-binding fragments according to the present disclosure may be designed and prepared using the sequences of monoclonal antibodies (mAbs) capable of binding to the relevant target molecule. Antigen-binding regions of antibodies, such as single chain variable fragment (scFv), Fab and Fab₂ fragments may also be used/provided. An 'antigen-binding region' is any fragment of an antibody which is capable of binding to the target for which the given antibody is specific.

In some embodiments the antibodies/fragments comprise the VL and VH regions of an antibody which is capable of binding to an angiogenic factor, an angiogenic factor containing complex, or a receptor for an angiogenic factor. The VL and VH region of an antigen-binding region of an antibody together constitute the Fv region. In some embodiments the antibodies/fragments comprise or consist of the Fv region of an antibody which is capable of binding to an angiogenic factor, an angiogenic factor containing complex, or a receptor for an angiogenic factor. The Fv region may be expressed as a single chain wherein the VH and VL regions are covalently linked, e.g. by a flexible oligopeptide. Accordingly, antibodies/fragments may comprise or consist of an scFv comprising the VL and VH regions of an antibody which is capable of binding to an angiogenic factor, an angiogenic factor containing complex, or a receptor for an angiogenic factor.

The VL and light chain constant (CL) region, and the VH region and heavy chain constant 1 (CH1) region of an antigen-binding region of an antibody together constitute the Fab region. In some embodiments the antibodies/fragments comprise or consist of the Fab region of an antibody which is capable of binding to an angiogenic factor, an angiogenic factor containing complex, or a receptor for an angiogenic factor.

In some embodiments, antibodies/fragments comprise, or consist of, whole antibody capable of binding to an angiogenic factor, an angiogenic factor containing complex, or a receptor for an angiogenic factor. A "whole antibody" refers to an antibody having a structure which is substantially similar to the structure of an immunoglobulin (Ig). Different kinds of immunoglobulins and their structures are described e.g. in Schroeder and Cavacini J Allergy Clin Immunol. (2010) 125(202): S41-S52. Immunoglobulins of type G (i.e. IgG) are ~150 kDa glycoproteins comprising two heavy chains and two light chains. From N- to C-terminus, the heavy chains comprise a VH followed by a heavy chain constant region comprising three constant domains (CH1, CH2, and CH3), and similarly the light chain comprise a VL followed by a CL. Depending on the heavy chain, immunoglobulins may be classed as IgG (e.g. IgG1, IgG2, IgG3, IgG4), IgA (e.g. IgA1, IgA2), IgD, IgE, or IgM. The light chain may be kappa (κ) or lambda (λ).

Fab, Fv, ScFv and dAb antibody fragments can all be expressed in and secreted from E. coli, thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining site. Synthetic antibodies capable of binding to an angiogenic factor, an angiogenic factor containing complex, or a receptor for an angiogenic factor may also be made using phage display technology as is well known in the art.

Antibodies may be produced by a process of affinity maturation in which a modified antibody is generated that has an improvement in the affinity of the antibody for antigen, compared to an unmodified parent antibody. Affinity-matured antibodies may be produced by procedures known in the art, e.g., Marks et al.,Rio/Technology 10:779-783 (1992); Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):331 0-15 9 (1995); and Hawkins et al, J. Mol. Biol. 226:889-896 (1992).

Antibodies/fragments include multispecific (e.g. bi-specific) antibodies, e.g. composed of fragments of two or more different antibodies, such that the multispecific antibody binds two or more types of antigen. In some embodiments the bispecific antibody comprises an antibody/fragment as described herein capable of binding to an angiogenic factor, an angiogenic factor containing complex, or a receptor for an angiogenic factor, IL-11, an IL-11 containing complex, or a receptor for IL-11. In some embodiments the bispecific antibody comprises (i) an antibody/fragment as described herein capable of binding to an angiogenic factor, an angiogenic factor containing complex, and (ii) an antibody/fragment as described herein capable of binding to IL-11, an IL-11 containing complex, or a receptor for IL-11. The antibody may contain a different fragment having affinity for a second antigen, which may be any desired antigen.

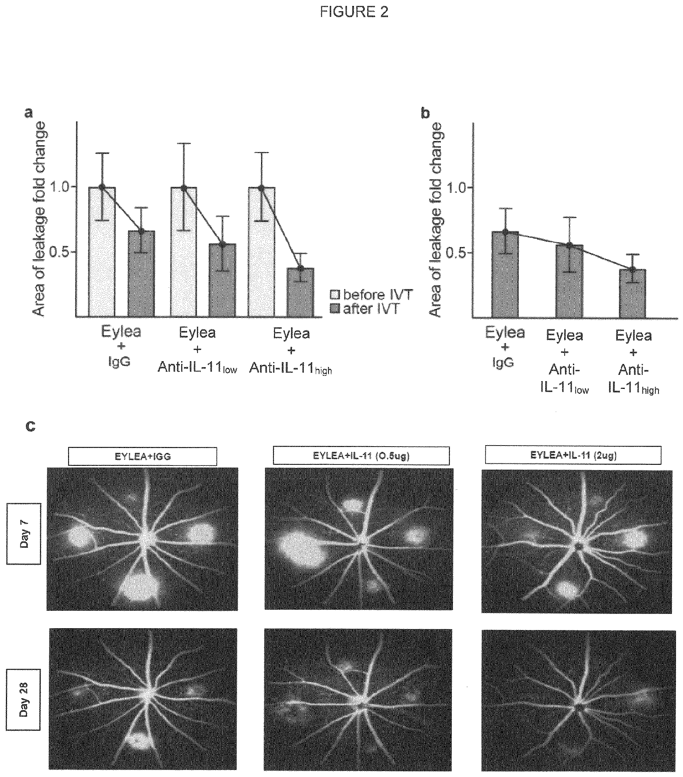

Techniques for the preparation of bi-specific antibodies are well known in the art, e.g. see Mueller, D et al., (2010 Biodrugs 24 (2): 89-98), Wozniak-Knopp G et al., (2010 Protein Eng Des 23 (4): 289-297. Baeuerle, PA et al., (2009 Cancer Res 69 (12): 4941-4944). Bispecific antibodies and bispecific antigen-binding fragments may be provided in any suitable format, such as those formats described in Kontermann MAbs 2012, 4(2): 182-197. For example, a bispecific antibody or bispecific antigen-binding fragment may be a bispecific antibody conjugate (e.g. an IgG2, F(ab')₂ or CovX-Body), a bispecific IgG or IgG-like molecule (e.g. an IgG, scFv₄-Ig, IgG-scFv, scFv-IgG, DVD-Ig, IgG-sVD, sVD-IgG, 2 in 1-IgG, mAb², or Tandemab common LC), an asymmetric bispecific IgG or IgG-like molecule (e.g. a kih IgG, kih IgG common LC, CrossMab, kih IgG-scFab, mAb-Fv, charge pair or SEED-body), a small bispecific antibody molecule (e.g. a Diabody (Db), dsDb, DART, scDb, tandAbs, tandem scFv (taFv), tandem dAb/VHH, triple body, triple head, Fab-scFv, or F(ab')₂-scFv₂), a bispecific Fc and C_{H}3 fusion protein (e.g. a taFv-Fc, Di-diabody, scDb-C_{H}3, scFv-Fc-scFv, HCAb-VHH, scFv-kih-Fc, or scFv-kih-C_{H}3), or a bispecific fusion protein (e.g. a scFv₂-albumin, scDb-albumin, taFv-toxin, DNL-Fab₃, DNL-Fab₄-IgG, DNL-Fab₄-IgG-cytokine₂). See in particular Figure 2 of Kontermann MAbs 2012, 4(2): 182-19.

Methods for producing bispecific antibodies include chemically crosslinking antibodies or antibody fragments, e.g. with reducible disulphide or non-reducible thioether bonds, for example as described in Segal and Bast, 2001. Production of Bispecific Antibodies. Current Protocols in Immunology. 14:IV:2.13:2.13.1-2.13.16. For example, *N*-succinimidyl-3-(-2-pyridyldithio)-propionate (SPDP) can be used to chemically crosslink e.g. Fab fragments via hinge region SH- groups, to create disulfide-linked bispecific F(ab)₂ heterodimers.

Other methods for producing bispecific antibodies include fusing antibody-producing hybridomas e.g. with polyethylene glycol, to produce a quadroma cell capable of secreting bispecific antibody, for example as described in D. M. and Bast, B. J. 2001. Production of Bispecific Antibodies. Current Protocols in Immunology. 14:IV:2.13:2.13.1-2.13.16.

Bispecific antibodies and bispecific antigen-binding fragments can also be produced recombinantly, by expression from e.g. a nucleic acid construct encoding polypeptides for the antigen binding molecules, for example as described in Antibody Engineering: Methods and Protocols, Second Edition (Humana Press, 2012), at Chapter 40: Production of Bispecific Antibodies: Diabodies and Tandem scFv (Hornig and Färber-Schwarz), or French, How to make bispecific antibodies, Methods Mol. Med. 2000; 40:333-339.

For example, a DNA construct encoding the light and heavy chain variable domains for the two antigen binding domains (i.e. the light and heavy chain variable domains for the antigen binding domain capable of binding to an angiogenic factor, an angiogenic factor containing complex, or a receptor for an angiogenic factor, and the light and heavy chain variable domains for the antigen binding domain capable of binding to another target protein), and including sequences encoding a suitable linker or dimerization domain between the antigen binding domains can be prepared by molecular cloning techniques. Recombinant bispecific antibody can thereafter be produced by expression (e.g. *in vitro*) of the construct in a suitable host cell (e.g. a mammalian host cell), and expressed recombinant bispecific antibody can then optionally be purified.

### Aptamers

In some examples, agents capable of inhibiting IL-11-mediated signalling or capable of inhibiting angiogenic factor-mediated signalling are aptamers.

Aptamers, also called nucleic acid/peptide ligands, are nucleic acid or peptide molecules characterised by the ability to bind to a target molecule with high specificity and high affinity. Almost every aptamer identified to date is a non-naturally occurring molecule.

Aptamers to a given target (e.g. IL-11, an IL-11 containing complex or a receptor for IL-11, an angiogenic factor, an angiogenic factor containing complex or a receptor for an angiogenic factor) may be identified and/or produced by the method of Systematic Evolution of Ligands by EXponential enrichment (SELEX^{™}), or by developing SOMAmers (slow off-rate modified aptamers) (Gold Let al. (2010) PLoS ONE 5(12):e15004). Aptamers and SELEX are described in Tuerk and Gold, Science (1990) 249(4968):505-10, and in WO 91/19813. Applying the SELEX and the SOMAmer technology includes for instance adding functional groups that mimic amino acid side chains to expand the aptamer's chemical diversity. As a result high affinity aptamers for a target may be enriched and identified.

Aptamers may be DNA or RNA molecules and may be single stranded or double stranded. The aptamer may comprise chemically modified nucleic acids, for example in which the sugar and/or phosphate and/or base is chemically modified. Such modifications may improve the stability of the aptamer or make the aptamer more resistant to degradation and may include modification at the 2' position of ribose.

Aptamers may be synthesised by methods which are well known to the skilled person. For example, aptamers may be chemically synthesised, e.g. on a solid support. Solid phase synthesis may use phosphoramidite chemistry. Briefly, a solid supported nucleotide is detritylated, then coupled with a suitably activated nucleoside phosphoramidite to form a phosphite triester linkage. Capping may then occur, followed by oxidation of the phosphite triester with an oxidant, typically iodine. The cycle may then be repeated to assemble the aptamer (e.g., see Sinha, N. D.; Biernat, J.; McManus, J.; Köster, H. Nucleic Acids Res. 1984, 12, 4539; and Beaucage, S. L.; Lyer, R. P. (1992). Tetrahedron 48 (12): 2223).

Suitable nucleic acid aptamers may optionally have a minimum length of one of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides. Suitable nucleic acid aptamers may optionally have a maximum length of one of 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides. Suitable nucleic acid aptamers may optionally have a length of one of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides.

Aptamers may be peptides selected or engineered to bind specific target molecules. Peptide aptamers and methods for their generation and identification are reviewed in Reverdatto et al., Curr Top Med Chem. (2015) 15(12):1082-101. Peptide aptamers may optionally have a minimum length of one of 2, 3, 4, 5, 6, 7, 8, 9 or 10 amino acids. Peptide aptamers may optionally have a maximum length of one of 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids. Suitable peptide aptamers may optionally have a length of one of 2-30, 2-25, 2-20, 5-30, 5-25 or 5-20 amino acids.

Aptamers may have K_{d}'s in the nM or pM range, e.g. less than one of 500nM, 100nM, 50nM, 10nM, 1nM, 500pM, 100pM.

### Agents capable of reducing expression of a factor or receptor

In aspects of the present disclosure, the antagonist of IL-11 mediated signalling and/or the antagonist of an angiogenic factor is capable of preventing or reducing the expression of one or more targets.

Expression may be gene or protein expression.

Gene expression can be measured by various means known to those skilled in the art, for example by measuring levels of mRNA by quantitative real-time PCR (qRT-PCR), or by reporter-based methods. Similarly, protein expression can be measured by various methods well known in the art, e.g. by antibody-based methods, for example by western blot, immunohistochemistry, immunocytochemistry, flow cytometry, ELISA, ELISPOT, or reporter-based methods. Expression may be by a cell/tissue/organ/organ system of a subject.

In some examples, the agent may be an inhibitory nucleic acid, such as antisense or small interfering RNA, including but not limited to shRNA or siRNA.

Oligonucleotide molecules, particularly RNA, may be employed to regulate gene expression. These include antisense oligonucleotides, targeted degradation of mRNAs by small interfering RNAs (siRNAs), post transcriptional gene silencing (PTGs), developmentally regulated sequence-specific translational repression of mRNA by micro-RNAs (miRNAs), and targeted transcriptional gene silencing.

An antisense oligonucleotide is an oligonucleotide, preferably single-stranded, that targets and binds, by complementary sequence binding, to a target oligonucleotide, e.g. mRNA. Where the target oligonucleotide is an mRNA, binding of the antisense to the mRNA blocks translation of the mRNA and expression of the gene product. Antisense oligonucleotides may be designed to bind sense genomic nucleic acid and inhibit transcription of a target nucleotide sequence.

A role for the RNAi machinery and small RNAs in targeting of heterochromatin complexes and epigenetic gene silencing at specific chromosomal loci has been demonstrated. Double-stranded RNA (dsRNA)-dependent post transcriptional silencing, also known as RNA interference (RNAi), is a phenomenon in which dsRNA complexes can target specific genes of homology for silencing in a short period of time. It acts as a signal to promote degradation of mRNA with sequence identity. A 20-nt siRNA is generally long enough to induce gene-specific silencing, but short enough to evade host response. The decrease in expression of targeted gene products can be extensive with 90% silencing induced by a few molecules of siRNA. RNAi based therapeutics have been progressed into Phase I, II and III clinical trials for a number of indications (Nature 2009 Jan 22; 457(7228):426-433).

In the art, these RNA sequences are termed "short or small interfering RNAs" (siRNAs) or "microRNAs" (miRNAs) depending on their origin. Both types of sequence may be used to down-regulate gene expression by binding to complementary RNAs and either triggering mRNA elimination (RNAi) or arresting mRNA translation into protein. siRNA are derived by processing of long double stranded RNAs and when found in nature are typically of exogenous origin. Micro-interfering RNAs (miRNA) are endogenously encoded small non-coding RNAs, derived by processing of short hairpins. Both siRNA and miRNA can inhibit the translation of mRNAs bearing partially complimentary target sequences without RNA cleavage and degrade mRNAs bearing fully complementary sequences.

siRNA ligands are typically double stranded and, in order to optimise the effectiveness of RNA mediated down-regulation of the function of a target gene, it is preferred that the length of the siRNA molecule is chosen to ensure correct recognition of the siRNA by the RISC complex that mediates the recognition by the siRNA of the mRNA target and so that the siRNA is short enough to reduce a host response.

miRNA ligands are typically single stranded and have regions that are partially complementary enabling the ligands to form a hairpin. miRNAs are RNA genes which are transcribed from DNA, but are not translated into protein. A DNA sequence that codes for a miRNA gene is longer than the miRNA. This DNA sequence includes the miRNA sequence and an approximate reverse complement. When this DNA sequence is transcribed into a single-stranded RNA molecule, the miRNA sequence and its reverse-complement base pair to form a partially double stranded RNA segment. The design of microRNA sequences is discussed in John et al, PLoS Biology, 11(2), 1862-1879, 2004.

Typically, the RNA ligands intended to mimic the effects of siRNA or miRNA have between 10 and 40 ribonucleotides (or synthetic analogues thereof), more preferably between 17 and 30 ribonucleotides, more preferably between 19 and 25 ribonucleotides and most preferably between 21 and 23 ribonucleotides. In some examples employing double-stranded siRNA, the molecule may have symmetric 3' overhangs, e.g. of one or two (ribo)nucleotides, typically a UU of dTdT 3' overhang. Based on the disclosure provided herein, the skilled person can readily design suitable siRNA and miRNA sequences, for example using resources such the Ambion siRNA finder. siRNA and miRNA sequences can be synthetically produced and added exogenously to cause gene downregulation or produced using expression systems (e.g. vectors). In an examplethe siRNA is synthesized synthetically.

Longer double stranded RNAs may be processed in the cell to produce siRNAs (see for example Myers (2003) Nature Biotechnology 21:324-328). The longer dsRNA molecule may have symmetric 3' or 5' overhangs, e.g. of one or two (ribo)nucleotides, or may have blunt ends. The longer dsRNA molecules may be 25 nucleotides or longer. Preferably, the longer dsRNA molecules are between 25 and 30 nucleotides long. More preferably, the longer dsRNA molecules are between 25 and 27 nucleotides long. Most preferably, the longer dsRNA molecules are 27 nucleotides in length. dsRNAs 30 nucleotides or more in length may be expressed using the vector pDECAP (Shinagawa et al., Genes and Dev., 17, 1340-5, 2003).

Another alternative is the expression of a short hairpin RNA molecule (shRNA) in the cell. shRNAs are more stable than synthetic siRNAs. A shRNA consists of short inverted repeats separated by a small loop sequence. One inverted repeat is complimentary to the gene target. In the cell the shRNA is processed by DICER into a siRNA which degrades the target gene mRNA and suppresses expression. In an examplethe shRNA is produced endogenously (within a cell) by transcription from a vector. shRNAs may be produced within a cell by transfecting the cell with a vector encoding the shRNA sequence under control of a RNA polymerase III promoter such as the human H1 or 7SK promoter or a RNA polymerase II promoter. Alternatively, the shRNA may be synthesised exogenously (*in vitro*) by transcription from a vector. The shRNA may then be introduced directly into the cell. Preferably, the shRNA sequence is between 40 and 100 bases in length, more preferably between 40 and 70 bases in length. The stem of the hairpin is preferably between 19 and 30 base pairs in length. The stem may contain G-U pairings to stabilise the hairpin structure.

siRNA molecules, longer dsRNA molecules or miRNA molecules may be made recombinantly by transcription of a nucleic acid sequence, preferably contained within a vector. Preferably, the siRNA molecule, longer dsRNA molecule or miRNA molecule comprises a partial sequence of a target factor or receptor.

In one example, the siRNA, longer dsRNA or miRNA is produced endogenously (within a cell) by transcription from a vector. The vector may be introduced into the cell in any of the ways known in the art. Optionally, expression of the RNA sequence can be regulated using a tissue specific (e.g. heart, liver, kidney or eye specific) promoter. In a further example, the siRNA, longer dsRNA or miRNA is produced exogenously (*in vitro*) by transcription from a vector.

Suitable vectors may be oligonucleotide vectors configured to express the oligonucleotide agent capable of repression. Such vectors may be viral vectors or plasmid vectors. The therapeutic oligonucleotide may be incorporated in the genome of a viral vector and be operably linked to a regulatory sequence, e.g. promoter, which drives its expression. The term "operably linked" may include the situation where a selected nucleotide sequence and regulatory nucleotide sequence are covalently linked in such a way as to place the expression of a nucleotide sequence under the influence or control of the regulatory sequence. Thus a regulatory sequence is operably linked to a selected nucleotide sequence if the regulatory sequence is capable of effecting transcription of a nucleotide sequence which forms part or all of the selected nucleotide sequence.

Viral vectors encoding promoter-expressed siRNA sequences are known in the art and have the benefit of long term expression of the therapeutic oligonucleotide. Examples include lentiviral (Nature 2009 Jan 22; 457(7228):426-433), adenovirus (Shen et al., FEBS Lett 2003 Mar 27;539(1-3)111-4) and retroviruses (Barton and Medzhitov PNAS November 12, 2002 vol.99, no.23 14943-14945).

In other examples a vector may be configured to assist delivery of the therapeutic oligonucleotide to the site at which repression of expression is required. Such vectors typically involve complexing the oligonucleotide with a positively charged vector (e.g., cationic cell penetrating peptides, cationic polymers and dendrimers, and cationic lipids); conjugating the oligonucleotide with small molecules (e.g., cholesterol, bile acids, and lipids), polymers, antibodies, and RNAs; or encapsulating the oligonucleotide in nanoparticulate formulations (Wang et al., AAPS J. 2010 Dec; 12(4): 492-503).

In one example, a vector may comprise a nucleic acid sequence in both the sense and antisense orientation, such that when expressed as RNA the sense and antisense sections will associate to form a double stranded RNA.

Alternatively, siRNA molecules may be synthesized using standard solid or solution phase synthesis techniques which are known in the art. Linkages between nucleotides may be phosphodiester bonds or alternatives, for example, linking groups of the formula P(O)S, (thioate); P(S)S, (dithioate); P(O)NR'2; P(O)R'; P(O)OR6; CO; or CONR'2 wherein R is H (or a salt) or alkyl (1-12C) and R6 is alkyl (1-9C) is joined to adjacent nucleotides through-O-or-S-.

Modified nucleotide bases can be used in addition to the naturally occurring bases, and may confer advantageous properties on siRNA molecules containing them.

For example, modified bases may increase the stability of the siRNA molecule, thereby reducing the amount required for silencing. The provision of modified bases may also provide siRNA molecules which are more, or less, stable than unmodified siRNA.

The term 'modified nucleotide base' encompasses nucleotides with a covalently modified base and/or sugar. For example, modified nucleotides include nucleotides having sugars which are covalently attached to low molecular weight organic groups other than a hydroxyl group at the 3'position and other than a phosphate group at the 5'position. Thus modified nucleotides may also include 2'substituted sugars such as 2'-O-methyl- ; 2'-O-alkyl ; 2'-O-allyl ; 2'-S-alkyl; 2'-S-allyl; 2'-fluoro- ; 2'-halo or azido-ribose, carbocyclic sugar analogues, α-anomeric sugars; epimeric sugars such as arabinose, xyloses or lyxoses, pyranose sugars, furanose sugars, and sedoheptulose.

Modified nucleotides are known in the art and include alkylated purines and pyrimidines, acylated purines and pyrimidines, and other heterocycles. These classes of pyrimidines and purines are known in the art and include pseudoisocytosine, N4,N4-ethanocytosine, 8-hydroxy-N6-methyladenine, 4-acetylcytosine,5-(carboxyhydroxylmethyl) uracil, 5 fluorouracil, 5-bromouracil, 5-carboxymethylaminomethyl-2-thiouracil, 5-carboxymethylaminomethyl uracil, dihydrouracil, inosine, N6-isopentyl-adenine, 1- methyladenine, 1-methylpseudouracil, 1-methylguanine, 2,2-dimethylguanine, 2-methyladenine, 2-methylguanine, 3-methylcytosine, 5-methylcytosine, N6-methyladenine, 7-methylguanine, 5-methylaminomethyl uracil, 5-methoxy amino methyl-2-thiouracil, -D-mannosylqueosine, 5-methoxycarbonylmethyluracil, 5methoxyuracil, 2 methylthio-N6-isopentenyladenine, uracil-5-oxyacetic acid methyl ester, psueouracil, 2-thiocytosine, 5-methyl-2 thiouracil, 2-thiouracil, 4-thiouracil, 5methyluracil, N-uracil-5-oxyacetic acid methylester, uracil 5-oxyacetic acid, queosine, 2-thiocytosine, 5-propyluracil, 5-propylcytosine, 5-ethyluracil, 5ethylcytosine, 5-butyluracil, 5-pentyluracil, 5-pentylcytosine, and 2,6,diaminopurine, methylpsuedouracil, 1-methylguanine, 1-methylcytosine.

Methods relating to the use of RNAi to silence genes in C. elegans, Drosophila, plants, and mammals are known in the art (Fire A, et al., 1998 Nature 391:806-811; Fire, A. Trends Genet. 15, 358-363 (1999); Sharp, P. A. RNA interference 2001. Genes Dev. 15, 485-490 (2001); Hammond, S. M., et al., Nature Rev. Genet. 2, 110-1119 (2001); Tuschl, T. Chem. Biochem. 2, 239-245 (2001); Hamilton, A. et al., Science 286, 950-952 (1999); Hammond, S. M., et al., Nature 404, 293-296 (2000); Zamore, P. D., et al., Cell 101, 25-33 (2000); Bernstein, E., et al., Nature 409, 363-366 (2001); Elbashir, S. M., et al., Genes Dev. 15, 188-200 (2001); WO0129058; WO9932619, and Elbashir S M, et al., 2001 Nature 411:494-498).

The nucleic acid may be a double-stranded siRNA. (As the skilled person will appreciate, and as explained further below, a siRNA molecule may include a short 3' DNA sequence also.)

It is expected that perfect identity/complementarity between the nucleic acid of the disclosure and the target sequence, although preferred, is not essential. Accordingly, the nucleic acid of the disclosure may include a single mismatch compared to the mRNA of the target. It is expected, however, that the presence of even a single mismatch is likely to lead to reduced efficiency, so the absence of mismatches is preferred. When present, 3' overhangs may be excluded from the consideration of the number of mismatches.

The term "complementarity" is not limited to conventional base pairing between nucleic acid consisting of naturally occurring ribo- and/or deoxyribonucleotides, but also includes base pairing between mRNA and nucleic acids of the disclosure that include non-natural nucleotides.

The strands that form the double-stranded RNA may have short 3' dinucleotide overhangs, which may be DNA or RNA. The use of a 3' DNA overhang has no effect on siRNA activity compared to a 3' RNA overhang, but reduces the cost of chemical synthesis of the nucleic acid strands (Elbashir et al., 2001c). For this reason, DNA dinucleotides may be preferred.

When present, the dinucleotide overhangs may be symmetrical to each other, though this is not essential. Indeed, the 3' overhang of the sense (upper) strand is irrelevant for RNAi activity, as it does not participate in mRNA recognition and degradation (Elbashir et al., 2001a, 2001b, 2001c).

While RNAi experiments in Drosophila show that antisense 3' overhangs may participate in mRNA recognition and targeting (Elbashir et al. 2001c), 3' overhangs do not appear to be necessary for RNAi activity of siRNA in mammalian cells. Incorrect annealing of 3' overhangs is therefore thought to have little effect in mammalian cells (Elbashir et al. 2001c; Czauderna et al. 2003).

Any dinucleotide overhang may therefore be used in the antisense strand of the siRNA. Nevertheless, the dinucleotide is preferably -UU or -UG (or -TT or -TG if the overhang is DNA), more preferably -UU (or - TT). The -UU (or -TT) dinucleotide overhang is most effective and is consistent with (i.e. capable of forming part of) the RNA polymerase III end of transcription signal (the terminator signal is TTTTT). Accordingly, this dinucleotide is most preferred. The dinucleotides AA, CC and GG may also be used, but are less effective and consequently less preferred.

Moreover, the 3' overhangs may be omitted entirely from the siRNA.

Disclosed herein are single-stranded nucleic acids (herein referred to as single-stranded siRNAs) respectively consisting of a component strand of one of the aforementioned double-stranded nucleic acids, preferably with the 3'-overhangs, but optionally without. Also disclosed herein are kits containing pairs of such single-stranded nucleic acids, which are capable of hybridising with each other *in vitro* to form the aforementioned double-stranded siRNAs, which may then be introduced into cells.

The complementary portions will generally be joined by a spacer, which has suitable length and sequence to allow the two complementary portions to hybridise with each other. The two complementary (i.e. sense and antisense) portions may be joined 5'-3' in either order. The spacer will typically be a short sequence, of approximately 4-12 nucleotides, preferably 4-9 nucleotides, more preferably 6-9 nucleotides.

Preferably the 5' end of the spacer (immediately 3' of the upstream complementary portion) consists of the nucleotides -UU- or -UG-, again preferably -UU- (though, again, the use of these particular dinucleotides is not essential). A suitable spacer, recommended for use in the pSuper system of OligoEngine (Seattle, Washington, USA) is UUCAAGAGA. In this and other cases, the ends of the spacer may hybridise with each other, e.g. elongating the double-stranded motif beyond the exact sequences of the target by a small number (e.g. 1 or 2) of base pairs.

Similarly, the transcribed RNA preferably includes a 3' overhang from the downstream complementary portion. Again, this is preferably -UU or -UG, more preferably -UU.

Such shRNA molecules may then be cleaved in the mammalian cell by the enzyme DICER to yield a double-stranded siRNA as described above, in which one or each strand of the hybridised dsRNA includes a 3' overhang.

Techniques for the synthesis of the nucleic acids of the disclosure are of course well known in the art.

The skilled person is well able to construct suitable transcription vectors for the DNA of the disclosure using well-known techniques and commercially available materials. In particular, the DNA will be associated with control sequences, including a promoter and a transcription termination sequence.

Of particular suitability are the commercially available pSuper and pSuperior systems of OligoEngine (Seattle, Washington, USA). These use a polymerase-III promoter (H1) and a T5 transcription terminator sequence that contributes two U residues at the 3' end of the transcript (which, after DICER processing, provide a 3' UU overhang of one strand of the siRNA).

Another suitable system is described in Shin et al. (RNA, 2009 May; 15(5): 898-910), which uses another polymerase-III promoter (U6).

Disclosed herein is a composition comprising a double-stranded siRNA of the disclosure or a transcription vector of the disclosure in admixture with one or more pharmaceutically acceptable carriers. Suitable carriers include lipophilic carriers or vesicles, which may assist in penetration of the cell membrane.

Materials and methods suitable for the administration of siRNA duplexes and DNA vectors of the disclosure are well known in the art and improved methods are under development, given the potential of RNAi technology.

Generally, many techniques are available for introducing nucleic acids into mammalian cells. The choice of technique will depend on whether the nucleic acid is transferred into cultured cells *in vitro* or *in vivo* in the cells of a patient. Techniques suitable for the transfer of nucleic acid into mammalian cells *in vitro* include the use of liposomes, electroporation, microinjection, cell fusion, DEAE, dextran and calcium phosphate precipitation. *In vivo* gene transfer techniques include transfection with viral (typically retroviral) vectors and viral coat protein-liposome mediated transfection (Dzau et al. (2003) Trends in Biotechnology 11, 205-210).

In particular, suitable techniques for cellular administration of the nucleic acids of the disclosure both *in vitro* and *in vivo* are disclosed in the following articles:
General reviews: Borkhardt, A. 2002. Blocking oncogenes in malignant cells by RNA interference--new hope for a highly specific cancer treatment? Cancer Cell. 2:167-8. Hannon, G.J. 2002. RNA interference. Nature. 418:244-51. McManus, M.T., and P.A. Sharp. 2002. Gene silencing in mammals by small interfering RNAs. Nat Rev Genet. 3:737-47. Scherr, M., M.A. Morgan, and M. Eder. 2003b. Gene silencing mediated by small interfering RNAs in mammalian cells. Curr Med Chem. 10:245-56. Shuey, D.J., D.E. McCallus, and T. Giordano. 2002. RNAi: gene-silencing in therapeutic intervention. Drug Discov Today. 7:1040-6.
Systemic delivery using liposomes: Lewis, D.L., J.E. Hagstrom, A.G. Loomis, J.A. Wolff, and H. Herweijer. 2002. Efficient delivery of siRNA for inhibition of gene expression in postnatal mice. Nat Genet. 32:107-8. Paul, C.P., P.D. Good, I. Winer, and D.R. Engelke. 2002. Effective expression of small interfering RNA in human cells. Nat Biotechnol. 20:505-8. Song, E., S.K. Lee, J. Wang, N. Ince, N. Ouyang, J. Min, J. Chen, P. Shankar, and J. Lieberman. 2003. RNA interference targeting Fas protects mice from fulminant hepatitis. Nat Med. 9:347-51. Sorensen, D.R., M. Leirdal, and M. Sioud. 2003. Gene silencing by systemic delivery of synthetic siRNAs in adult mice. J Mol Biol. 327:761-6.
Virus mediated transfer: Abbas-Terki, T., W. Blanco-Bose, N. Deglon, W. Pralong, and P. Aebischer. 2002. Lentiviral-mediated RNA interference. Hum Gene Ther. 13:2197-201. Barton, G.M., and R. Medzhitov. 2002. Retroviral delivery of small interfering RNA into primary cells. Proc Natl Acad Sci U S A. 99:14943-5. Devroe, E., and P.A. Silver. 2002. Retrovirus-delivered siRNA. BMC Biotechnol. 2:15. Lori, F., P. Guallini, L. Galluzzi, and J. Lisziewicz. 2002. Gene therapy approaches to HIV infection. Am J Pharmacogenomics. 2:245-52. Matta, H., B. Hozayev, R. Tomar, P. Chugh, and P.M. Chaudhary. 2003. Use of lentiviral vectors for delivery of small interfering RNA. Cancer Biol Ther. 2:206-10. Qin, X.F., D.S. An, I.S. Chen, and D. Baltimore. 2003. Inhibiting HIV-1 infection in human T cells by lentiviral-mediated delivery of small interfering RNA against CCR5. Proc Natl Acad Sci U S A. 100:183-8. Scherr, M., K. Battmer, A. Ganser, and M. Eder. 2003a. Modulation of gene expression by lentiviral-mediated delivery of small interfering RNA. Cell Cycle. 2:251-7. Shen, C., A.K. Buck, X. Liu, M. Winkler, and S.N. Reske. 2003. Gene silencing by adenovirus-delivered siRNA. FEBS Lett. 539:111-4.
Peptide delivery: Morris, M.C., L. Chaloin, F. Heitz, and G. Divita. 2000. Translocating peptides and proteins and their use for gene delivery. Curr Opin Biotechnol. 11:461-6. Simeoni, F., M.C. Morris, F. Heitz, and G. Divita. 2003. Insight into the mechanism of the peptide-based gene delivery system MPG: implications for delivery of siRNA into mammalian cells. Nucleic Acids Res. 31:2717-24. Other technologies that may be suitable for delivery of siRNA to the target cells are based on nanoparticles or nanocapsules such as those described in US patent numbers 6,649,192B and 5,843,509B.

### Angiogenesis and angiogenic factors

Angiogenesis is the physiological process through which new blood vessels form from pre-existing vessels. It is a vital process in growth, would healing, and in tumour malignancy. Upregulation of angiogenesis is a common feature in diseases of the eye, and especially in neovascular diseases of the retina. Development and maintenance of the vasculature is critical to the normal function of all parenchymal tissues, and defects result in disease and organ failure, especially in ocular diseases described herein. Most neovascular retinal diseases result in fibrosis.

Numerous inducers of angiogenesis have been identified, including the members of the vascular endothelial growth factor (VEGF) family, angiopoietins, transforming growth factors (TGF), platelet-derived growth factor, tumour necrosis factor-a, interleukins and the members of the fibroblast growth factor (FGF) family. The cellular and molecular mechanisms of angiogenesis are described in Adams & Alitalo, Nature Reviews Molecular Cell Biology volume 8, pages 464-478 (2007), and Otrock et al, Understanding the biology of angiogenesis: Review of the most important molecular mechanisms, Blood Cells, Molecules, and Diseases, Volume 39, Issue 2, 2007, Pages 212-220.

As used herein, an "angiogenic factor" refers to any factor (e.g. peptide/polypeptide, glycoprotein, lipoprotein, glycan, glycolipid, lipid, nucleic acid or fragment thereof) capable of inducing/potentiating/promoting angiogenesis. Factors capable of inducing/potentiating/promoting angiogenesis can be identified e.g. by analysis in an *in vitro* or *in vivo* assay of angiogenesis. Suitable assays for analysis of angiogenesis include endothelial cell migration assays, endothelial cell proliferation assays, and endothelial tube formation assays. Angiogenesis assays are described in detail in Adair TH, Montani JP. Angiogenesis. San Rafael (CA): Morgan & Claypool Life Sciences; 2010, Chapter 2, Angiogenesis Assays.

In some examples, an angiogenic factor is a polypeptide or a polypeptide complex capable of inducing/promoting angiogenesis. An angiogenic factor may be soluble or membrane-bound, and may be a polypeptide or polypeptide complex ligand, or a polypeptide or polypeptide complex receptor for such a ligand. An angiogenic factor may be from any species, and includes isoforms, fragments, variants or homologues of an angiogenic factor from any species. In preferred embodiments the species is human (Homo sapiens). Isoforms, fragments, variants or homologues of an angiogenic factor may optionally be characterised as having at least 70%, preferably one of 80%, 85%, 90%, 91%, 92%, 93%, 94%, 95%, 96%, 97%, 98%, 99% or 100% amino acid sequence identity to the amino acid sequence of immature or mature angiogenic factor from a given species, e.g. human. Isoforms, fragments, variants or homologues of an angiogenic factor may optionally be characterised by ability to bind to an interaction partner for the angiogenic factor (e.g. a receptor for the angiogenic factor, or a ligand for the angiogenic factor) and/or ability to induce/promote angiogenesis For example, an angiogenic factor may be a VEGF, a VEGF receptor, an FGF, an FGF receptor, a PDGF, or a PDGF receptor, or a fragment, variant, isoform or homologue of a VEGF, a VEGF receptor, an FGF, an FGF receptor, a PDGF, or a PDGF receptor.

A fragment of a polypeptide of an angiogenic factor may be of any length (by number of amino acids), although may optionally be at least 25% of the length of mature angiogenic factor and may have a maximum length of one of 50%, 75%, 80%, 85%, 90%, 91%, 92%, 93%, 94%, 95%, 96%, 97%, 98%, or 99% of the length of the mature angiogenic factor. An angiogenic factor may be a complex comprising one or more subunits, such as a hetero or homodimer.

As used herein, an "interaction partner for an angiogenic factor" refers to any factor (e.g. polypeptide or polypeptide complex) capable of interacting with (e.g. binding to) an angiogenic factor. The interaction may be non-covalent association.

An angiogenic factor and an interaction partner for the angiogenic factor preferably associate to form a biologically-functional complex, e.g. a receptor:ligand complex capable of initiating/promoting signalling (e.g. proangiogenic signalling). An interaction partner for an angiogenic factor may e.g. be a receptor for the angiogenic factor, or a ligand for the angiogenic factor. In some examples, interaction between an interaction partner for an angiogenic factor and the angiogenic factor induces/promotes proangiogenic signalling and/or angiogenesis. For example, binding of VEGF to a VEGF receptor is capable of triggering intracellular signalling by cells expressing the VEGF receptor, inducing/promoting angiogenic processes.

For example, an interaction partner for an angiogenic factor may be an angiogenic factor receptor. In some examples an angiogenic factor receptor may be a polypeptide or polypeptide complex capable of binding a VEGF, an FGF, a PDGF, or a fragment, variant, isoform or homologue of a VEGF, an FGF, or a PDGF. Angiogenic factor receptors include e.g. VEGF receptors, FGF receptors, and PDGF receptors. It will be appreciated that angiogenic factor receptors are angiogenic factors according to the present disclosure. For example, VEGFR is both an angiogenic factor (i.e. a factor capable of inducing/potentiating/promoting angiogenesis) and an angiogenic factor receptor (i.e. a receptor for VEGF).

Similarly, an interaction partner for an angiogenic factor may be a ligand for an angiogenic factor receptor. In some examples a ligand for an angiogenic factor receptor may be a polypeptide or polypeptide complex capable of binding a VEGF receptor, an FGF receptor, a PDGF receptor, or a fragment, variant, isoform or homologue of a VEGF receptor, an FGF receptor, or a PDGF receptor. Angiogenic factor ligands include e.g. VEGF, FGF, and PDGF. It will be appreciated that angiogenic factor ligands are angiogenic factors according to the present disclosure. For example, VEGF is both an angiogenic factor (i.e. a factor capable of inducing/potentiating/promoting angiogenesis) and a ligand for an angiogenic factor receptor (i.e. a ligand for VEGF receptor).

In some embodiments, any method or composition provided herein may be used for treating/preventing angiogenesis, in a fibrotic context or otherwise.

### Vascular endothelial growth factor (VEGF)

The angiogenic factor is a VEGF. VEGF is a growth factor, encoded by a gene family that includes placental growth factor (PIGF, AAD30179.1 GI: 4809334), VEGF-A (AAP86646.1 GI: 32699990), VEGF-B (AAC50721.1 GI: 1488259), VEGF-C (CAA63907.1 GI: 1182005), VEGF-D (BAA24264.1 GI: 2766190), and the orf virus encoded VEGF-E (ABA00650.1 GI: 74230845). In some embodiments, the VEGF is VEGF-A. Differences in exon splicing result in the generation of four main VEGF-A isoforms: VEGF121, VEGF165, VEGF189, and VEGF206, which have 121, 165, 189, and 206 amino acids after cleavage of the signal sequence, respectively [65]. VEGF165 appears to be the dominant isoform.

VEGF stimulates the growth of vascular endothelial cells derived from arteries, veins, and the lymphatic system, as well as inducing angiogenesis in a variety of *in vivo* models (i.e. the formation of thin-walled endothelium-lined structures), inducing rapid elevations in microvascular permeability [66-68].

In this specification, "VEGF" refers to a VEGF (e.g. VEGF-A, B, C, D or E) from any species and includes isoforms, fragments, variants or homologues of a VEGF from any species. In preferred embodiments the species is human (Homo sapiens). A fragment of VEGF may be of any length (by number of amino acids), although may optionally be at least 25% of the length of mature VEGF and may have a maximum length of one of 50%, 75%, 80%, 85%, 90%, 91%, 92%, 93%, 94%, 95%, 96%, 97%, 98%, or 99% of the length of mature VEGF. A fragment of VEGF may have a minimum length of 10 amino acids, and a maximum length of one of 15, 20, 25, 30, 40, 50, 100, 110, 120, 130, 140, 150, 160, 170, 180, 190, 200, 210, 220 or 225 amino acids

VEGF exerts its biological effects through binding to three receptors - VEGFR1 (AAH39007.1 GI: 24660372), VEGFR2 (P35968.2 GI: 9087218) and VEGFR3 (AAA85215.1 GI: 1150991). Each receptor has extracellular binding domains for VEGF, a transmembrane sequence and intracellular tyrosine kinase moieties. VEGF binding to the extracellular receptor domain dimerizes the receptors and results in phosphorylation of the intracellular tyrosine kinase moieties.

In this specification, "a receptor for VEGF" or "a VEGF receptor" refers to a polypeptide or polypeptide complex capable of binding VEGF. In some embodiments a VEGF receptor is capable of binding VEGF and inducing signal transduction in cells expressing the receptor. In some embodiments, "a receptor for VEGF" refers to a VEGFR. In this specification, unless otherwise stated, "VEGFR" refers to VEGFR1, VEGFR2 and/or VEGFR3.

VEGFR may optionally be characterised as having at least 70%, preferably one of 80%, 85%, 90%, 91%, 92%, 93%, 94%, 95%, 96%, 97%, 98%, 99% or 100% amino acid sequence identity to the amino acid sequence of VEGFR from a given species, e.g. human. Isoforms, fragments, variants or homologues of VEGFR may optionally be characterised by ability to bind VEGF (preferably from the same species) and stimulate signal transduction in cells expressing the VEGFR. A fragment of an VEGF receptor may be of any length (by number of amino acids), although may optionally be at least 25% of the length of the mature VEGFR and have a maximum length of one of 50%, 75%, 80%, 85%, 90%, 91%, 92%, 93%, 94%, 95%, 96%, 97%, 98%, or 99% of the length of the mature VEGFR. A fragment of an VEGF receptor fragment may have a minimum length of 10 amino acids, and a maximum length of one of 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, 1510, 1520, 1530, 1540, 1550, 1560, 1570, 1580, 1590, 1600, 1610, 1620, 1630, 1640, 1650, 1660, 1670, 1680, 1690, 1700, 1710, 1720, 1730, 1740, 1750, 1760, 1770, 1780, 1790, or 1795 amino acids.

### Fibroblast growth factors (FGF)

Acidic and basic fibroblast growth factors, aFGF and bFGF respectively, are heparin-binding protein mitogens that are thought to play an important role in angiogenesis. There are 22 distinct FGFs and four different tyrosine kinase receptors (FGFRs). FGF and its receptors are reviewed in Presta et al., Fibroblast growth factor/fibroblast growth factor receptor system in angiogenesis, Cytokine Growth Factor Rev., 16 (2005), pp. 159-178.

As used herein, "FGF" refers to an aFGF or bFGF from any species and includes isoforms, fragments, variants or homologues thereof. For example and FGF may be: FGF1 (AAH32697.1 GI: 21595687), FGF2 (P09038.3 GI: 261260095), FGF3 (P11487.1 GI: 122748), FGF4 (NP_001998.1 GI: 4503701), FGF5 (P12034.4 GI: 85700417), FGF6 (P10767.4 GI: 1169676), FGF7 (P21781.1 GI: 122756), FGF8 (NP_001193318.1 GI: 329755303), FGF9 (NP_002001.1 GI: 4503707), FGF10 (CAG46466.1 GI: 49456291), FGF11 (Q92914.1 GI: 2494457), FGF12 (P61328.1 GI: 47117683), FGF13 (Q92913.1 GI: 2494461), FGF14 (NP_001308867.1 GI: 1013165743), FGF15 (AAO13811.1 GI: 27448228), FGF16 (NP_003859.1 GI: 4503691), FGF17 (AAQ89228.1 GI: 37182856), FGF18 (AAQ89954.1 GI: 37222209), FGF19 (NP_005108.1 GI: 4826726), FGF20 (AAH98339.1 GI: 68226699), FGF21 (AAQ89444.1 GI: 37183289), or FGF22 (Q9HCT0.1 GI: 13626689), or isoforms, fragments, variants or homologues thereof. An "FGF receptor" refers to a polypeptide or polypeptide complex capable of binding an FGF, and isoforms, fragments, variants or homologues thereof. FGF receptors include FGFR1 (AAH15035.1 GI: 21955340), FGFR2 (AAA61188.1 GI: 3397110), FGFR3 (P22607.1 GI: 120050), and FGFR4 (AAH11847.1 GI: 15080148), and isoforms, fragments, variants or homologues thereof. In some examples, the FGF receptor is capable of binding FGF and inducing signal transduction in cells expressing the FGF receptor.

### Platelet-derived growth factor

Platelet-derived growth factor (PDGF) was initially purified from platelets and was then identified in fibroblasts, astrocytes, keratinocytes, epithelial cells and other cell types. PDGFs exist as heterodimers (PDGF-AB) or homodimers (PDGF-AA or -BB) composed of chains A (P04085.1 GI: 129719) and B (CAG46606.1 GI: 49456571). There are two forms of the PDGF-R, alpha (P16234.1 GI: 129892) and beta (P09619.1 GI: 129890) each encoded by a different gene. Depending on which growth factor is bound, PDGF-R homo- or heterodimerizes. PDGF and its receptors are described in Ross et al, The Biology of Platelet-Derived Growth Factor, Cell, Vol. 46, 155-169. July 18, 1986.

As used herein, "PDGF" refers to PDGF-A, PDGF-B, PDGF-AB, PDGF-AA, PDGF-BA, or PDGF-BB from any species and includes isoforms, fragments, variants or homologues thereof. A "PDGF receptor" refers to a polypeptide or polypeptide complex capable of binding a PDGF, or isoforms, fragments, variants or homologues thereof. In some examples, the PDGF receptor is PDGF-R alpha, PDGF-R beta, PDGF-R alpha-alpha homodimer, PDGF-R beta-beta homodimer, or the PDGF-R alpha-beta heterodimer. In some examples, the PDGF receptor is capable of binding PDGF and inducing signal transduction in cells expressing the PDGF receptor.

Other angiogenic factors include angiogenin (NP_001091046.1 GI: 148277046), angiopoietin (AAD19608.1 GI: 4378598), integrins (e.g. selected from (NP_001004439.1 GI: 52485853, NP_002194.2 GI: 116295258, NP_002195.1 GI: 4504747, NP_000876.3 GI: 67191027, NP_002196.4 GI: 1017029567, NP_001303235.1 GI: 937834186, NP_001138468.1 GI: 222418613, NP_003629.2 GI: 612407851, NP_002198.2 GI: 52485941, NP_001273304.1 GI: 556503454, NP_001004439.1 GI: 52485853, NP_001305114.1 GI: 970949440, NP_001273304.1 GI: 556503454, NP_002200.2 GI: 167466215, NP_001139280.1 GI: 224831239, NP_002201.1 GI: 4504763, EAW51597.1 GI: 119571982, NP_001273304.1 GI: 556503454, NP_391988.1 GI: 19743819, NP_001120963.2 GI: 735367803, NP_000203.2 GI: 47078292, NP_000204.3 GI: 54607035, NP_001341694.1 GI: 1269208512, NP_001269282.1 GI: 538916664, NP_000880.1 GI: 4504777, NP_002205.1 GI: 4504779), and TNFα (AQY77150.1 GI: 1159611449). Inhibitors of TNFα suitable for use according to the disclosure include the antibody infliximab.

### Fibrosis

As used herein, "fibrosis" refers to the formation of excess fibrous connective tissue as a result of the excess deposition of extracellular matrix components, for example collagen.

Fibrous connective tissue is characterised by having extracellular matrix (ECM) with a high collagen content. The collagen may be provided in strands or fibres, which may be arranged irregularly or aligned. The ECM of fibrous connective tissue may also include glycosaminoglycans.

As used herein, "excess fibrous connective tissue" refers to an amount of connective tissue at a given location (e.g. a given tissue or organ, or part of a given tissue or organ) which is greater than the amount of connective tissue present at that location in the absence of fibrosis, e.g. under normal, non-pathological conditions. As used herein, "excess deposition of extracellular matrix components" refers to a level of deposition of one or more extracellular matrix components which is greater than the level of deposition in the absence of fibrosis, e.g. under normal, non-pathological conditions.

The cellular and molecular mechanisms of fibrosis are described in Wynn, J. Pathol. (2008) 214(2): 199-210, and Wynn and Ramalingam, Nature Medicine (2012) 18:1028-1040.

Important cellular effectors of fibrosis are myofibroblasts, which produce a collagen-rich extracellular matrix.

In response to tissue injury, damaged cells and leukocytes produce pro-fibrotic factors such as TGFβ, IL-13 and PDGF, which activate fibroblasts to αSMA-expressing myofibroblasts, and recruit myofibroblasts to the site of injury. Myofibroblasts produce a large amount of extracellular matrix, and are important mediators in aiding contracture and closure of the wound. However, under conditions of persistent infection or during chronic inflammation there can be overactivation and recruitment of myofibroblasts, and thus over-production of extracellular matrix components, resulting in the formation of excess fibrous connective tissue.

In some embodiments fibrosis may be triggered by pathological conditions, e.g. conditions, infections or disease states that lead to production of pro-fibrotic factors such as TGFβ1. In some embodiments, fibrosis may be caused by physical injury/stimuli, chemical injury/stimuli or environmental injury/stimuli. Physical injury/stimuli may occur during surgery, e.g. iatrogenic causes. Chemical injury/stimuli may include drug induced fibrosis, e.g. following chronic administration of drugs such as bleomycin, cyclophosphamide, amiodarone, procainamide, penicillamine, gold and nitrofurantoin (Daba et al., Saudi Med J 2004 Jun; 25(6): 700-6). Environmental injury/stimuli may include exposure to asbestos fibres or silica.

Fibrosis can occur in many tissues of the body. For example, fibrosis can occur in the liver (e.g. cirrhosis), lungs, kidney, heart, blood vessels, eye, skin, pancreas, intestine, brain, and bone marrow. Fibrosis may also occur in multiple organs at once.

In some embodiments, fibrosis may involve an organ of the gastrointestinal system, e.g. of the liver, small intestine, large intestine, or pancreas. In some embodiments, fibrosis may involve an organ of the respiratory system, e.g. the lungs. In some embodiments, fibrosis may involve an organ of the cardiovascular system, e.g. of the heart or blood vessels. In some embodiments, fibrosis may involve the skin. In some embodiments, fibrosis may involve an organ of the nervous system, e.g. the brain. In some embodiments, fibrosis may involve an organ of the urinary system, e.g. the kidneys. In some embodiments, fibrosis may involve an organ of the musculoskeletal system, e.g. muscle tissue.

In some embodiments fibrosis may be cardiac or myocardial fibrosis, hepatic fibrosis, or renal fibrosis. In some embodiments cardiac or myocardial fibrosis is associated with dysfunction of the musculature or electrical properties of the heart, or thickening of the walls of valves of the heart. In some embodiments fibrosis is of the atrium and/or ventricles of the heart. Treatment or prevention of atrial or ventricular fibrosis may help reduce risk or onset of atrial fibrillation, ventricular fibrillation, or myocardial infarction. In some embodiments hepatic fibrosis is associated with chronic liver disease or liver cirrhosis. In some embodiments renal fibrosis is associated with chronic kidney disease.

In some embodiments, fibrosis may be associated with angiogenesis. In some embodiments, methods of treating or preventing fibrosis, methods of determining the suitability of a subject for such treatment/prevention and methods of diagnosing/prognosing fibrosis as described herein are also applicable to treating/preventing/diagnosing/prognosing angiogenesis, and vice versa.

Diseases/conditions characterised by fibrosis in accordance with the present invention include but are not limited to: diseases of the eye such as Grave's opthalmopathy, epiretinal fibrosis, retinal fibrosis, idiopathic premacular fibrosis, subretinal fibrosis (e.g. associated with retinal detachment or macular degeneration (e.g. wet age-related macular degeneration (AMD)), choroidal neovascularisation (CNV), diabetic retinopathy, glaucoma, geographic atrophy (dry age-related macular degeneration (AMD)), corneal fibrosis, post-surgical fibrosis (e.g. of the posterior capsule following cataract surgery, or of the bleb following trabeculectomy for glaucoma), conjunctival fibrosis, or subconjunctival fibrosis; respiratory conditions such as pulmonary fibrosis, cystic fibrosis, idiopathic pulmonary fibrosis, progressive massive fibrosis, scleroderma, obliterative bronchiolitis, Hermansky-Pudlak syndrome, asbestosis, silicosis, chronic pulmonary hypertension, AIDS associated pulmonary hypertension, sarcoidosis, tumor stroma in lung disease, and asthma; chronic liver disease, primary biliary cirrhosis (PBC), schistosomal liver disease, liver cirrhosis; cardiovascular conditions such as hypertrophic cardiomyopathy, dilated cardiomyopathy (DCM), fibrosis of the atrium, atrial fibrillation, fibrosis of the ventricle, ventricular fibrillation, myocardial fibrosis, Brugada syndrome, myocarditis, endomyocardial fibrosis, myocardial infarction, fibrotic vascular disease, hypertensive heart disease, arrhythmogenic right ventricular cardiomyopathy (ARVC), tubulointerstitial and glomerular fibrosis, atherosclerosis, varicose veins, cerebral infarcts; neurological conditions such as gliosis and Alzheimer's disease; muscular dystrophy such as Duchenne muscular dystrophy (DMD) or Becker's muscular dystrophy (BMD); gastrointestinal conditions such as Chron's disease, microscopic colitis and primary sclerosing cholangitis (PSC); skin conditions such as scleroderma, nephrogenic systemic fibrosis and cutis keloid; arthrofibrosis; Dupuytren's contracture; mediastinal fibrosis; retroperitoneal fibrosis; myelofibrosis; Peyronie's disease; adhesive capsulitis; kidney disease (e.g., renal fibrosis, nephritic syndrome, Alport's syndrome, HIV associated nephropathy, polycystic kidney disease, Fabry's disease, diabetic nephropathy, chronic glomerulonephritis, nephritis associated with systemic lupus); progressive systemic sclerosis (PSS); chronic graft versus host disease; arthritis; fibrotic pre-neoplastic and fibrotic neoplastic disease; and fibrosis induced by chemical or environmental insult (e.g., cancer chemotherapy, pesticides, radiation/cancer radiotherapy).

It will be appreciated that the many of the diseases/conditions listed above are interrelated. For example, fibrosis of the ventricle may occur post myocardial infarction, and is associated with DCM, HCM and myocarditis.

### Fibrosis of the eye

In some embodiments, the invention relates to methods, combination for uses in methods, or the use of a composition in methods of treating or preventing fibrosis in the eye. Fibrosis of the eye is described in detail in Friedlander, Fibrosis and diseases of the eye, J Clin Invest. 2007 Mar 1; 117(3): 576-586.

Fibrosis of the eye may result from a mechanical wound or various metabolic malfunctions, including responses to inflammation, ischemia, and degenerative disease. Fibrosis of the eye may refer to wound-healing or disease events in the anterior segment of the eye, or within the posterior (i.e. retinal) segment of the eye.

Fibrosis of the eye may be a retinal, epiretinal or subretinal fibrosis. It may be a result of or be associated with an ischemic retinopathy. The fibrosis of the eye may be a fibrosis associated with ischemia and/or neovascularisation on the surface of the retina. The fibrosis of the eye may be associated with subretinal neovascularisation, e.g. neovascularization originating from the choriocapillaris. Fibrosis may be associated with fibrovascular scarring, e.g. of the retina. Fibrosis of the eye may be associated with choroidal neovascularization (CNV).

Fibrosis of the eye may also occur in the anterior segment of the eye. For example, fibrosis may affect the cornea, or the trabecular meshwork (the tracts through which the intraocular fluid leaves the eye). Fibrosis may be characterised by fibrovascular growths on the surface of the cornea, for example pingueculae and pterygia.

Fibrosis of the eye may be associated with a disease/disorder such as an inflammatory or ischemic disorder. Such diseases/disorders include Grave's opthalmopathy, epiretinal fibrosis, retinal fibrosis, idiopathic premacular fibrosis, subretinal fibrosis (e.g. associated with retinal detachment or macular degeneration (e.g. wet age-related macular degeneration (AMD)), diabetic retinopathy, glaucoma, geographic atrophy (dry age-related macular degeneration (AMD)), corneal fibrosis, post-surgical fibrosis (e.g. of the posterior capsule following cataract surgery, or of the bleb following trabeculectomy for glaucoma), conjunctival fibrosis, or subconjunctival fibrosis.

Treatment, prevention or alleviation of fibrosis according to the present invention may be of fibrosis that is associated with an upregulation of IL-11, e.g. an upregulation of IL-11 in cells or tissue in which the fibrosis occurs or may occur, or upregulation of extracellular IL-11 or IL-11R.

Treatment or alleviation of fibrosis may be effective to prevent progression of the fibrosis, e.g. to prevent worsening of the condition or to slow the rate of development of the fibrosis. In some embodiments treatment or alleviation may lead to an improvement in the fibrosis, e.g. a reduction in the amount of deposited collagen fibres.

Prevention of fibrosis may refer to prevention of a worsening of the condition or prevention of the development of fibrosis, e.g. preventing an early stage fibrosis developing to a later, chronic, stage.

### Antagonists of IL-11 mediated signalling

Aspects of the present disclosure involve antagonism (i.e. inhibition) of IL-11-mediated signalling.

Herein, 'inhibition' refers to a reduction, decrease or lessening relative to a control condition. For example, inhibition of the action of IL-11 by an antagonist of IL-11-mediated signalling refers to a reduction, decrease or lessening of the extent/degree of IL-11-mediated signalling in the absence of the agent, and/or in the presence of an appropriate control agent.

Inhibition may herein also be referred to as neutralisation or antagonism. An antagonist of IL-11-mediated signalling (e.g. an antagonist of an activity mediated by IL-11 or an IL-11-containing complex) may be said to be a 'neutralising' or 'antagonist' agent with respect to the relevant function or process. For example, an agent which is capable of inhibiting IL-11-mediated signalling may be referred to as an agent which is capable of neutralising IL-11-mediated signalling, or may be referred to as an antagonist of IL-11-mediated signalling.

The IL-11 signalling pathway offers multiple routes for inhibition of IL-11 signalling. An antagonist of IL-11-mediated signalling may inhibit the action of IL-11 e.g. through inhibiting the action of one or more factors involved in, or necessary for, signalling through a receptor for IL-11.

For example, inhibition of IL-11 signalling may be achieved by disrupting interaction between IL-11 (or an IL-11 containing complex, e.g. a complex of IL-11 and IL-11Rα) and a receptor for IL-11 (e.g. IL-11Rα, a receptor complex comprising IL-11Rα, gp130 or a receptor complex comprising IL-11Rα and gp130). In some examples, inhibition of IL-11-mediated signalling is achieved by inhibiting the gene or protein expression of one or more of e.g. IL-11, IL-11Rα and gp130.

In embodiments, inhibition of IL-11-mediated signalling is achieved by disrupting IL-11-mediated *cis* signalling but not disrupting IL-11-mediated *trans* signalling, e.g. inhibition of IL-11-mediated signalling is achieved by inhibiting gp130-mediated *cis* complexes involving membrane bound IL-11Rα . In embodiments, inhibition of IL-11-mediated signalling is achieved by disrupting IL-11-mediated *trans* signalling but not disrupting IL-11-mediated *cis* signalling, i.e. inhibition of IL-11-mediated signalling is achieved by inhibiting gp130-mediated *trans* signalling complexes such as IL-11 bound to soluble IL-11Rα or IL-6 bound to soluble IL-6R. In embodiments, inhibition of IL-11-mediated signalling is achieved by disrupting IL-11-mediated *cis* signalling and IL-11-mediated *trans* signalling. Any agent as described herein may be used to inhibit IL-11-mediated *cis* and/or *trans* signalling.

In other examples, inhibition of IL-11 signalling may be achieved by disrupting signalling pathways downstream of IL-11/IL-11Rα/gp130.

In some examples, the methods of the present disclosure employ agents capable of inhibiting JAK/STAT signalling. In some examples, agents capable of inhibiting JAK/STAT signalling are capable of inhibiting the action of JAK1, JAK2, JAK3, TYK2, STAT1, STAT2, STAT3, STAT4, STAT5A, STAT5B and/or STAT6. For example, agents may be capable of inhibiting activation of JAK/STAT proteins, inhibiting interaction of JAK or STAT proteins with cell surface receptors e.g. IL-11Rα or gp130, inhibiting phosphorylation of JAK proteins, inhibiting interaction between JAK and STAT proteins, inhibiting phosphorylation of STAT proteins, inhibiting dimerization of STAT proteins, inhibiting translocation of STAT proteins to the cell nucleus, inhibiting binding of STAT proteins to DNA, and/or promoting degradation of JAK and/or STAT proteins. In some examples, a JAK/STAT inhibitor is selected from Ruxolitinib (Jakafi/Jakavi; Incyte), Tofacitinib (Xeljanz/Jakvinus; NIH/Pfizer), Oclacitinib (Apoquel), Baricitinib (Olumiant; Incyte/Eli Lilly), Filgotinib (G-146034/GLPG-0634; Galapagos NV), Gandotinib (LY-2784544; Eli Lilly), Lestaurtinib (CEP-701; Teva), Momelotinib (GS-0387/CYT-387; Gilead Sciences), Pacritinib (SB1518; CTI), PF-04965842 (Pfizer), Upadacitinib (ABT-494; AbbVie), Peficitinib (ASP015K/JNJ-54781532; Astellas), Fedratinib (SAR302503; Celgene), Cucurbitacin I (JSI-124) and CHZ868.

In some examples, the methods of the present disclosure employ agents capable of inhibiting MAPK/ERK signalling. In some examples, agents capable of inhibiting MAPK/ERK signalling are capable of inhibiting ERK p42/44. In some examples, an ERK inhibitor is selected from SCH772984, SC1, VX-11e and DEL-22379. In some examples, agents capable of inhibiting MAPK/ERK signalling are capable of inhibiting the action of GRB2, inhibiting the action of RAF kinase, inhibiting the action of MEK proteins, inhibiting the activation of MAP3K/MAP2K/MAPK and/or Myc, and/or inhibiting the phosphorylation of STAT proteins. In some examples, an ERK inhibitor is selected from Sorafenib (Nexavar; Bayer/Onyx), SB590885, PLX4720, XL281, RAF265 (Novartis), encorafenib (LGX818/Braftovi; Array BioPharma), dabrafenib (Tafinlar; GSK), vemurafenib (Zelboraf; Roche), cobimetinib (Cotellic; Roche), CI-1040, PD0325901, Binimetinib (MEK162/MEKTOVI; Array BioPharma), selumetinib (AZD6244; Array/AstraZeneca) and Trametinib (GSK1120212/Mekinist; Novartis).

### Binding agents

In some examples, agents capable of inhibiting IL-11-mediated signalling may bind to IL-11. In some examples, agents capable of inhibiting IL-11-mediated signalling may bind to a receptor for IL-11 (e.g. IL-11Rα, gp130, or a complex containing IL-11Rα and/or gp130). Binding of such agents may inhibit IL-11-mediated signalling by reducing/preventing the ability of IL-11 to bind to receptors for IL-11, thereby inhibiting downstream signalling. Binding of such agents may inhibit IL-11 mediated *cis* and/or *trans-*signalling by reducing/preventing the ability of IL-11 to bind to receptors for IL-11, e.g. IL-11Rα and/or gp130, thereby inhibiting downstream signalling. Agents may bind to *trans*-signalling complexes such as IL-11 and soluble IL-11Rα and inhibit gp130-mediated signalling.

Disclosed herein are agents capable of binding to IL-11/an IL-11 containing complex or a receptor for IL-11 of any kind. In some examples the agent may be an antibody, an antigen-binding fragment thereof, a polypeptide, a peptide, a nucleic acid, an oligonucleotide, an aptamer or a small molecule. The agents may be provided in isolated or purified form, or may be formulated as a pharmaceutical composition or medicament.

### Properties of IL-11 binding agents

Agents capable of binding to IL-11/an IL-11 containing complex or a receptor for IL-11 according to the present invention may exhibit one or more of the following properties:
- Specific binding to IL-11/IL-11 containing complex or a receptor for IL-11;
- Binding to IL-11/IL-11 containing complex, or a receptor for IL-11, with a K_{D} of 10µM or less, preferably one of ≤ 5µM, ≤ 1µM, ≤ 100nM, ≤10nM, ≤1nM or ≤100pM;
- Inhibition of interaction between IL-11 and IL-11Rα;
- Inhibition of interaction between IL-11 and gp130;
- Inhibition of interaction between IL-11 and IL-11Rα:gp130 receptor complex;
- Inhibition of interaction between IL-11:IL-11Rα complex and gp130;
- Inhibition of interaction between IL-11 and IL-11.

These properties can be determined by analysis of the relevant agent in a suitable assay, which may involve comparison of the performance of the agent to suitable control agents. The skilled person is able to identify an appropriate control conditions for a given assay.

For example, a suitable negative control for the analysis of the ability of a test antibody/antigen-binding fragment to bind to IL-11/an IL-11 containing complex/a receptor for IL-11 may be an antibody/antigen-binding fragment directed against a non-target protein (i.e. an antibody/antigen-binding fragment which is not specific for IL-11/an IL-11 containing complex/a receptor for IL-11). A suitable positive control may be a known, validated (e.g. commercially available) IL-11- or IL-11 receptor-binding antibody. Controls may be of the same isotype as the putative IL-11/IL-11 containing complex/IL-11 receptor-binding antibody/antigen-binding fragment being analysed, and may e.g. have the same constant regions.

The agent is capable of binding specifically to IL-11 or an IL-11 containing complex, or a receptor for IL-11 (e.g. IL-11Rα, gp130, or a complex containing IL-11Rα and/or gp130). An agent which specifically binds to a given target molecule preferably binds the target with greater affinity, and/or with greater duration than it binds to other, non-target molecules.

In some embodiments the agent may bind to IL-11 or an IL-11 containing complex with greater affinity than the affinity of binding to one or more other members of the IL-6 cytokine family (e.g. IL-6, leukemia inhibitory factor (LIF), oncostatin M (OSM), cardiotrophin-1 (CT-1), ciliary neurotrophic factor (CNTF), and cardiotrophin-like cytokine (CLC)). In some embodiments the agent may bind to a receptor for IL-11 (e.g. IL-11Rα, gp130, or a complex containing IL-11Rα and/or gp130) with greater affinity than the affinity of binding to one or more other members of the IL-6 receptor family. In some embodiments the agent may bind with greater affinity to IL-11Rα than the affinity of binding to one or more of IL-6Rα, leukemia inhibitory factor receptor (LIFR), oncostatin M receptor (OSMR) and ciliary neurotrophic factor receptor alpha (CNTFRα).

In some embodiments, the extent of binding of a binding agent to an non-target is less than about 10% of the binding of the agent to the target as measured, e.g., by ELISA, SPR, Bio-Layer Interferometry (BLI), MicroScale Thermophoresis (MST), or by a radioimmunoassay (RIA). Alternatively, the binding specificity may be reflected in terms of binding affinity, where the binding agent binds to IL-11, an IL-11 containing complex or a receptor for IL-11 with a K_{D} that is at least 0.1 order of magnitude (i.e. 0.1 x 10ⁿ, where n is an integer representing the order of magnitude) greater than the K_{D} towards another, non-target molecule. This may optionally be one of at least 0.2, 0.3, 0.4, 0.5, 0.6, 0.7, 0.8, 0.9, 1.0, 1.5, or 2.0.

Binding affinity for a given binding agent for its target is often described in terms of its dissociation constant (K_{D}). Binding affinity can be measured by methods known in the art, such as by ELISA, Surface Plasmon Resonance (SPR; see e.g. Hearty et al., Methods Mol Biol (2012) 907:411-442; or Rich et al., Anal Biochem. 2008 Feb 1; 373(1):112-20), Bio-Layer Interferometry (see e.g. Lad et al., (2015) J Biomol Screen 20(4): 498-507; or Concepcion et al., Comb Chem High Throughput Screen. 2009 Sep; 12(8):791-800), MicroScale Thermophoresis (MST) analysis (see e.g. Jerabek-Willemsen et al., Assay Drug Dev Technol. 2011 Aug; 9(4): 342-353), or by a radiolabelled antigen binding assay (RIA).

In some embodiments, the agent is capable of binding to IL-11 or an IL-11 containing complex, or a receptor for IL-11 with a K_{D} of 50 µM or less, preferably one of ≤10 µM, ≤5 µM, ≤4 µM, ≤3 µM, ≤2 µM, ≤1 µM, ≤500 nM, ≤100 nM, ≤75 nM, ≤50 nM, ≤40 nM, ≤30 nM, ≤20 nM, ≤15 nM, ≤12.5 nM, ≤10 nM, ≤9 nM, ≤8 nM, ≤7 nM, ≤6 nM, ≤5 nM, ≤4 nM ≤3 nM, ≤2 nM, ≤1nM, ≤500 pM, ≤400 pM, ≤300 pM, ≤200 pM, or ≤100 pM.

In some embodiments, the agent binds to IL-11, an IL-11 containing complex or a receptor for IL-11 with an affinity of binding (e.g. as determined by ELISA) of EC50 = 10,000 ng/ml or less, preferably one of ≤5,000 ng/ml, ≤1000 ng/ml, ≤900 ng/ml, ≤800 ng/ml, ≤700 ng/ml, ≤600 ng/ml, ≤500 ng/ml, ≤400 ng/ml, ≤300 ng/ml, ≤200 ng/ml, ≤100 ng/ml, ≤90 ng/ml, ≤80 ng/ml, ≤70 ng/ml, ≤60 ng/ml, ≤50 ng/ml, ≤40 ng/ml, ≤30 ng/ml, ≤20 ng/ml, ≤15 ng/ml, ≤10 ng/ml, ≤7.5 ng/ml, ≤5 ng/ml, ≤2.5 ng/ml, or ≤1ng/ml. Such ELISAs can be performed e.g. as described in Antibody Engineering, vol. 1 (2nd Edn), Springer Protocols, Springer (2010), Part V, pp657-665.

In some embodiments, the agent binds to IL-11 or an IL-11-containing complex in a region which is important for binding to a receptor for the IL-11 or IL-11-containing complex, e.g. gp130 or IL-11 Rα, and thereby inhibits interaction between IL-11 or an IL-11-containing complex and a receptor for IL-11, and/or signalling through the receptor. In some embodiments, the agent binds to a receptor for IL-11 in a region which is important for binding to IL-11 or an IL-11-containing complex, and thereby inhibits interaction between IL-11 or an IL-11-containing complex and a receptor for IL-11, and/or signalling through the receptor.

The ability of a given binding agent (e.g. an agent capable of binding IL-11/an IL-11 containing complex or a receptor for IL-11) to inhibit interaction between two proteins can be determined for example by analysis of interaction in the presence of, or following incubation of one or both of the interaction partners with, the binding agent. An example of a suitable assay to determine whether a given binding agent is capable of inhibiting interaction between two interaction partners is a competition ELISA.

An binding agent which is capable of inhibiting a given interaction (e.g. between IL-11 and IL-11Rα, or between IL-11 and gp130, or between IL-11 and IL-11Rα:gp130, or between IL-11:IL-11Rα and gp130) is identified by the observation of a reduction/decrease in the level of interaction between the interaction partners in the presence of - or following incubation of one or both of the interaction partners with - the binding agent, as compared to the level of interaction in the absence of the binding agent (or in the presence of an appropriate control binding agent). Suitable analysis can be performed *in vitro,* e.g. using recombinant interaction partners or using cells expressing the interaction partners. Cells expressing interaction partners may do so endogenously, or may do so from nucleic acid introduced into the cell. For the purposes of such assays, one or both of the interaction partners and/or the binding agent may be labelled or used in conjunction with a detectable entity for the purposes of detecting and/or measuring the level of interaction. For example, the agent may be labelled with a radioactive atom or a coloured molecule or a fluorescent molecule or a molecule which can be readily detected in any other way. Suitable detectable molecules include fluorescent proteins, luciferase, enzyme substrates, and radiolabels. The binding agent may be directly labelled with a detectable label or it may be indirectly labelled. For example, the binding agent may be unlabelled, and detected by another binding agent which is itself labelled. Alternatively, the second binding agent may have bound to it biotin and binding of labelled streptavidin to the biotin may be used to indirectly label the first binding agent.

Ability of a binding agent to inhibit interaction between two binding partners can also be determined by analysis of the downstream functional consequences of such interaction, e.g. IL-11-mediated signalling. For example, downstream functional consequences of interaction between IL-11 and IL-11Rα:gp130 or between IL-11:IL-11Rα and gp130 may include e.g. a process mediated by IL-11, myofibroblast generation from fibroblasts, proliferation or migration by secretory smooth muscle cells (SMCs), or gene/protein expression of e.g. collagen or IL-11.

The ability of a binding agent to inhibit interaction between IL-11 or an IL-11 containing complex and a receptor for IL-11 can, for example, be analysed by stimulating fibroblasts with TGFβ1, incubating the cells in the presence of the binding agent and analysing the proportion of cells having αSMA-positive phenotype after a defined period of time. In such examples, inhibition of interaction between IL-11 or an IL-11 containing complex and a receptor for IL-11 can be identified by observation of a lower proportion of cells having an αSMA-positive phenotype as compared to positive control condition in which cells are treated with TGFβ1 in the absence of the binding agent (or in the presence of an appropriate control binding agent), or in the presence of an appropriate control binding agent. Such assays are also suitable for analysing the ability of a binding agent to inhibit IL-11-mediated signalling. Inhibition of interaction between IL-11 or an IL-11 containing complex and a receptor for IL-11 can also be analysed using ³H-thymidine incorporation and/or Ba/F3 cell proliferation assays such as those described in e.g. Curtis et al. Blood, 1997, 90(11) and Karpovich et al. Mol. Hum. Reprod. 2003 9(2): 75-80. Ba/F3 cells co-express IL-11Rα and gp130.

In some embodiments, the binding agent may be capable of inhibiting interaction between IL-11 and IL-11Rα to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of interaction between IL-11 and IL-11 Rα in the absence of the binding agent (or in the presence of an appropriate control binding agent). In some embodiments, the binding agent may be capable of inhibiting interaction between IL-11 and IL-11Rα to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of interaction between IL-11 and IL-11 Rα in the absence of the binding agent (or in the presence of an appropriate control binding agent).

In some embodiments, the binding agent may be capable of inhibiting interaction between IL-11 and gp130 to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of interaction between IL-11 and gp130 in the absence of the binding agent (or in the presence of an appropriate control binding agent). In some embodiments, the binding agent may be capable of inhibiting interaction between IL-11 and gp130 to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of interaction between IL-11 and gp130 in the absence of the binding agent (or in the presence of an appropriate control binding agent).

In some embodiments, the binding agent may be capable of inhibiting interaction between IL-11 and IL-11Rα:gp130 to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of interaction between IL-11 and IL-11 Rα:gp130 in the absence of the binding agent (or in the presence of an appropriate control binding agent). In some embodiments, the binding agent may be capable of inhibiting interaction between IL-11 and IL-11 Rα:gp130 to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55times, ≤0.5 times, ≤0.45 times, ≤0.4 times, ≤0.35 times, ≤0.3 times, ≤0.25 times, ≤0.2 times, ≤0.15 times, ≤0.1 times the level of interaction between IL-11 and IL-11 Rα:gp130 in the absence of the binding agent (or in the presence of an appropriate control binding agent).

In some embodiments, the binding agent may be capable of inhibiting interaction between IL-11:IL-11Rα complex and gp130 to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of interaction between IL-11:IL-11Rα complex and gp130 in the absence of the binding agent (or in the presence of an appropriate control binding agent). In some embodiments, the binding agent is capable of inhibiting interaction between IL-11:IL-11Rα complex and gp130 to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of interaction between IL-11:IL-11Rα complex and gp130 in the absence of the binding agent.

In some embodiments, the binding agent may be capable of inhibiting interaction between IL-11 and IL-11 to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of interaction between IL-11 and IL-11 in the absence of the binding agent (or in the presence of an appropriate control binding agent). In some embodiments, the binding agent is capable of inhibiting interaction between IL-11 and IL-11 to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of interaction between IL-11 and IL-11 in the absence of the binding agent.

### Antibodies and antigen-binding fragments

An agent capable of binding to IL-11/an IL-11 containing complex or a receptor for IL-11 is an antibody, or an antigen-binding fragment thereof. In some examples, an agent capable of binding to IL-11/an IL-11 containing complex or a receptor for IL-11 is a polypeptide, e.g. a decoy receptor molecule. In some examples, an agent capable of binding to IL-11/an IL-11 containing complex or a receptor for IL-11 may be an aptamer.

An agent capable of binding to IL-11/an IL-11 containing complex or a receptor for IL-11 is an antibody, or an antigen-binding fragment thereof. Antibodies capable of binding to IL-11 include e.g. monoclonal mouse anti-human IL-11 antibody clone #22626; Catalog No. MAB218 (R&D Systems, MN, USA), used e.g. in Bockhorn et al. Nat. Commun. (2013) 4(0):1393, clone 6D9A (Abbiotec), clone KT8 (Abbiotec), clone M3103F11 (BioLegend), clone 1F1 (Abnova Corporation), clone 3C6 (Abnova Corporation), clone GF1 (LifeSpan Biosciences), clone 13455 (Source BioScience) and anti-IL-11 antibodies disclosed in US 2009/0202533 A1, WO 99/59608 A2 and WO 2018/109174 A2.

Antibodies capable of binding to IL-11Rα include e.g. monoclonal antibody clone 025 (Sino Biological), clone EPR5446 (Abcam), clone 473143 (R & D Systems), clones 8E2 and 8E4 described in US 2014/0219919 A1, the monoclonal antibodies described in Blanc et al (J. Immunol Methods. 2000 Jul 31;241(1-2);43-59), antibodies disclosed in WO 2014121325 A1 and US 2013/0302277 A1, and anti-IL-11Rα antibodies disclosed in US 2009/0202533 A1, WO 99/59608 A2 and WO 2018/109170 A2.

The antibodies/fragments may be antagonist antibodies/fragments that inhibit or reduce a biological activity of IL-11. The antibodies/fragments may be neutralising antibodies that neutralise the biological effect of IL-11, e.g. its ability to stimulate productive signalling via an IL-11 receptor. Neutralising activity may be measured by ability to neutralise IL-11 induced proliferation in the T11 mouse plasmacytoma cell line (Nordan, R. P. et al. (1987) J. Immunol. 139:813).

### Decoy receptors

Peptide or polypeptide based agents capable of binding to IL-11 or IL-11 containing complexes may be based on the IL-11 receptor, e.g. an IL-11 binding fragment of an IL-11 receptor.

In some examples, the binding agent may comprise an IL-11-binding fragment of the IL-11 Rα chain, and may preferably be soluble and/or exclude one or more, or all, of the transmembrane domain(s). In some examples, the binding agent may comprise an IL-11-binding fragment of gp130, and may preferably be soluble and/or exclude one or more, or all, of the transmembrane domain(s). Such molecules may be described as decoy receptors. Binding of such agents may inhibit IL-11 mediated *cis* and/or *trans-*signalling by reducing/preventing the ability of IL-11 to bind to receptors for IL-11, e.g. IL-11Rα or gp130, thereby inhibiting downstream signalling.

Curtis et al (Blood 1997 Dec 1;90 (11):4403-12) report that a soluble murine IL-11 receptor alpha chain (sIL-11R) was capable of antagonizing the activity of IL-11 when tested on cells expressing the transmembrane IL-11R and gp130. They proposed that the observed IL-11 antagonism by the sIL-11R depends on limiting numbers of gp130 molecules on cells already expressing the transmembrane IL-11R.

The use of soluble decoy receptors as the basis for inhibition of signal transduction and therapeutic intervention has also been reported for other signalling molecule:receptor pairs, e.g. VEGF and the VEGF receptor (De-Chao Yu et al., Molecular Therapy (2012); 20 5, 938-947; Konner and Dupont Clin Colorectal Cancer 2004 Oct;4 Suppl 2:S81-5).

As such, in some examples a binding agent may be a decoy receptor, e.g. a soluble receptor for IL-11 and/or IL-11 containing complexes. Competition for IL-11 and/or IL-11 containing complexes provided by a decoy receptor has been reported to lead to IL-11 antagonist action (Curtis et al., *supra*). Decoy IL-11 receptors are also described in WO 2017/103108 A1 and WO 2018/109168 A1.

Decoy IL-11 receptors preferably bind IL-11 and/or IL-11 containing complexes, and thereby make these species unavailable for binding to gp130, IL-11Rα and/or gp130:IL-11Rα receptors. As such, they act as 'decoy' receptors for IL-11 and IL-11 containing complexes, much in the same way that etanercept acts as a decoy receptor for TNFα. IL-11-mediated signalling is reduced as compared to the level of signalling in the absence of the decoy receptor.

Decoy IL-11 receptors preferably bind to IL-11 through one or more cytokine binding modules (CBMs). The CBMs are, or are derived from or homologous to, the CBMs of naturally occurring receptor molecules for IL-11. For example, decoy IL-11 receptors may comprise, or consist of, one or more CBMs which are from, are derived from or homologous to the CBM of gp130 and/or IL-11Rα.

In some examples, a decoy IL-11 receptor may comprise, or consist of, an amino acid sequence corresponding to the cytokine binding module of gp130. In some examples, a decoy IL-11 receptor may comprise an amino acid sequence corresponding to the cytokine binding module of IL-11Rα. Herein, an amino acid sequence which 'corresponds' to a reference region or sequence of a given peptide/polypeptide has at least 60%, e.g. one of at least 65%, 70%, 75%, 80%, 85%, 90%, 91%, 92%, 93%, 94%, 95%, 96%, 97%, 98% or 99% sequence identity to the amino acid sequence of the reference region/sequence.

In some examples a decoy receptor may be able to bind IL-11, e.g. with binding affinity of at least 100µM or less, optionally one of 10µM or less, 1µM or less, 100nM or less, or about 1 to 100nM. In some examples a decoy receptor may comprise all or part of the IL-11 binding domain and may optionally lack all or part of the transmembrane domains. The decoy receptor may optionally be fused to an immunoglobulin constant region, e.g. IgG Fc region.

### Inhibitors

The present disclosure contemplates the use of inhibitor molecules capable of binding to one or more of IL-11, an IL-11 containing complex, IL-11Rα, gp130, or a complex containing IL-11Rα and/or gp130, and inhibiting IL-11 mediated signalling.

In some examples the agent is a peptide- or polypeptide-based binding agent based on IL-11, e.g. mutant, variant or binding fragment of IL-11. Suitable peptide or polypeptide based agents may bind to a receptor for IL-11 (e.g. IL-11Rα, gp130, or a complex containing IL-11Rα and/or gp130) in a manner that does not lead to initiation of signal transduction, or which produces sub-optimal signalling. IL-11 mutants of this kind may act as competitive inhibitors of endogenous IL-11.

For example, W147A is an IL-11 antagonist in which the amino acid 147 is mutated from a tryptophan to an alanine, which destroys the so-called 'site III' of IL-11. This mutant can bind to IL-11Rα, but engagement of the gp130 homodimer fails, resulting in efficient blockade of IL-11 signalling (Underhill-Day et al., 2003; Endocrinology 2003 Aug;144(8):3406-14). Lee et al (Am J respire Cell Mol Biol. 2008 Dec; 39(6):739-746) also report the generation of an IL-11 antagonist mutant (a "mutein") capable of specifically inhibiting the binding of IL-11 to IL-11Rα. IL-11 muteins are also described in WO 2009/052588 A1.

Menkhorst et al (Biology of Reproduction May 1, 2009 vol.80 no.5 920-927) describe a PEGylated IL-11 antagonist, PEGIL11A (CSL Limited, Parkvill, Victoria, Australia) which is effective to inhibit IL-11 action in female mice.

Pasqualini et al. Cancer (2015) 121(14):2411-2421 describe a ligand-directed, peptidomimetic drug, bone metastasis-targeting peptidomimetic-11 (BMTP-11) capable of binding to IL-11Rα.

In some examples a binding agent capable of binding to a receptor for IL-11 may be provided in the form of a small molecule inhibitor of one of IL-11Rα, gp130, or a complex containing IL-11Rα and/or gp130. In some examples a binding agent may be provided in the form of a small molecule inhibitor of IL-11 or an IL-11 containing complex, e.g. IL-11 inhibitor described in Lay et al., Int. J. Oncol. (2012); 41(2): 759-764.

### Aptamers

In some examples, an agent capable of binding to IL-11/an IL-11 containing complex or a receptor for IL-11 (e.g. IL-11Rα, gp130, or a complex containing IL-11Rα and/or gp130) is an aptamer.

### Agents capable of reducing expression of IL-11 or an IL-11 receptor

In aspects of the present disclosure the antagonist of IL-11-mediated signalling may be provided capable of preventing or reducing the expression of one or more of IL-11, IL-11Rα or gp130.

Expression may be gene or protein expression, and may be determined as described herein. Expression may be by a cell/tissue/organ/organ system of a subject. For example, expression may be prevented/reduced in smooth muscle cells.

Suitable agents may be of any kind, but in some examples an agent capable of preventing or reducing the expression of one or more of IL-11, IL-11Rα or gp130 may be a small molecule or an oligonucleotide.

An agent capable of preventing or reducing of the expression of one or more of IL-11, IL-11Rα or gp130 may do so e.g. through inhibiting transcription of the gene encoding IL-11, IL-11Rα or gp130, inhibiting post-transcriptional processing of RNA encoding IL-11, IL-11Rα or gp130, reducing the stability of RNA encoding IL-11, IL-11Rα or gp130, promoting degradation of RNA encoding IL-11, IL-11Rα or gp130, inhibiting post-translational processing of IL-11, IL-11Rα or gp130 polypeptide, reducing the stability of IL-11, IL-11Rα or gp130 polypeptide or promoting degradation of IL-11, IL-11Rα or gp130 polypeptide.

Taki et al. Clin Exp Immunol (1998) Apr; 112(1): 133-138 reported a reduction in the expression of IL-11 in rheumatoid synovial cells upon treatment with indomethacin, dexamethasone or interferon-gamma (IFNy).

The present disclosure contemplates the use of antisense nucleic acid to prevent/reduce expression of IL-11, IL-11Rα or gp130. In some examples, an agent capable of preventing or reducing the expression of IL-11, IL-11Rα or gp130 may cause reduced expression by RNA interference (RNAi).

In some examples, the agent may be an inhibitory nucleic acid, such as antisense or small interfering RNA, including but not limited to shRNA, dsRNA, miRNA or siRNA.

In some examples the inhibitory nucleic acid is provided in a vector. For example, in some examples the agent may be a lentiviral vector encoding shRNA for one or more of IL-11, IL-11Rα or gp130.

In view of the known nucleic acid sequences for IL-11, IL-11Rα and gp130 (e.g. the known mRNA sequences available from GenBank under Accession No.s: BC012506.1 GI:15341754 (human IL-11), BC134354.1 GI:126632002 (mouse IL-11), AF347935.1 GI:13549072 (rat IL-11), NM_001142784.2 GI:391353394 (human IL-11Rα), NM_001163401.1 GI:254281268 (mouse IL-11Rα), NM_139116.1 GI:20806172 (rat IL-11Rα), NM_001190981.1 GI:300244534 (human gp130), NM_010560.3 GI:225007624 (mouse gp130), NM_001008725.3 GI:300244570 (rat gp130)) oligonucleotides may be designed to repress or silence the expression of IL-11, IL-11Rα or gp130.

Such oligonucleotides may have any length, but may preferably be short, e.g. less than 100 nucleotides, e.g. 10-40 nucleotides, or 20-50 nucleotides, and may comprise a nucleotide sequence having complete- or near- complementarity (e.g. 80%, 85%, 90%, 91%, 92%, 93%, 94%, 95%, 96%, 97%, 98%, 99% or 100% complementarity) to a sequence of nucleotides of corresponding length in the target oligonucleotide, e.g. the IL-11, IL-11Rα or gp130 mRNA. The complementary region of the nucleotide sequence may have any length, but is preferably at least 5, and optionally no more than 50, nucleotides long, e.g. one of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides.

Repression of expression of IL-11, IL-11Rα or gp130 will preferably result in a decrease in the quantity of IL-11, IL-11Rα or gp130 expressed by a cell/tissue/organ/organ system/subject. For example, in a given cell the repression of IL-11, IL-11Rα or gp130 by administration of a suitable nucleic acid will result in a decrease in the quantity of IL-11, IL-11Rα or gp130 expressed by that cell relative to an untreated cell. Repression may be partial. Preferred degrees of repression are at least 50%, more preferably one of at least 60%, 70%, 80%, 85% or 90%. A level of repression between 90% and 100% is considered a 'silencing' of expression or function.

Another alternative is the expression of a short hairpin RNA molecule (shRNA) in the cell. Preferably, the shRNA molecule comprises a partial sequence of IL-11, IL-11Rα or gp130. Preferably, the shRNA sequence is between 40 and 100 bases in length, more preferably between 40 and 70 bases in length. The stem of the hairpin is preferably between 19 and 30 base pairs in length. The stem may contain G-U pairings to stabilise the hairpin structure.

siRNA molecules, longer dsRNA molecules or miRNA molecules may be made recombinantly by transcription of a nucleic acid sequence, preferably contained within a vector. Preferably, the siRNA molecule, longer dsRNA molecule or miRNA molecule comprises a partial sequence of IL-11, IL-11Rα or gp130.

In one example, the siRNA, longer dsRNA or miRNA is produced endogenously (within a cell) by transcription from a vector. Suitable vectors may be oligonucleotide vectors configured to express the oligonucleotide agent capable of IL-11, IL-11Rα or gp130 repression.

In other examples a vector may be configured to assist delivery of the therapeutic oligonucleotide to the site at which repression of IL-11, IL-11Rα or gp130 expression is required.

In other examples, the disclosure provides nucleic acid that is capable, when suitably introduced into or expressed within a mammalian, e.g. human, cell that otherwise expresses IL-11, IL-11Rα or gp130, of suppressing IL-11, IL-11Rα or gp130 expression by RNAi.

Nucleic acid sequences for IL-11, IL-11Rα and gp130 (e.g. the known mRNA sequences available from GenBank under Accession No.s: BC012506.1 GI:15341754 (human IL-11), BC134354.1 GI:126632002 (mouse IL-11), AF347935.1 GI:13549072 (rat IL-11), NM_001142784.2 GI:391353394 (human IL-11Rα), NM_001163401.1 GI:254281268 (mouse IL-11Rα), NM_139116.1 GI:20806172 (rat IL-11Rα), NM_001190981.1 GI:300244534 (human gp130), NM_010560.3 GI:225007624 (mouse gp130), NM_001008725.3 GI:300244570 (rat gp130)) oligonucleotides may be designed to repress or silence the expression of IL-11, IL-11Rα or gp130.

The nucleic acid may have substantial sequence identity to a portion of IL-11, IL-11Rα or gp130 mRNA, e.g. as defined in GenBank accession no. NM_000641.3 GI:391353405 (IL-11), NM_001142784.2 GI:391353394 (IL-11Rα), NM_001190981.1 GI:300244534 (gp130) or the complementary sequence to said mRNA.

The nucleic acid may be a double-stranded siRNA. (As the skilled person will appreciate, and as explained further below, a siRNA molecule may include a short 3' DNA sequence also.)

Alternatively, the nucleic acid may be a DNA (usually double-stranded DNA) which, when transcribed in a mammalian cell, yields an RNA having two complementary portions joined via a spacer, such that the RNA takes the form of a hairpin when the complementary portions hybridise with each other. In a mammalian cell, the hairpin structure may be cleaved from the molecule by the enzyme DICER, to yield two distinct, but hybridised, RNA molecules.

In some examples, the nucleic acid is generally targeted to the sequence of one of SEQ ID NOs 4 to 7 (IL-11) or to one of SEQ ID NOs 8 to 11 (IL-11Rα).

Only single-stranded (i.e. non self-hybridised) regions of an mRNA transcript are expected to be suitable targets for RNAi. It is therefore proposed that other sequences very close in the IL-11 or IL-11Rα mRNA transcript to the sequence represented by one of SEQ ID NOs 4 to 7 or 8 to 11 may also be suitable targets for RNAi. Such target sequences are preferably 15-21 nucleotides in length and preferably overlap one of SEQ ID NOs 4 to 7 or 8 to 11 by at least 1, 2, 3, 4, 5, 6, 7, 8, 9, 10, 11, 12, 13, 14, 15, 16, 17, 18 or all 19 nucleotides (at either end of one of SEQ ID NOs 4 to 7 or 8 to 11).

Accordingly, the disclosure provides nucleic acid that is capable, when suitably introduced into or expressed within a mammalian cell that otherwise expresses IL-11 or IL-11 Rα, of suppressing IL-11 or IL-11Rα expression by RNAi, wherein the nucleic acid is generally targeted to the sequence of one of SEQ ID NOs 4 to 7 or 8 to 11.

By "generally targeted" the nucleic acid may target a sequence that overlaps with SEQ ID NOs 4 to 7 or 8 to 11. In particular, the nucleic acid may target a sequence in the mRNA of human IL-11 or IL-11Rα that is slightly longer or shorter than one of SEQ ID NOs 4 to 7 or 8 to 11 (preferably from 17-23 nucleotides in length), but is otherwise identical to one of SEQ ID NOs 4 to 7 or 8 to 11.

It is expected that perfect identity/complementarity between the nucleic acid of the disclosure and the target sequence, although preferred, is not essential. Accordingly, the nucleic acid of the disclosure may include a single mismatch compared to the mRNA of IL-11 or IL-11Rα. It is expected, however, that the presence of even a single mismatch is likely to lead to reduced efficiency, so the absence of mismatches is preferred. When present, 3' overhangs may be excluded from the consideration of the number of mismatches.

In one example, the nucleic acid (herein referred to as double-stranded siRNA) includes the double-stranded RNA sequences shown in SEQ ID NOs 12 to 15. In another example, the nucleic acid (herein referred to as double-stranded siRNA) includes the double-stranded RNA sequences shown in SEQ ID NOs 16 to 19.

However, it is also expected that slightly shorter or longer sequences directed to the same region of IL-11 or IL-11Rα mRNA will also be effective. In particular, it is expected that double-stranded sequences between 17 and 23 bp in length will also be effective.

The disclosure also provides DNA that, when transcribed in a mammalian cell, yields an RNA (herein also referred to as an shRNA) having two complementary portions which are capable of self-hybridising to produce a double-stranded motif, e.g. including a sequence selected from the group consisting of SEQ ID NOs: 12 to 15 or 16 to 19 or a sequence that differs from any one of the aforementioned sequences by a single base pair substitution.

The complementary portions may be joined by a spacer, which has suitable length and sequence to allow the two complementary portions to hybridise with each other. Preferably the 5' end of the spacer (immediately 3' of the upstream complementary portion) consists of the nucleotides -UU- or -UG-, again preferably -UU- (though, again, the use of these particular dinucleotides is not essential). A suitable spacer, recommended for use in the pSuper system of OligoEngine (Seattle, Washington, USA) is UUCAAGAGA. In this and other cases, the ends of the spacer may hybridise with each other, e.g. elongating the double-stranded motif beyond the exact sequences of SEQ ID NOs 12 to 15 or 16 to 19 by a small number (e.g. 1 or 2) of base pairs.

The double-stranded siRNAs of the disclosure may be introduced into mammalian cells *in vitro* or *in vivo* using known techniques, as described below, to suppress expression of IL-11 or a receptor for IL-11.

Similarly, transcription vectors containing the DNAs of the disclosure may be introduced into tumour cells *in vitro* or *in vivo* using known techniques, as described below, for transient or stable expression of RNA, again to suppress expression of IL-11 or a receptor for IL-11.

Accordingly, the disclosure also provides a method of suppressing expression of IL-11 or a receptor for IL-11 in a mammalian, e.g. human, cell, the method comprising administering to the cell a double-stranded siRNA of the disclosure or a transcription vector of the disclosure.

Similarly, the disclosure further provides a method of treating a disease/condition in which angiogenesis are pathologically implicated, the method comprising administering to a subject a double-stranded siRNA of the disclosure or a transcription vector of the disclosure in combination with an antagonist of IL-11 signalling.

The disclosure further provides the double-stranded siRNAs of the disclosure and the transcription vectors of the disclosure, for use in a method of treatment in combination with an antagonist of IL-11 signalling.

The disclosure further provides the use of the double-stranded siRNAs of the disclosure and the transcription vectors of the disclosure in the preparation of a medicament for the treatment of a disease/condition in combination with an antagonist of IL-11 signalling.

### Inhibition of IL-11-mediated signalling

In embodiments of the present invention, agents capable of inhibiting the action of IL-11 may possess one or more of the following functional properties:
- Inhibition of signalling mediated by IL-11;
- Inhibition of signalling mediated by binding of IL-11 to IL-11 Rα:gp130 receptor complex;
- Inhibition of signalling mediated by binding of IL-11:IL-11Rα complex to gp130 (i.e. IL-11 *trans* signalling);
- Inhibition of a process mediated by IL-11;
- Inhibition of myofibroblast generation;
- Inhibition of SMC proliferation/migration;
- Inhibition of gene/protein expression of collagen or IL-11.

These properties can be determined by analysis of the relevant agent in a suitable assay, which may involve comparison of the performance of the agent to suitable control agents. The skilled person is able to identify an appropriate control conditions for a given assay.

IL-11-mediated signalling and/or processes mediated by IL-11 includes signalling mediated by fragments of IL-11 and polypeptide complexes comprising IL-11 or fragments thereof. IL-11-mediated signalling may be signalling mediated by human IL-11 and/or mouse IL-11. Signalling mediated by IL-11 may occur following binding of IL-11 or an IL-11 containing complex to a receptor to which IL-11 or said complex binds.

In some examples, an agent may be capable of inhibiting the biological activity of IL-11 or an IL-11-containing complex.

In some examples, the agent is an antagonist of one or more signalling pathways which are activated by signal transduction through receptors comprising IL-11Rα and/or gp130, e.g. IL-11 Rα:gp130. In some examples, the agent is capable of inhibiting signalling through one or more immune receptor complexes comprising IL-11 Rα and/or gp130, e.g. IL-11 Rα:gp130. In various aspects of the present disclosure, an agent provided herein is capable of inhibiting IL-11-mediated *cis* and/or *trans* signalling.

In some embodiments, the agent may be capable of inhibiting IL-11-mediated signalling to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of signalling in the absence of the agent (or in the presence of an appropriate control agent). In some embodiments, the agent is capable of reducing IL-11-mediated signalling to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of signalling in the absence of the agent (or in the presence of an appropriate control agent).

In some embodiments, the IL-11-mediated signalling may be signalling mediated by binding of IL-11 to IL-11Rα:gp130 receptor. Such signalling can be analysed e.g. by treating cells expressing IL-11Rα and gp130 with IL-11, or by stimulating IL-11 production in cells which express IL-11Rα and gp130.

The IC₅₀ for agent for inhibition of IL-11-mediated signalling may be determined, e.g. by culturing Ba/F3 cells expressing IL-11Rα and gp130 in the presence of human IL-11 and the agent, and measuring ³H-thymidine incorporation into DNA. In some embodiments, the agent may exhibit an IC₅₀ of 10 µg/ml or less, preferably one of ≤ 5 µg/ml, ≤ 4 µg/ml, ≤ 3.5 µg/ml, ≤ 3 µg/ml, ≤ 2 µg/ml, ≤ 1 µg/ml, ≤ 0.9 µg/ml, ≤ 0.8 µg/ml, ≤ 0.7 µg/ml, ≤ 0.6 µg/ml, or ≤ 0.5 µg/ml in such an assay.

In some embodiments, the IL-11-mediated signalling may be signalling mediated by binding of IL-11:IL-11Rα complex to gp130. In some embodiments, the IL-11:IL-11Rα complex may be soluble, e.g. complex of extracellular domain of IL-11Rα and IL-11, or complex of soluble IL-11Rα isoform/fragment, and IL-11. In some embodiments, the soluble IL-11Rα is a soluble (secreted) isoform of IL-11Rα, or is the liberated product of proteolytic cleavage of the extracellular domain of cell membrane bound IL-11 Rα.

In some embodiments, the IL-11:IL-11Rα complex may be cell-bound, e.g. complex of cell-membrane bound IL-11Rα and IL-11. Signalling mediated by binding of IL-11:IL-11Rα complex to gp130 can be analysed by treating cells expressing gp130 with IL-11:IL-11Rα complex, e.g. recombinant fusion protein comprising IL-11 joined by a peptide linker to the extracellular domain of IL-11Rα (e.g. hyper IL-11 as described herein).

In some embodiments, the agent may be capable of inhibiting signalling mediated by binding of IL-11:IL-11Rα complex to gp130, and is also capable of inhibiting signalling mediated by binding of IL-11 to IL-11Rα:gp130 receptor.

In some embodiments, the agent may be capable of inhibiting a process mediated by IL-11, e.g. following stimulation with TGFβ1. Processes mediated by IL-11 include e.g. myofibroblast generation from fibroblasts, proliferation/migration of SMCs, and gene/protein expression of e.g. collagen and IL-11, and can be evaluated either *in vitro* or *in vivo.*

In some embodiments, the agent may be capable of inhibiting myofibroblast generation from fibroblasts, e.g. following exposure of the fibroblasts to profibrotic factor (e.g. TGFβ1). Myofibroblast generation from fibroblasts can be investigated by analysis for myofibroblast markers.

The fibroblasts may be derived from any tissue, including liver, lungs, kidney, heart, blood vessels, eye, skin, pancreas, spleen, bowel (e.g. large or small intestine), brain, and bone marrow. In particular embodiments, the fibroblasts may be cardiac fibroblasts (e.g. atrial fibroblasts), skin fibroblasts, lung fibroblasts, kidney fibroblasts or liver fibroblasts. Fibroblasts may be characterised by gene or protein expression of one or more of COL1A, ACTA2, prolyl-4-hydroxylase, MAS516, and FSP1. Myofibroblast markers may include one or more of increased αSMA, vimentin, palladin, cofilin or desmin (as compared to the level of expression by comparable fibroblasts (e.g. fibroblasts derived from the same tissue)).

Myofibroblast generation from fibroblasts can analysed by measuring αSMA protein expression levels using Operetta High-Content Imaging System following stimulation of the fibroblasts with TGFβ1; see e.g. WO 2017/103108 A1.

In some embodiments, the agent may be capable of inhibiting myofibroblast generation from fibroblasts to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of myofibroblast generation from fibroblasts in the absence of the agent (or in the presence of an appropriate control agent). In some embodiments, the agent is capable of reducing myofibroblast generation from fibroblasts to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of myofibroblast generation from fibroblasts in the absence of the agent (or in the presence of an appropriate control agent).

In some embodiments, the agent may be capable of inhibiting proliferation of SMCs (e.g. secretory SMCs), e.g. following stimulation with TGFβ1. SMC proliferation can be measured using e.g. ³H-thymidine incorporation, CFSE dilution or EdU incorporation assays as described herein.

In some embodiments, the agent may be capable of inhibiting proliferation of SMCs to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of proliferation in the absence of the agent (or in the presence of an appropriate control agent). In some embodiments, the agent is capable of inhibiting proliferation of SMCs to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of proliferation in the absence of the agent (or in the presence of an appropriate control agent).

In some embodiments, the agent may be capable of inhibiting migration of SMCs (e.g. secretory SMCs), e.g. following stimulation with TGFβ1. SMC migration can be measured using a scratch assay e.g. as described in Example 9 and in Liang et al., Nat Protoc. (2007) 2(2):329-33, or using a Boyden chamber assay as described in Example 9 and in Chen, Methods Mol Biol. (2005) 294:15-22.

In some embodiments, the agent may be capable of inhibiting migration of SMCs to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of migration in the absence of the agent (or in the presence of an appropriate control agent). In some embodiments, the agent is capable of inhibiting migration of SMCs to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of migration in the absence of the agent (or in the presence of an appropriate control agent).

In some embodiments, the agent may be capable of inhibiting gene/protein expression of collagen or IL-11. Gene and/or protein expression can be measured as described herein.

In some embodiments, the agent may be capable of inhibiting gene/protein expression of collagen or IL-11 to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of expression in the absence of the agent (or in the presence of an appropriate control agent). In some embodiments, the agent is capable of inhibiting gene/protein expression of collagen or IL-11 to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of expression in the absence of the agent (or in the presence of an appropriate control agent).

### Antagonists of angiogenic factors

Aspects of the present disclosure involve antagonism (i.e. inhibition) of angiogenesis, e.g. by antagonism of one or more angiogenic factors.

Herein, 'inhibition' refers to a reduction, decrease or lessening relative to a control condition. For example, inhibition of the action of an angiogenic factor by an antagonist of angiogenic signalling refers to a reduction, decrease or lessening of the extent/degree of angiogenic signalling in the absence of the agent, and/or in the presence of an appropriate control agent.

Inhibition may herein also be referred to as neutralisation or antagonism. An antagonist of angiogenic signalling (e.g. an antagonist of an activity mediated by an angiogenic factor) may be said to be a 'neutralising' or 'antagonist' agent with respect to the relevant function or process. For example, an agent which is capable of inhibiting angiogenic signalling may be referred to as an agent which is capable of neutralising angiogenic signalling, or may be referred to as an antagonist of angiogenic signalling.

Angiogenic signalling pathways offer multiple routes for inhibition of signalling. An antagonist of angiogenic signalling may inhibit the action of an angiogenic factor through inhibiting the action of one or more factors involved in, or necessary for, signalling through a receptor for that factor.

For example, inhibition may be achieved by disrupting interaction between an angiogenic factor (e.g. VEGF) and a receptor for that factor (e.g. VEGFR1, 2 or 3). In some examples, inhibition of angiogenic signalling is achieved by inhibiting the gene or protein expression of one or more of e.g. VEGF, VEGFR1, VEGFR2, and VEGFR3.

In some embodiments, antagonism (i.e. inhibition) of angiogenesis results in antagonism, inhibition or reduction of fibrosis.

### Binding agents

In some examples, agents capable of inhibiting angiogenic factor-mediated signalling may bind to an angiogenic factor. In some examples, agents capable of inhibiting angiogenic factor-mediated signalling may bind to a receptor for an angiogenic factor (e.g. VEGFR1, VEGFR2 or VEGFR3 for VEGF). Binding of such agents may inhibit angiogenic factor-mediated signalling by reducing/preventing the ability of an angiogenic factor ligand to bind to receptors, thereby inhibiting downstream signalling.

Agents capable of binding to an angiogenic factor or an interaction partner for an angiogenic factor may be of any kind, but in some examples the agent may be an antibody, an antigen-binding fragment thereof, a polypeptide, a peptide, a nucleic acid, an oligonucleotide, an aptamer or a small molecule. The agents may be provided in isolated or purified form, or may be formulated as a pharmaceutical composition or medicament.

### Properties of angiogenic factor binding agents

Agents capable of binding to an angiogenic factor/a complex comprising the angiogenic factor or an interaction partner for a given angiogenic factor according to the present invention may exhibit one or more of the following properties:
- Specific binding to an angiogenic factor/a complex comprising the angiogenic factor or an interaction partner for the angiogenic factor;
- Binding to an angiogenic factor/a complex comprising the angiogenic factor, or an interaction partner for the angiogenic factor, with a K_{D} of 10µM or less, preferably one of ≤ 5µM ≤ 1µM, ≤ 100nM, ≤10nM, ≤1nM or ≤100pM;
- Inhibition of interaction between an angiogenic factor and an interaction partner for the angiogenic factor;

These properties can be determined by analysis of the relevant agent in a suitable assay, which may involve comparison of the performance of the agent to suitable control agents. The skilled person is able to identify appropriate control conditions for a given assay. As explained herein, an interaction partner for an angiogenic factor may e.g. be a ligand for an angiogenic factor (e.g. where the angiogenic factor is a receptor, e.g. a VEGF receptor, an FGF receptor or a PDGF receptor), or may be a receptor for an angiogenic factor (e.g. where the angiogenic factor is a ligand, e.g. a VEGF, an FGF or a PDGF).

For example, a suitable negative control for the analysis of the ability of a test antibody/antigen-binding fragment to bind to an angiogenic factor/a complex comprising the angiogenic factor /an interaction partner for the angiogenic factor may be an antibody/antigen-binding fragment directed against a non-target protein (i.e. which is not specific for an angiogenic factor/a complex comprising the angiogenic factor/interaction partner for the angiogenic factor). A suitable positive control may be a known, validated (e.g. commercially available) an angiogenic factor- or interaction partner-binding antibody. Controls may be of the same isotype as the putative an angiogenic factor/a complex comprising the angiogenic factor/interaction partner-binding antibody/antigen-binding fragment being analysed, and may e.g. have the same constant regions.

In some examples, the agent may be capable of binding specifically to an angiogenic factor or a complex comprising the angiogenic factor, or an interaction partner for the angiogenic factor. An agent which specifically binds to a given target molecule preferably binds the target with greater affinity, and/or with greater duration than it binds to other, non-target molecules.

In some embodiments, the extent of binding of a binding agent to an non-target is less than about 10% of the binding of the agent to the target as measured, e.g., by ELISA, SPR, Bio-Layer Interferometry (BLI), MicroScale Thermophoresis (MST), or by a radioimmunoassay (RIA). Alternatively, the binding specificity may be reflected in terms of binding affinity, where the binding agent binds to an angiogenic factor, a complex comprising the angiogenic factor or an interaction partner for an angiogenic factor with a K_{D} that is at least 0.1 order of magnitude (i.e. 0.1 x 10ⁿ, where n is an integer representing the order of magnitude) greater than the K_{D} towards another, non-target molecule. This may optionally be one of at least 0.2, 0.3, 0.4, 0.5, 0.6, 0.7, 0.8, 0.9, 1.0, 1.5, or 2.0.

Binding affinity for a given binding agent for its target is often described in terms of its dissociation constant (K_{D}). Binding affinity can be measured by methods known in the art, such as by ELISA, Surface Plasmon Resonance (SPR; see e.g. Hearty et al., Methods Mol Biol (2012) 907:411-442; or Rich et al., Anal Biochem. 2008 Feb 1; 373(1):112-20), Bio-Layer Interferometry (see e.g. Lad et al., (2015) J Biomol Screen 20(4): 498-507; or Concepcion et al., Comb Chem High Throughput Screen. 2009 Sep; 12(8):791-800), MicroScale Thermophoresis (MST) analysis (see e.g. Jerabek-Willemsen et al., Assay Drug Dev Technol. 2011 Aug; 9(4): 342-353), or by a radiolabelled antigen binding assay (RIA).

In some embodiments, the agent capable of binding to an angiogenic factor or a complex comprising the angiogenic factor, or an interaction partner for the angiogenic factor with a K_{D} of 50 µM or less, preferably one of ≤10 µM, ≤5 µM, ≤4 µM, ≤3 µM, ≤2 µM, ≤1 µM, ≤500 nM, ≤100 nM, ≤75 nM, ≤50 nM, ≤40 nM, ≤30 nM, ≤20 nM, ≤15 nM, ≤12.5 nM, ≤10 nM, ≤9 nM, ≤8 nM, ≤7 nM, ≤6 nM, ≤5 nM, ≤4 nM ≤3 nM, ≤2 nM, ≤1 nM, ≤500 pM, ≤400 pM, ≤300 pM, ≤200 pM, or ≤100 pM.

In some embodiments, the agent binds to an angiogenic factor, a complex comprising the angiogenic factor or an interaction partner for the angiogenic factor with an affinity of binding (e.g. as determined by ELISA) of EC50 = 10,000 ng/ml or less, preferably one of ≤5,000 ng/ml, ≤1000 ng/ml, ≤900 ng/ml, ≤800 ng/ml, ≤700 ng/ml, ≤600 ng/ml, ≤500 ng/ml, ≤400 ng/ml, ≤300 ng/ml, ≤200 ng/ml, ≤100 ng/ml, ≤90 ng/ml, ≤80 ng/ml, ≤70 ng/ml, ≤60 ng/ml, ≤50 ng/ml, ≤40 ng/ml, ≤30 ng/ml, ≤20 ng/ml, ≤15 ng/ml, ≤10 ng/ml, ≤7.5 ng/ml, ≤5 ng/ml, ≤2.5 ng/ml, or ≤1 ng/ml. Such ELISAs can be performed e.g. as described in Antibody Engineering, vol. 1 (2nd Edn), Springer Protocols, Springer (2010), Part V, pp657-665.

In some examples, the agent binds to an angiogenic factor or a complex comprising the angiogenic factor in a region which is important for binding to an interaction partner for the angiogenic factor or a complex comprising the angiogenic factor, and thereby inhibits interaction between an angiogenic factor or a complex comprising the angiogenic factor complex comprising the and an interaction partner for the angiogenic factor (e.g. signalling through an angiogenic factor receptor). In some examples, the agent binds to an interaction partner for the angiogenic factor in a region which is important for binding to the angiogenic factor or a complex comprising the angiogenic factor, and thereby inhibits interaction between an angiogenic factor or a complex comprising the angiogenic factor and an interaction partner for the angiogenic factor (e.g. signalling through an angiogenic factor receptor).

The ability of a given binding agent (e.g. an agent capable of binding an angiogenic factor/a complex comprising the angiogenic factor or an interaction partner for the angiogenic factor) to inhibit interaction between two proteins can be determined for example by analysis of interaction in the presence of, or following incubation of one or both of the interaction partners with, the binding agent. An example of a suitable assay to determine whether a given binding agent is capable of inhibiting interaction between two interaction partners is a competition ELISA.

An binding agent which is capable of inhibiting a given interaction (e.g. between an angiogenic factor and an interaction partner for the angiogenic factor, such as between VEGF and one or more of VEGFR1-3) is identified by the observation of a reduction/decrease in the level of interaction between the interaction partners in the presence of - or following incubation of one or both of the interaction partners with - the binding agent, as compared to the level of interaction in the absence of the binding agent (or in the presence of an appropriate control binding agent). Suitable analysis can be performed *in vitro,* e.g. using recombinant interaction partners or using cells expressing the interaction partners. Cells expressing interaction partners may do so endogenously, or may do so from nucleic acid introduced into the cell. For the purposes of such assays, one or both of the interaction partners and/or the binding agent may be labelled or used in conjunction with a detectable entity for the purposes of detecting and/or measuring the level of interaction. For example, the agent may be labelled with a radioactive atom or a coloured molecule or a fluorescent molecule or a molecule which can be readily detected in any other way. Suitable detectable molecules include fluorescent proteins, luciferase, enzyme substrates, and radiolabels. The binding agent may be directly labelled with a detectable label or it may be indirectly labelled. For example, the binding agent may be unlabelled, and detected by another binding agent which is itself labelled. Alternatively, the second binding agent may have bound to it biotin and binding of labelled streptavidin to the biotin may be used to indirectly label the first binding agent.

Ability of a binding agent to inhibit interaction between two binding partners can also be determined by analysis of the downstream functional consequences of such interaction, e.g. an angiogenic factor-mediated signalling.

In some embodiments, the binding agent may be capable of inhibiting interaction between an angiogenic factor and an interaction partner for the angiogenic factor to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of interaction between the angiogenic factor and the interaction partner for the angiogenic factor in the absence of the binding agent (or in the presence of an appropriate control binding agent). In some embodiments, the binding agent may be capable of inhibiting interaction between an angiogenic factor and an interaction partner for the angiogenic factor to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55times, ≤0.5 times, ≤0.45 times, ≤0.4 times, ≤0.35 times, ≤0.3 times, ≤0.25 times, ≤0.2 times, ≤0.15 times, ≤0.1 times the level of interaction between the angiogenic factor and the interaction partner for the angiogenic factor in the absence of the binding agent (or in the presence of an appropriate control binding agent).

### Antibodies and antigen-binding fragments

In some examples, an agent capable of binding to an angiogenic factor or an interaction partner for the angiogenic factor is an antibody, or an antigen-binding fragment thereof. In some examples, an agent capable of binding to an angiogenic factor or an interaction partner for the angiogenic factor is a polypeptide, e.g. a decoy receptor molecule. In some examples, an agent capable of binding to an angiogenic factor or an interaction partner for the angiogenic factor may be an aptamer.

In some examples, an agent capable of binding to an angiogenic factor or an interaction partner for the angiogenic factor is an antibody, or an antigen-binding fragment thereof. Exemplary antibodies capable of binding to angiogenic factors include antibodies capable of binging VEGF, e.g. bevacizumab and ranibizumab.

The antibodies/fragments may be antagonist antibodies/fragments that inhibit or reduce a biological activity of an angiogenic factor. The antibodies/fragments may be neutralising antibodies that neutralise the biological effect of an angiogenic factor, e.g. its ability to stimulate productive signalling via an angiogenic factor receptor.

### Decoy receptors

Peptide or polypeptide based agents capable of binding to an angiogenic factor or a complex comprising the angiogenic factor may be based on an angiogenic factor receptor, e.g. an angiogenic factor-binding fragment of an angiogenic factor receptor.

In some examples, the binding agent may comprise an angiogenic factor-binding fragment of the receptor extracellular domain, and may preferably be soluble and/or exclude one or more, or all, of the transmembrane domain(s). Such molecules may be described as decoy receptors.

In some embodiments, the binding agent may comprise recombinant fusion protein of the second immunoglobulin (lg) domain of VEGFR-1 and the third Ig domain of VEGFR-2, fused to the constant region (Fc) of human IgG1. An exemplary angiogenesis decoy receptor is aflibercept (Eylea, SEQ ID NO:24).

As such, in some examples a binding agent may be a decoy receptor, e.g. a soluble receptor for an angiogenic factor and/or complexes comprising the angiogenic factor.

Decoy angiogenic factor receptors preferably bind to an angiogenic factor and/or complexes comprising the angiogenic factor, and thereby make these species unavailable for binding to their receptors. As such, they act as 'decoy' receptors, and angiogenic signalling is reduced as compared to the level of signalling in the absence of the decoy receptor.

In some embodiments a decoy receptor may be able to bind an angiogenic factor, e.g. with binding affinity of at least 100µM or less, optionally one of 10µM or less, 1µM or less, 100nM or less, or about 1 to 100nM. In some embodiments a decoy receptor may comprise all or part of the angiogenic factor binding domain and may optionally lack all or part of the transmembrane domains. The decoy receptor may optionally be fused to an immunoglobulin constant region, e.g. IgG Fc region.

### Inhibitors

The present disclosure contemplates the use of inhibitor molecules capable of binding to one or more of an angiogenic factor, a complex comprising the angiogenic factor, or an interaction partner for an angiogenic factor, and inhibiting angiogenic signalling.

In some examples the agent is a peptide or polypeptide based binding agent based on an angiogenic factor, e.g. mutant, variant or binding fragment of an angiogenic factor. Suitable peptide or polypeptide based agents may bind to a receptor for an angiogenic factor (e.g. VEGFR1, 2 and/or 3) in a manner that does not lead to initiation of signal transduction, or which produces sub-optimal signalling. Mutants of this kind may act as competitive inhibitors of endogenous angiogenic factors.

In some examples a binding agent capable of antagonism of an angiogenic factor may take the form of a small molecule inhibitor.

Small molecule inhibitors of angiogenesis may be tyrosine kinase inhibitors. Multiple small-molecule tyrosine kinase inhibitors have been developed. Given the functional redundancy of angiogenic pathway components, these drugs target multiple kinases, such as VEGFRs. They include pazopanib (Votrient^{®}), an inhibitor of VEGF, platelet-derived growth factor receptor (PDGFR), and KIT; sorafenib (Nexavar^{®}), an inhibitor of VEGFR-1, VEGFR-2, VEGFR-3, PDGFR-β, and RAF1; sunitinib (Sutent^{®}), an inhibitor of VEGFR-1, VEGFR-2, VEGFR-3, PDGFR-β, and RET; vandetanib (Caprelsa^{®}), an inhibitor of VEGFR and epidermal growth factor receptor; cabozantinib (Cometrig^{®}), an inhibitor of MET and VEGFR2; axitinib (Inlyta^{®}), an inhibitor of VEGFR-1, VEGFR-2, and VEGFR-3; vatalanib, an inhibitor of known VEGF receptors, as well as platelet-derived growth factor receptor-beta and c-kit, but is most selective for VEGFR-2; and regorafenib (Stivarga^{®}), an inhibitor of VEGFR-1, VEGFR-2, VEGFR-3, TIE2, PDGFR, fibroblast growth factor receptor, KIT, RET, and RAFf.

Other small molecule inhibitors of angiogenesis include anecortave acetate, and squalamine lactate.

### Aptamers

In some examples, an agent capable of binding to an angiogenic factor/a complex comprising the angiogenic factor or an interaction partner for the angiogenic factor is an aptamer.

An exemplary aptamer inhibitor of an angiogenic factor is the anti-VEGF aptamer pegaptanib, which is described in WO9818480 A1.

### Agents capable of reducing expression of angiogenic factor or an angiogenic factor receptor

In aspects of the present disclosure the antagonist of an angiogenic factor-mediated signalling may be provided, which is capable of preventing or reducing the expression of one or more of an angiogenic factor, or an interaction partner for the angiogenic factor.

Expression may be gene or protein expression, and may be determined as described herein. Expression may be by a cell/tissue/organ/organ system of a subject.

Suitable agents may be of any kind, but in some examples an agent capable of preventing or reducing the expression of one or more of an angiogenic factor, or an interaction partner for the angiogenic factor may be a small molecule or an oligonucleotide.

An agent capable of preventing or reducing of the expression of one or more of an angiogenic factor, or an interaction partner for the angiogenic factor may do so e.g. through inhibiting transcription of the gene encoding an angiogenic factor, or interaction partner for the angiogenic factor, inhibiting post-transcriptional processing of RNA encoding an angiogenic factor, or interaction partner for the angiogenic factor, reducing the stability of RNA encoding an angiogenic factor, or an interaction partner for the angiogenic factor, promoting degradation of RNA encoding an angiogenic factor, or an interaction partner for the angiogenic factor, inhibiting post-translational processing of an angiogenic factor, or an interaction partner for the angiogenic factor, reducing the stability of an angiogenic factor, or interaction partner for the angiogenic factor, or promoting degradation of an angiogenic factor, or an interaction partner for the angiogenic factor.

The present disclosure contemplates the use of antisense nucleic acid to prevent/reduce expression of an angiogenic factor, or an interaction partner for the angiogenic factor. In some examples, an agent capable of preventing or reducing the expression of an angiogenic factor, or an interaction partner for the angiogenic factor may cause reduced expression by RNA interference (RNAi).

In some examples, the agent may be an inhibitory nucleic acid, such as antisense or small interfering RNA, including but not limited to shRNA, dsRNA, miRNA or siRNA.

In some examples the inhibitory nucleic acid is provided in a vector. For example, in some examples the agent may be a lentiviral vector encoding shRNA for one or more of an angiogenic factor, or an interaction partner for the angiogenic factor.

In view of the known nucleic acid sequences for angiogenic factors and their interaction partners (e.g. the known mRNA sequences available from GenBank under Accession No.s: AY047581.1 GI: 15422108(human VEGF-A), AF317892.1 GI: 12802453 (mouse VEGF), AY033508.1 GI: 15822724 (rat VEGF), NM_002253.3 GI: 1333881084 (human VEGF receptor), NM_025809.5 GI: 237512936 (mouse VEGF receptor), NM_019306.2 GI: 828178218 (rat VEGF receptor),) oligonucleotides may be designed to repress or silence the expression of an angiogenic factor, or an angiogenic factor receptor.

Such oligonucleotides may have any length, but may preferably be short, e.g. less than 100 nucleotides, e.g. 10-40 nucleotides, or 20-50 nucleotides, and may comprise a nucleotide sequence having complete- or near- complementarity (e.g. 80%, 85%, 90%, 91%, 92%, 93%, 94%, 95%, 96%, 97%, 98%, 99% or 100% complementarity) to a sequence of nucleotides of corresponding length in the target oligonucleotide, e.g. the mRNA of an angiogenic factor, or the mRNA of an interaction partner for the angiogenic factor. The complementary region of the nucleotide sequence may have any length, but is preferably at least 5, and optionally no more than 50, nucleotides long, e.g. one of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides.

Repression of expression of an angiogenic factor, or an interaction partner for the angiogenic factor will preferably result in a decrease in the quantity of an angiogenic factor, or an interaction partner for the angiogenic factor expressed by a cell/tissue/organ/organ system/subject. For example, in a given cell the repression of an angiogenic factor, or an interaction partner for the angiogenic factor by administration of a suitable nucleic acid will result in a decrease in the quantity of an angiogenic factor, or an interaction partner for the angiogenic factor expressed by that cell relative to an untreated cell. Repression may be partial. Preferred degrees of repression are at least 50%, more preferably one of at least 60%, 70%, 80%, 85% or 90%. A level of repression between 90% and 100% is considered a 'silencing' of expression or function.

Another alternative is the expression of a short hairpin RNA molecule (shRNA) in the cell. Preferably, the shRNA molecule comprises a partial sequence of an angiogenic factor, or an interaction partner for the angiogenic factor. Preferably, the shRNA sequence is between 40 and 100 bases in length, more preferably between 40 and 70 bases in length. The stem of the hairpin is preferably between 19 and 30 base pairs in length. The stem may contain G-U pairings to stabilise the hairpin structure.

siRNA molecules, longer dsRNA molecules or miRNA molecules may be made recombinantly by transcription of a nucleic acid sequence, preferably contained within a vector. Preferably, the siRNA molecule, longer dsRNA molecule or miRNA molecule comprises a partial sequence of an angiogenic factor, or an interaction partner for the angiogenic factor.

In one example, the siRNA, longer dsRNA or miRNA is produced endogenously (within a cell) by transcription from a vector. Suitable vectors may be oligonucleotide vectors configured to express the oligonucleotide agent capable of repression of an angiogenic factor, or an interaction partner for the angiogenic factor . Such vectors may be viral vectors or plasmid vectors.

In other examples, the disclosure provides nucleic acid that is capable, when suitably introduced into or expressed within a mammalian, e.g. human, cell that otherwise expresses an angiogenic factor, or an angiogenic factor receptor, of suppressing expression of an angiogenic factor, or an interaction partner for the angiogenic factor by RNAi.

In view of the known nucleic acid sequences for angiogenic factors and their interaction partners (e.g. the known mRNA sequences available from GenBank under Accession No.s: AF437895.1 GI: 16660685 (human VEGF), U41383.1 GI: 1134964 (mouse VEGF), NM_001287107.1 GI: 560186576 (rat VEGF), NM_002253.3 GI: 1333881084 (human VEGF receptor), NM_001001183.1 GI: 47564089 (mouse VEGF receptor), NM_019306.2 GI: 828178218 (rat VEGF receptor)) oligonucleotides may be designed to repress or silence the expression of an angiogenic factor, or an angiogenic factor receptor.

The nucleic acid may have substantial sequence identity to a portion of mRNA of an angiogenic factor, or an interaction partner for the angiogenic factor, e.g. as defined in GenBank accession M32977.1 GI: 181970 (VEGF), BC131822.1 GI: 124297527 (VEGF receptor) or the complementary sequence to said mRNA.

The nucleic acid may be a double-stranded siRNA. (As the skilled person will appreciate, and as explained further below, a siRNA molecule may include a short 3' DNA sequence also.)

Alternatively, the nucleic acid may be a DNA (usually double-stranded DNA) which, when transcribed in a mammalian cell, yields an RNA having two complementary portions joined via a spacer, such that the RNA takes the form of a hairpin when the complementary portions hybridise with each other. In a mammalian cell, the hairpin structure may be cleaved from the molecule by the enzyme DICER, to yield two distinct, but hybridised, RNA molecules.

The disclosure also provides DNA that, when transcribed in a mammalian cell, yields an RNA (herein also referred to as an shRNA) having two complementary portions which are capable of self-hybridising to produce a double-stranded motif, e.g. including a sequence selected from the group consisting of SEQ ID NOs: 14 to 17 or 18 to 21 or a sequence that differs from any one of the aforementioned sequences by a single base pair substitution.

The complementary portions will generally be joined by a spacer, which has suitable length and sequence to allow the two complementary portions to hybridise with each other. Preferably the 5' end of the spacer (immediately 3' of the upstream complementary portion) consists of the nucleotides -UU- or -UG-, again preferably -UU- (though, again, the use of these particular dinucleotides is not essential). A suitable spacer, recommended for use in the pSuper system of OligoEngine (Seattle, Washington, USA) is UUCAAGAGA. In this and other cases, the ends of the spacer may hybridise with each other, e.g. elongating the double-stranded motif beyond the exact sequences of SEQ ID NOs 14 to 17 or 18 to 21 by a small number (e.g. 1 or 2) of base pairs.

The double-stranded siRNAs of the disclosure may be introduced into mammalian cells *in vitro* or *in vivo* using known techniques, as described below, to suppress expression of an angiogenic factor or an interaction partner for the angiogenic factor.

Similarly, transcription vectors containing the DNAs of the disclosure may be introduced into tumour cells *in vitro* or *in vivo* using known techniques, as described below, for transient or stable expression of RNA, again to suppress expression of an angiogenic factor or an interaction partner for the angiogenic factor.

Accordingly, the disclosure also provides a method of suppressing expression of an angiogenic factor or an interaction partner for the angiogenic factor in a mammalian, e.g. human, cell, the method comprising administering to the cell a double-stranded siRNA of the disclosure or a transcription vector of the disclosure.

Similarly, the disclosure further provides a method of treating a disease/condition in which fibrosis and/or angiogenesis is pathologically implicated, the method comprising administering to a subject a double-stranded siRNA of the disclosure or a transcription vector of the disclosure in combination with an antagonist of an angiogenic factor.

The disclosure further provides the double-stranded siRNAs of the disclosure and the transcription vectors of the disclosure, for use in a method of treatment in combination with an antagonist of an angiogenic factor, preferably a method of treating a disease/condition in which fibrosis and/or angiogenesis is pathologically implicated

The disclosure further provides the use of the double-stranded siRNAs of the disclosure and the transcription vectors of the disclosure in combination with an antagonist of an angiogenic factor in the preparation of a medicament for the treatment of a disease/condition in which fibrosis and/or angiogenesis is pathologically implicated.

### Inhibition of angiogenic factor-mediated signalling

In embodiments of the present invention, agents capable of inhibiting the action of (i.e. antagonising) an angiogenic factor may possess one or more of the following functional properties:
- Inhibition of signalling mediated by an angiogenic factor;
- Inhibition of signalling mediated by binding of an angiogenic factor to an interaction partner for the angiogenic factor;
- Inhibition of a process mediated by an angiogenic factor;
- Inhibition of intussusception;
- Inhibition of capillary proliferation;
- Inhibition of edema.

These properties can be determined by analysis of the relevant agent in a suitable assay, which may involve comparison of the performance of the agent to suitable control agents. The skilled person is able to identify an appropriate control conditions for a given assay.

An angiogenic factor-mediated signalling and/or processes mediated by an angiogenic factor includes signalling mediated by fragments of an angiogenic factor and polypeptide complexes comprising an angiogenic factor or fragments thereof. Angiogenic factor-mediated signalling may be signalling mediated by human an angiogenic factor and/or mouse an angiogenic factor. Signalling mediated by an angiogenic factor may occur following binding of an angiogenic factor or an angiogenic factor containing complex to an interaction partner (e.g. receptor or ligand) to which an angiogenic factor or said complex binds.

In some embodiments, an agent may be capable of inhibiting the biological activity of an angiogenic factor or a complex comprising the angiogenic factor.

In some embodiments, the agent is an antagonist of one or more signalling pathways which are activated by signal transduction through receptors comprising an angiogenic factor receptor.

In some embodiments, the agent may be capable of inhibiting an angiogenic factor-mediated signalling to less than 100%, e.g. one of 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, or 1% or less of the level of signalling in the absence of the agent (or in the presence of an appropriate control agent). In some embodiments, the agent is capable of reducing an angiogenic factor-mediated signalling to less than 1 times, e.g. one of ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times the level of signalling in the absence of the agent (or in the presence of an appropriate control agent).

In some embodiments, the angiogenic factor-mediated signalling may be signalling mediated by binding of an angiogenic factor to an interaction partner for the angiogenic factor (e.g. a receptor or ligand for the angiogenic factor). Such signalling can be analysed e.g. by treating cells expressing an interaction partner for the angiogenic factor with the angiogenic factor, or by stimulating production of the angiogenic factor in cells which express an interaction partner for the angiogenic factor.

The IC₅₀ for agent for inhibition of an angiogenic factor-mediated signalling may be determined, e.g. by culturing cells expressing an interaction partner for the angiogenic factor receptor in the presence of an angiogenic factor and the agent, and measuring a functional consequence of signalling mediated by the angiogenic factor. For example, in an assay of cell proliferation mediated by an angiogenic factor ³H-thymidine incorporation into DNA may be analysed. In some embodiments, the agent may exhibit an IC₅₀ of 10 µg/ml or less, preferably one of ≤ 5 µg/ml, ≤ 4 µg/ml, ≤ 3.5 µg/ml, ≤ 3 µg/ml, ≤ 2 µg/ml, ≤ 1 µg/ml, ≤ 0.9 µg/ml, ≤ 0.8 µg/ml, ≤ 0.7 µg/ml, ≤ 0.6 µg/ml, or ≤ 0.5 µg/ml in such an assay.

In some embodiments, the agent may be capable of inhibiting a process mediated by an angiogenic factor (e.g. following stimulation with VEGF). Processes mediated by an angiogenic factor include e.g. intussusception, capillary proliferation, and gene/protein expression of e.g. collagen and an angiogenic factor, and can be evaluated either *in vitro* or *in vivo.*

In some examples, the antagonist of an angiogenic factor is an angiogenesis inhibitor selected from: endostatin (NP_569711.2 GI: 206597445), prolactin (CAA38264.1 GI: 531103), T2-TrpRS (as described in Otani A et al., *A fragment of human TrpRS as a potent antagonist of ocular angiogenesis* Proceedings of the National Academy of Sciences of the United States of America. 2002;99(1):178-183), or vasostatin (AAL13126.1 GI: 16151097), or a fragment, isoform, homologue or variant thereof.

### Therapeutic and prophylactic methods

The present disclosure provides methods and compositions for the treatment/prevention of fibrosis (particularly fibrosis of the eye) by antagonism of IL-11 mediated signalling and antagonism of angiogenesis. Also provided are methods and compositions for the treatment/prevention of angiogenesis by antagonism of IL-11 mediated signalling and antagonism of angiogenesis.

In some embodiments, the disease/condition to be treated/prevented in accordance with the present invention is characterised by an increase in the level of IL-11 mediated signalling and/or angiogenesis, e.g. in an organ/tissue which is affected by the disease/condition (e.g. the organ/tissue in which the symptoms of the disease/condition manifest).

In some embodiments, the disease/condition to be treated/prevented may be characterised by an increase in one or more of the following in an organ/tissue/subject affected by the disease, e.g. as compared to normal (i.e. non-diseased) organ/tissue/subject: IL-11, IL-11Rα, TGFβ, VEGF, FGF, PDGF, VEGFR, FGFR or PDGFR.

Treatment may be effective to prevent progression of the disease/condition, e.g. to reduce/delay/prevent worsening of the disease/condition or to reduce/delay/prevent development of the disease/condition. In some embodiments treatment may lead to an improvement in the disease/condition, e.g. a reduction in the severity of, and/or a reversal of, the symptoms of the disease/disorder. In some embodiments treatment may increase survival. Prevention may refer to prevention of development of the disease/condition, and/or prevention of worsening of the disease/condition, e.g. prevention of progression of the disease/condition to a later or chronic stage.

In some embodiments the disease/condition to be treated/prevented is choroidal neovascularization (CNV) and/or age-related macular degeneration (AMD), e.g. 'wet' AMD.

### Administration

Administration of the agents according to the present disclosure is preferably in a "therapeutically effective" or "prophylactically effective" amount, this being sufficient to show benefit to the subject.

The actual amount administered, and rate and time-course of administration, will depend on the nature and severity of the disease/condition and the nature of the agent. Prescription of treatment, e.g. decisions on dosage etc., is within the responsibility of general practitioners and other medical doctors, and typically takes account of the disease/condition to be treated, the condition of the individual subject, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

In therapeutic applications, agents capable of antagonising IL-11-mediated signalling and agents capable of antagonising an angiogenic factor are preferably formulated as a medicament or pharmaceutical together with one or more other pharmaceutically acceptable ingredients well known to those skilled in the art, including, but not limited to, pharmaceutically acceptable carriers, adjuvants, excipients, diluents, fillers, buffers, preservatives, anti-oxidants, lubricants, stabilisers, solubilisers, surfactants (e.g., wetting agents), masking agents, colouring agents, flavouring agents, and sweetening agents.

The term "pharmaceutically acceptable" as used herein pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, adjuvant, excipient, etc. must also be "acceptable" in the sense of being compatible with the other ingredients of the formulation.

Suitable carriers, adjuvants, excipients, etc. can be found in standard pharmaceutical texts, for example, Remington's Pharmaceutical Sciences, 18th edition, Mack Publishing Company, Easton, Pa., 1990; and Handbook of Pharmaceutical Excipients, 2nd edition, 1994.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulations may be prepared for administration as suitable for the disease/condition to be treated. For example, formulations may be formulated for topical, parenteral, systemic, intravenous, intra-arterial, intramuscular, intrathecal, intraocular, local ocular (e.g. subconjunctival, intravitreal, retrobulbar, intracameral), intra-conjunctival, subcutaneous, oral, or transdermal routes of administration which may include injection. Injectable formulations may comprise the selected agent in a sterile or isotonic medium. In particular embodiments the formulations are formulated for ocular administration.

Aspects of the present disclosure include compositions and methods comprising/employing an antagonist of IL-11 mediated signalling and an antagonist of an angiogenic factor.

"Combinations" as referred to herein encompass products and compositions (e.g. pharmaceutical compositions) comprising the components of the combination. "Combinations" also encompass therapeutic regimens employing the components of the combination.

In some embodiments the components of a combination are provided in separate compositions. In some embodiments more than one component of a combination is provided in a composition. In some embodiments the components of a combination are provided in one composition.

Similarly, in some embodiments the components of a combination are administered separately. In some embodiments a component of a combination is administered with another component of the combination. In some embodiments the components of a combination are administered together.

By way of illustration, in the example of a combination comprising an antagonist anti-IL-11 antibody and an antagonist anti-VEGF antibody, the anti-IL-11 antibody and anti-VEGF antibody may be administered together, or may be administered separately (e.g. subsequently).

Where components of a combination are administered together administration may be simultaneous administration. Where components of a combination are administered separately, administration may be simultaneous administration or sequential administration.

Disclosed herein is an antagonist of IL-11 mediated signalling for use in a method of treating or preventing fibrosis and/or angiogenesis, the method comprising separate or simultaneous administration to a subject of an antagonist of IL-11 mediated signalling and an antagonist of an angiogenic factor.

Also disclosed is an antagonist of an angiogenic factor for use in a method of treating or preventing fibrosis and/or angiogenesis, the method comprising separate or simultaneous administration to a subject of an antagonist of IL-11 mediated signalling and an antagonist of an angiogenic factor.

Also disclosed is the use of an antagonist of IL-11 mediated signalling in the manufacture of a medicament for use in a method comprising separate or simultaneous administration to a subject of an antagonist of IL-11 mediated signalling and an antagonist of an angiogenic factor.

Also disclosed is the use of an antagonist of an angiogenic factor in the manufacture of a medicament for use in a method comprising separate or simultaneous administration to a subject of an antagonist of IL-11 mediated signalling and an antagonist of an angiogenic factor.

Simultaneous administration refers to administration of the agents together, for example as a pharmaceutical composition containing the agents (*i*.*e*. a combined preparation), or immediately after each other and optionally via the same route of administration, *e*.*g*. to the same artery, vein or other blood vessel. In particular embodiments, the oncolytic virus and virus comprising nucleic acid encoding an immunomodulatory factor may be administered simultaneously in a combined preparation. In certain embodiments upon simultaneous administration the two or more of the agents may be administered via different routes of administration. In some embodiments simultaneous administration refers to administration at the same time, or within e.g. 1 hr, 2 hrs, 3 hrs, 4 hrs, 5 hrs, 6 hrs, 8 hrs, 12 hrs, 24 hrs, 36 hrs or 48 hrs.

Sequential administration refers to administration of one or more of the agents followed after a given time interval by separate administration of another of the agents. It is not required that the two agents are administered by the same route, although this is the case in some embodiments. The time interval may be any time interval, including hours, days, weeks, months, or years. In some embodiments sequential administration refers to administrations separated by a time interval of one of at least 10 min, 30 min, 1 hr, 6 hrs, 8 hrs, 12 hrs, 24 hrs, 36 hrs, 48 hrs, 3 days, 4 days, 5 days, 6 days, 1 week, 2 weeks, 3 weeks, 1 month, 6 weeks, 2 months, 3 months, 4 months, 5 months or 6 months.

In some embodiments, the treatment may further comprise other therapeutic or prophylactic intervention, e.g. surgery. Such other therapeutic or prophylactic intervention may occur before, during and/or after the therapies encompassed by the disclosure, and the deliveries of the other therapeutic or prophylactic interventions may occur via the same or different administration routes as the therapies of the disclosure.

Multiple doses of the agents (antagonist of IL-11 mediated signalling, antagonist of an angiogenic factor, compositions, combinations, etc.) of the present disclosure may be provided. One or more, or each, of the doses may be accompanied by simultaneous or sequential administration of another therapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months. By way of example, doses may be given once every 7, 14, 21 or 28 days (plus or minus 3, 2, or 1 days).

### Functional properties of the combination treatment

The combinations and methods of the present disclosure may be defined by reference to one or more functional properties and/or effects, and may be evaluated for such properties/effects e.g. by analysis as described in the experimental examples.

In some embodiments the combination of an antagonist of IL-11 mediated signalling and an antagonist of an angiogenic factor may possess one or more of the following functional properties:
- Improved ability to reduce fibrosis (e.g. retinal fibrosis) as compared to the ability to reduce fibrosis (e.g. retinal fibrosis) by either component used alone.
- Ability to reduce fibrosis (e.g. retinal fibrosis) which is synergistic (i.e. super-additive) as compared to the ability to reduce fibrosis (e.g. retinal fibrosis) by the components used alone.
- Ability to reduce fibrosis (e.g. retinal fibrosis) in a dose-dependent manner.
- Improved ability to prevent fibrosis (e.g. retinal fibrosis) as compared to the ability to prevent fibrosis (e.g. retinal fibrosis) by either component used alone.
- Improved ability to reduce angiogenesis as compared to the ability to reduce angiogenesis by either component used alone.
- Ability to reduce angiogenesis which is synergistic (i.e. super-additive) as compared to the ability to reduce angiogenesis by the components used alone.
- Ability to reduce angiogenesis in a dose-dependent manner.
- Improved ability to prevent angiogenesis as compared to the ability to prevent angiogenesis by either component used alone.
- Improved ability to reduce subretinal neovascularisation (e.g. choroidal neovascularization (CNV)) as compared to the ability to reduce subretinal neovascularisation (e.g. choroidal neovascularization (CNV)) by either component used alone.
- Ability to reduce subretinal neovascularisation (e.g. choroidal neovascularization (CNV)) which is synergistic (i.e. super-additive) as compared to the ability to reduce subretinal neovascularisation (e.g. choroidal neovascularization (CNV)) by the components used alone.
- Ability to reduce subretinal neovascularisation (e.g. choroidal neovascularization (CNV)) in a dose-dependent manner.
- Improved ability to prevent subretinal neovascularisation (e.g. choroidal neovascularization (CNV)) as compared to the ability to prevent subretinal neovascularisation (e.g. choroidal neovascularization (CNV)) by either component used alone.

Analysis of the ability to reduce/prevent fibrosis, angiogenesis and/or subretinal neovascularisation may be assessed e.g. *in vivo,* by analysis as described in the experimental examples. For example, the combination of an antagonist of IL-11 mediated signalling and an antagonist of an angiogenic factor may reduce choroidal neovascularization (CNV) as assessed via fluorescein fundus angiogram (FFA) and/or posterior-segment Optical Coherence Tomography (PS- OCT). The combination may also result in a decrease in the fibrotic area of the retina as observed through histopathological examination, or through immunofluorescence staining and examination. In some embodiments the combination has an improved ability to reduce blood vessel leakage compared to the ability to reduce blood vessel leakage by either component used alone.

In some embodiments, the antagonist of IL-11 mediated signalling is capable of improving the efficacy of the antagonist of an angiogenic factor. In some embodiments, the antagonist of an angiogenic factor is capable of improving the efficacy of the antagonist of IL-11 mediated signalling.

### Compositions/products/kits

The present disclosure also provides compositions comprising an antagonist of IL-11 mediated signalling as described herein and an antagonist of an angiogenic factor as described herein. Combinations of the present disclosure may be provided in a single composition, or may be provided as plural compositions comprising the components of the combination.

The present disclosure also provides a kit of parts comprising an antagonist of IL-11 mediated signalling as described herein and an antagonist of an angiogenic factor as described herein, or a composition according to the present disclosure.

In some embodiments the kit may have at least one container having a predetermined quantity of an antagonist of IL-11 mediated signalling as described herein, an antagonist of an angiogenic factor as described herein, or a composition according to the present disclosure. The kit may have containers containing individual components of the combinations of the present disclosure, or may have containers containing combinations of the components of the combinations of the present disclosure.

The kit may provide the antagonist of IL-11 mediated signalling, antagonist of an angiogenic factor, or composition with instructions for administration to a patient in order to treat fibrosis (e.g. fibrosis of the eye). The antagonist of IL-11 mediated signalling, antagonist of an angiogenic factor, or composition may be formulated so as to be suitable for ocular administration.

### Subjects

Subjects may be animal or human. Subjects are preferably mammalian, more preferably human. The subject may be a non-human mammal, but is more preferably human. The subject may be male or female. The subject may be a patient. The patient may have a disease/condition as described herein. A subject may have been diagnosed with a disease/condition requiring treatment, may be suspected of having such a disease/condition, or may be at risk from developing such disease/condition.

In embodiments according to the present invention the subject is preferably a human subject. In some embodiments, the subject to be treated according to a therapeutic or prophylactic method of the invention herein is a subject having, or at risk of developing, a disease/condition as described herein. In embodiments according to the present invention, a subject may be selected for treatment according to the methods based on characterisation for certain markers of such disease/disorder. A subject may have been diagnosed with the disease or disorder requiring treatment, or be suspected of having such a disease or disorder.

### Sequence identity

Pairwise and multiple sequence alignment for the purposes of determining percent identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780 software. When using such software, the default parameters, e.g. for gap penalty and extension penalty, are preferably used.

### Sequences

| SEQ ID NO: | DESCRIPTION | SEQUENCE |
|---|---|---|
| 1 | Human IL-11 (UniProt P20809) | MNCVCRLVLVVLSLWPDTAVAPGPPPGPPRVSPDPRAELDSTVLLTRSLLADTRQLAAQLRDKFP ADGDHNLDSLPTLAMSAGALGALQLPGVLTRLRADLLSYLRHVQWLRRAGGSSLKTLEPELGTLQ ARLDRLLRRLQLLMSRLALPQPPPDPPAPPLAPPSSAWGGIRAAHAILGGLHLTLDWAVRGLLLLK TRL |
| 2 | Human gp130 (UniProt P40189-1) | MLTLQTWLVQALFIFLTTESTGELLDPCGYISPESPVVQLHSNFTAVCVLKEKCMDYFHVNANYIV WKTNHFTIPKEQYTIINRTASSVTFTDIASLNIQLTCNILTFGQLEQNVYGITIISGLPPEKPKNLSCIV NEGKKMRCEWDGGRETHLETNFTLKSEWATHKFADCKAKRDTPTSCTVDYSTVYFVNIEVWVEA ENALGKVTSDHINFDPVYKVKPNPPHNLSVINSEELSSILKLTWTNPSIKSVIILKYNIQYRTKDAST WSQIPPEDTASTRSSFTVQDLKPFTEYVFRIRCMKEDGKGYWSDWSEEASGITYEDRPSKAPSF WYKIDPSHTQGYRTVQLVWKTLPPFEANGKILDYEVTLTRWKSHLQNYTVNATKLTVNLTNDRYL ATLTVRNLVGKSDAAVLTIPACDFQATHPVMDLKAFPKDNMLWVEWTTPRESVKKYILEWCVLSD KAPCITDWQQEDGTVHRTYLRGNLAESKCYLITVTPVYADGPGSPESIKAYLKQAPPSKGPTVRT KKVGKNEAVLEWDQLPVDVQNGFIRNYTIFYRTIIGNETAVNVDSSHTEYTLSSLTSDTLYMVRMA AYTDEGGKDGPEFTFTTPKFAQGEIEAIVVPVCLAFLLTTLLGVLFCFNKRDLIKKHIWPNVPDPSK SHIAQWSPHTPPRHNFNSKDQMYSDGNFTDVSVVEIEANDKKPFPEDLKSLDLFKKEKINTEGHS SGIGGSSCMSSSRPSISSSDENESSQNTSSTVQYSTVVHSGYRHQVPSVQVFSRSESTQPLLDS EERPEDLQLVDHVDGGDGILPRQQYFKQNCSQHESSPDISHFERSKQVSSVNEEDFVRLKQQIS DHISQSCGSGQMKMFQEVSAADAFGPGTEGQVERFETVGMEAATDEGMPKSYLPQTVRQGGY MPQ |
| 3 | Human IL11RA (UniProt Q14626) | |
| 4 | siRNA target IL-11 | CCTTCCAAAGCCAGATCTT |
| 5 | siRNA target IL-11 | GCCTGGGCAGGAACATATA |
| 6 | siRNA target IL-11 | CCTGGGCAGGAACATATAT |
| 7 | siRNA target IL-11 | GGTTCATTATGGCTGTGTT |
| 8 | siRNA target IL-11Rα | GGACCATACCAAAGGAGAT |
| 9 | siRNA target IL-11Rα | GCGTCTTTGGGAATCCTTT |
| 10 | siRNA target IL-11Rα | GCAGGACAGTAGATCCCT |
| 11 | siRNA target IL-11Rα | GCTCAAGGAACGTGTGTAA |
| 12 | siRNA to IL-11 (NM_000641.3) | CCUUCCAAAGCCAGAUCUUdTdT-AAGAUCUGGCUUUGGAAGGdTdT |
| 13 | siRNA to IL-11 (NM_000641.3) | GCCUGGGCAGGAACAUAUAdTdT-UAUAUGUUCCUGCCCAGGCdTdT |
| 14 | siRNA to IL-11 (NM_000641.3) | CCUGGGCAGGAACAUAUAUdTdT-AUAUAUGUUCCUGCCCAGGdTdT |
| 15 | siRNA to IL-11 (NM_000641.3) | GGUUCAUUAUGGCUGUGUUdTdT-AACACAGCCAUAAUGAACCdTdT |
| 16 | siRNA to IL-11Rα (U32324.1) | GGACCAUACCAAAGGAGAUdTdT-AUCUCCUUUGGUAUGGUCCdTdT |
| 17 | siRNA to IL-11Rα (U32324.1) | GCGUCUUUGGGAAUCCUUUdTdT-AAAGGAUUCCCAAAGACGCdTdT |
| 18 | siRNA to IL-11Rα (U32324.1) | GCAGGACAGUAGAUCCCUAdTdT-UAGGGAUCUACUGUCCUGCdTdT |
| 19 | siRNA to IL-11Rα (U32324.1) | GCUCAAGGAACGUGUGUAAdTdT-UUACACACGUUCCUUGAGCdTdT |
| 20 | Human VEGF-A (UniProtKB - P15692) | MNFLLSWVHWSLALLLYLHHAKWSQAAPMAEGGGQNHHEVVKFMDVYQRSYCHPIETLVD IFQEYPDEIEYIFKPSCVPLMRCGGCCNDEGLECVPTEESNITMQIMRIKPHQGQHIGEM SFLQHNKCECRPKKDRARQEKKSVRGKGKGQKRKRKKSRYKSWSVYVGARCCLMPWSLPG PHPCGPCSERRKHLFVQDPQTCKCSCKNTDSRCKARQLELNERTCRCDKPRR |
| 21 | Human VEGFR-1 (UniProtKB - P17948) | MVSYWDTGVLLCALLSCLLLTGSSSGSKLKDPELSLKGTQHIMQAGQTLHLQCRGEAAHK WSLPEMVSKESERLSITKSACGRNGKQFCSTLTLNTAQANHTGFYSCKYLAVPTSKKKET ESAIYIFISDTGRPFVEMYSEIPEIIHMTEGRELVIPCRVTSPNITVTLKKFPLDTLIPD GKRIIWDSRKGFIISNATYKEIGLLTCEATVNGHLYKTNYLTHRQTNTIIDVQISTPRPV KLLRGHTLVLNCTATTPLNTRVQMTWSYPDEKNKRASVRRRIDQSNSHANIFYSVLTIDK MQNKDKGLYTCRVRSGPSFKSVNTSVHIYDKAFITVKHRKQQVLETVAGKRSYRLSMKVK AFPSPEVVWLKDGLPATEKSARYLTRGYSLIIKDVTEEDAGNYTILLSIKQSNVFKNLTA TLIVNVKPQIYEKAVSSFPDPALYPLGSRQILTCTAYGIPQPTIKWFWHPCNHNHSEARC DFCSNNEESFILDADSNMGNRIESITQRMAIIEGKNKMASTLVVADSRISGIYICIASNK VGTVGRNISFYITDVPNGFHVNLEKMPTEGEDLKLSCTVNKFLYRDVTWILLRTVNNRTM HYSISKQKMAITKEHSITLNLTIMNVSLQDSGTYACRARNVYTGEEILQKKEITIRDQEA PYLLRNLSDHTVAISSSTTLDCHANGVPEPQITWFKNNHKIQQEPGIILGPGSSTLFIER VTEEDEGVYHCKATNQKGSVESSAYLTVQGTSDKSNLELITLTCTCVAATLFWLLLTLFI RKMKRSSSEIKTDYLSIIMDPDEVPLDEQCERLPYDASKWEFARERLKLGKSLGRGAFGK VVQASAFGIKKSPTCRTVAVKMLKEGATASEYKALMTELKILTHIGHHLNVVNLLGACTK QGGPLMVIVEYCKYGNLSNYLKSKRDLFFLNKDAALHMEPKKEKMEPGLEQGKKPRLDSV TSSESFASSGFQEDKSLSDVEEEEDSDGFYKEPITMEDLISYSFQVARGMEFLSSRKCIH RDLAARNILLSENNVVKICDFGLARDIYKNPDYVRKGDTRLPLKWMAPESIFDKIYSTKS DVWSYGVLLWEIFSLGGSPYPGVQMDEDFCSRLREGMRMRAPEYSTPEIYQIMLDCWHRD PKERPRFAELVEKLGDLLQANVQQDGKDYIPINAILTGNSGFTYSTPAFSEDFFKESISA PKFNSGSSDDVRYVNAFKFMSLERIKTFEELLPNATSMFDDYQGDSSTLLASPMLKRFTW TDSKPKASLKIDLRVTSKSKESGLSDVSRPSFCHSSCGHVSEGKRRFTYDHAELERKIAC CSPPPDYNSVVLYSTPPI |
| 22 | Human VEGFR-2 (UniProtKB - P35968) | |
| 23 | Human VEGFR-3 (UniProtKB - P35916) | MQRGAALCLRLWLCLGLLDGLVSGYSMTPPTLNITEESHVIDTGDSLSISCRGQHPLEWA WPGAQEAPATGDKDSEDTGVVRDCEGTDARPYCKVLLLHEVHANDTGSYVCYYKYIKARI EGTTAASSYVFVRDFEQPFINKPDTLLVNRKDAMWVPCLVSIPGLNVTLRSQSSVLWPDG QEVVWDDRRGMLVSTPLLHDALYLQCETTWGDQDFLSNPFLVHITGNELYDIQLLPRKSL ELLVGEKLVLNCTVWAEFNSGVTFDWDYPGKQAERGKWVPERRSQQTHTELSSILTIHNV SQHDLGSYVCKANNGIQRFRESTEVIVHENPFISVEWLKGPILEATAGDELVKLPVKLAA YPPPEFQWYKDGKALSGRHSPHALVLKEVTEASTGTYTLALWNSAAGLRRNISLELVVNV PPQIHEKEASSPSIYSRHSRQALTCTAYGVPLPLSIQWHWRPWTPCKMFAQRSLRRRQQQ DLMPQCRDWRAVTTQDAVNPIESLDTWTEFVEGKNKTVSKLVIQNANVSAMYKCVVSNKV GQDERLIYFYVTTIPDGFTIESKPSEELLEGQPVLLSCQADSYKYEHLRWYRLNLSTLHD AHGNPLLLDCKNVHLFATPLAASLEEVAPGARHATLSLSIPRVAPEHEGHYVCEVQDRRS HDKHCHKKYLSVQALEAPRLTQNLTDLLVNVSDSLEMQCLVAGAHAPSIVWYKDERLLEE KSGVDLADSNQKLSIQRVREEDAGRYLCSVCNAKGCVNSSASVAVEGSEDKGSMEIVILV |
| | | GTGVIAVFFWVLLLLIFCNMRRPAHADIKTGYLSIIMDPGEVPLEEQCEYLSYDASQWEF PRERLHLGRVLGYGAFGKVVEASAFGIHKGSSCDTVAVKMLKEGATASEHRALMSELKIL IHIGNHLNVVNLLGACTKPQGPLMVIVEFCKYGNLSNFLRAKRDAFSPCAEKSPEQRGRF RAMVELARLDRRRPGSSDRVLFARFSKTEGGARRASPDQEAEDLWLSPLTMEDLVCYSFQ VARGMEFLASRKCIHRDLAARNILLSESDVVKICDFGLARDIYKDPDYVRKGSARLPLKW MAPESIFDKVYTTQSDVWSFGVLLWEIFSLGASPYPGVQINEEFCQRLRDGTRMRAPELA TPAIRRIMLNCWSGDPKARPAFSELVEILGDLLQGRGLQEEEEVCMAPRSSQSSEEGSFS QVSTMALHIAQADAEDSPPSLQRHSLAARYYNWVSFPGCLARGAETRGSSRMKTFEEFPM TPTTYKGSVDNQTDSGMVLASEEFEQIESRHRQESGFSCKGPGQNVAVTRAHPDSQGRRR RPERGARGGQVFYNSEYGELSEPSEEDHCSPSARVTFFTDNSY |
| 24 | Aflibercept | SDTGRPFVEMYSEIPEIIHMTEGRELVIPCRVTSPNITVTLKKFPLDTLIPDGKRIIWDS RKGFIISNATYKEIGLLTCEATVNGHLYKTNYLTHRQTNTIIDVVLSPSHGIELSVGEKL VLNCTARTELNVGIDFNWEYPSSKHQHKKLVNRDLKTQSGSEMKKFLSTLTIDGVTRSDQ GLYTCAASSGLMTKKNSTFVRVHEKDKTHTCPPCPAPELLGGPSVFLFPPKPKDTLMISR TPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVMHEALHNH YTQKSLSLSPG |

### Numbered statements

The following numbered paragraphs (paras) describe particular aspects and embodiments of the present invention:
1. A method of treating or preventing fibrosis in a subject, the method comprising administering to a subject a therapeutically or prophylactically effective amount of an antagonist of IL-11 mediated signalling and an antagonist of an angiogenic factor.
2. A combination of an antagonist of IL-11 mediated signalling and an antagonist of an angiogenic factor for use in a method of treating or preventing fibrosis.
3. Use of an antagonist of IL-11 mediated signalling and an antagonist of an angiogenic factor in the manufacture of a medicament for use in a method of treating or preventing fibrosis.
4. The method according to para 1, the combination for use according to para 2, or the use according to para 3, wherein the fibrosis is fibrosis in the eye.
5. The method, the combination for use, or the use according to any one of paras 1 to 4, wherein the fibrosis is selected from Grave's opthalmopathy, epiretinal fibrosis, retinal fibrosis, idiopathic premacular fibrosis, subretinal fibrosis (e.g. associated with retinal detachment or macular degeneration (e.g. wet age-related macular degeneration (AMD)), diabetic retinopathy, glaucoma, geographic atrophy, corneal fibrosis, post-surgical fibrosis (e.g. of the posterior capsule following cataract surgery, or of the bleb following trabeculectomy for glaucoma), conjunctival fibrosis, or subconjunctival fibrosis.
6. The method, the combination for use, or the use according to any one of paras 1 to 5, wherein the fibrosis is retinal fibrosis, epiretinal fibrosis, or subretinal fibrosis.
7. The method according to para 1, the combination for use according to para 2, or the use according to para 3, wherein the fibrosis is fibrosis of the heart, liver, or kidney.
8. The method, the combination for use, or the use according to para 7, wherein the fibrosis is:
   in the liver and is associated with chronic liver disease or liver cirrhosis;
   in the kidney and is associated with chronic kidney disease; or
   in the heart and is associated with dysfunction of the musculature or electrical properties of the heart, or thickening of the walls or valves of the heart.
9. The method, the combination for use, or the use according to any one of paras 1 to 8, wherein the angiogenic factor is selected from vascular endothelial growth factor (VEGF), a fibroblast growth factor (FGF), and platelet-derived growth factor (PDGF).
10. The method, the combination for use, or the use according to any one of paras 1 to 9, wherein the angiogenic factor is VEGF.
11. The method, the combination for use, or the use according to any one of paras 1 to 10 wherein the antagonist of IL-11 mediated signalling is an agent capable of preventing or reducing the binding of IL-11 to an IL-11 receptor.
12. The method, the combination for use, or the use according to any one of paras 1 to 11, wherein the antagonist of IL-11 mediated signalling is an agent capable of binding to IL-11 or a receptor for IL-11.
13. The method, the combination for use, or the use according to para 12, wherein the antagonist of IL-11 mediated signalling is selected from the group consisting of: an antibody or an antigen-binding fragment thereof, a polypeptide, a peptide, an oligonucleotide, an aptamer or a small molecule.
14. The method, the combination for use, or the use according to para 13, wherein the antagonist of IL-11 mediated signalling is an anti-IL-11 antibody or an anti-IL-11Rα antibody.
15. The method, the combination for use, or the use according to para 13, wherein the antagonist of IL-11 mediated signalling is a decoy IL-11 receptor.
16. The method, the combination for use, or the use according to any one of paras 1 to 10, wherein the antagonist of IL-11 mediated signalling is capable of reducing the expression of IL-11 or a receptor for IL-11.
17. The method, the combination for use, or the use according to para 16, wherein the antagonist of IL-11 mediated signalling is an oligonucleotide or a small molecule.
18. The method, the combination for use, or the use according to any one of paras 1 to 17, wherein the antagonist of an angiogenic factor is an agent capable of preventing or reducing the binding of angiogenic factor to an interaction partner for an angiogenic factor.
19. The method, the combination for use, or the use according to any one of paras 1 to 18, wherein the antagonist of an angiogenic factor is an agent capable of binding to an angiogenic factor or an interaction partner for an angiogenic factor.
20. The method, the combination for use, or the use according to para 19, wherein the antagonist of an angiogenic factor is selected from the group consisting of: an antibody or an antigen-binding fragment thereof, a polypeptide, a peptide, an oligonucleotide, an aptamer or a small molecule.
21. The method, the combination for use, or the use according to para 20, wherein the antagonist of an angiogenic factor is an anti-VEGF antibody or an anti-VEGF receptor antibody.
22. The method, the combination for use, or the use according to para 20, wherein the antagonist of an angiogenic factor is a decoy VEGF receptor.
23. The method, the combination for use, or the use according to para 22, wherein the antagonist of an angiogenic factor is aflibercept.
24. The method, the combination for use, or the use according to any one of paras 1 to 17, wherein the antagonist of an angiogenic factor is capable of reducing the expression of an angiogenic factor or an interaction partner for an angiogenic factor.
25. The method, the combination for use, or the use according to para 24, wherein the antagonist of an angiogenic factor is an oligonucleotide or a small molecule.
26. A combination comprising an antagonist of IL-11 mediated signalling and an antagonist of an angiogenic factor.
27. A pharmaceutical composition comprising an antagonist of IL-11 mediated signalling and an antagonist of an angiogenic factor.
28. A kit of parts comprising a predetermined quantity of an antagonist of IL-11 mediated signalling and an antagonist of an angiogenic factor, a combination according to para 26 or a pharmaceutical composition according to para 27.
29. The combination according to para 26, the pharmaceutical composition according to para 27, or the kit of parts according to para 28, wherein the angiogenic factor is selected from vascular endothelial growth factor (VEGF), a fibroblast growth factor (FGF), and platelet-derived growth factor (PDGF).
30. The combination, the pharmaceutical composition or the kit of parts according to any one of paras 26 to 29, wherein the angiogenic factor is VEGF.
31. The combination, the pharmaceutical composition or the kit of parts according to any one of paras 26 to 30, wherein the antagonist of IL-11 mediated signalling is an agent capable of preventing or reducing the binding of IL-11 to an IL-11 receptor.
32. The combination, the pharmaceutical composition or the kit of parts according to any one of paras 26 to 31, wherein the antagonist of IL-11 mediated signalling is an agent capable of binding to IL-11 or a receptor for IL-11.
33. The combination, the pharmaceutical composition or the kit of parts according to para 32, wherein the antagonist of IL-11 mediated signalling is selected from the group consisting of: an antibody or an antigen-binding fragment thereof, a polypeptide, a peptide, an oligonucleotide, an aptamer or a small molecule.
34. The combination, the pharmaceutical composition or the kit of parts according to para 33, wherein the antagonist of IL-11 mediated signalling is an anti-IL-11 antibody or an anti-IL-11Rα antibody.
35. The combination, the pharmaceutical composition or the kit of parts according to para 33, wherein the antagonist of IL-11 mediated signalling is a decoy IL-11 receptor.
36. The combination, the pharmaceutical composition or the kit of parts according to any one of paras 26 to 30, wherein the antagonist of IL-11 mediated signalling is capable of reducing the expression of IL-11 or a receptor for IL-11.
37. The combination, the pharmaceutical composition or the kit of parts according to para 36, wherein the antagonist of IL-11 mediated signalling is an oligonucleotide or a small molecule.
38. The combination, the pharmaceutical composition or the kit of parts according to any one of paras 26 to 37, wherein the antagonist of an angiogenic factor is an agent capable of preventing or reducing the binding of angiogenic factor to an interaction partner for an angiogenic factor.
39. The combination, the pharmaceutical composition or the kit of parts according to any one of paras 26 to 38, wherein the antagonist of an angiogenic factor is an agent capable of binding to an angiogenic factor or an interaction partner for an angiogenic factor.
40. The combination, the pharmaceutical composition or the kit of parts according to para 39, wherein the antagonist of an angiogenic factor is selected from the group consisting of: an antibody or an antigen-binding fragment thereof, a polypeptide, a peptide, an oligonucleotide, an aptamer or a small molecule.
41. The combination, the pharmaceutical composition or the kit of parts according to para 40, wherein the antagonist of an angiogenic factor is an anti-VEGF antibody or an anti-VEGF receptor antibody.
42. The combination, the pharmaceutical composition or the kit of parts according to para 40, wherein the antagonist of an angiogenic factor is a decoy VEGF receptor.
43. The combination, the pharmaceutical composition or the kit of parts according to para 42, wherein the antagonist of an angiogenic factor is aflibercept.
44. The combination, the pharmaceutical composition or the kit of parts according to any one of paras 26 to 37, wherein the antagonist of an angiogenic factor is capable of reducing the expression of an interaction partner for an angiogenic factor.
45. The combination, the pharmaceutical composition or the kit of parts according to para 44, wherein the antagonist of an angiogenic factor is an oligonucleotide or a small molecule.
***

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

Where a nucleic acid sequence is disclosed herein, the reverse complement thereof is also expressly contemplated.

Methods described herein may preferably performed *in vitro.* The term *"in vitro"* is intended to encompass procedures performed with cells in culture whereas the term *"in vivo"* is intended to encompass procedures with/on intact multi-cellular organisms.

### Brief Description of the Figures

Embodiments and experiments illustrating the principles of the invention will now be discussed with reference to the accompanying figures.

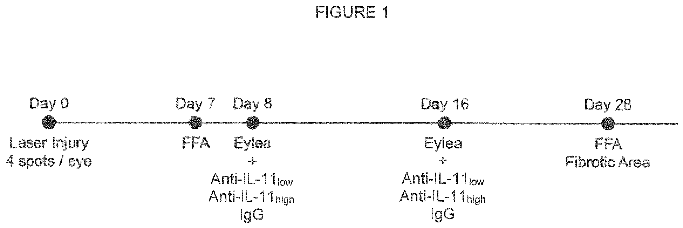

**Figure 1****.** Experimental outline of anti-VEGF and anti-IL-11 antibody combination treatment.
**Figure 2****.** Fluorescein fundus angiogram (FFA) analysis of laser generated choroidal neovascularization (CNV) monitored *in vivo* 7 and 28 days after laser-induced rupture of Bruch's membrane. (a) Measurements indicate the extent of CNV before and after anti-VEGF and anti-IL-11 intravitreal injections (IVT), data are mean±sem; **(b)** Leakage area after antibody treatment from a is plotted, we observe a dose dependent effect of anti-IL-11 antibody in reducing CNV in combination with Eylea; **(c)** FFA images of control (EYLEA+IGG), high dosage (2µg) and low dosage (0.5µg) Eylea + anti-IL-11 combination therapy at 7 and 28 days.

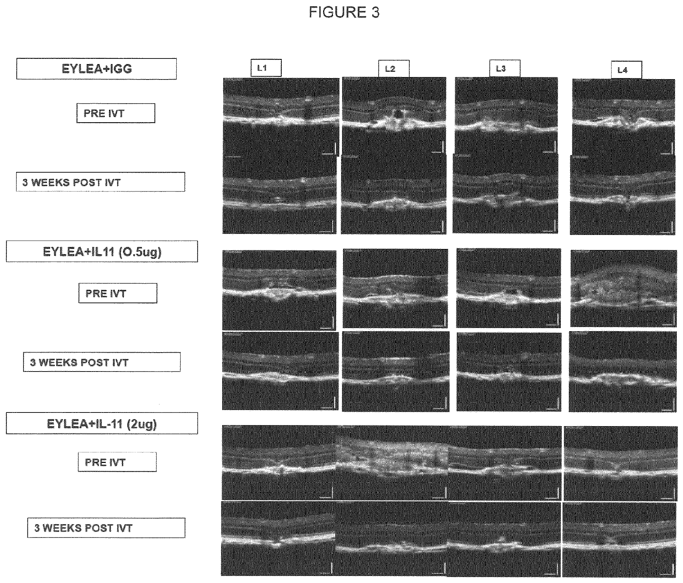

**Figure 3****.** Retinal structure imaging by posterior-segment Optical Coherence Tomography (PS-OCT) of subjects undergoing control (EYLEA+IGG), high dosage (2µg) or low dosage (0.5µg) Eylea + anti-IL-11 combination therapy.

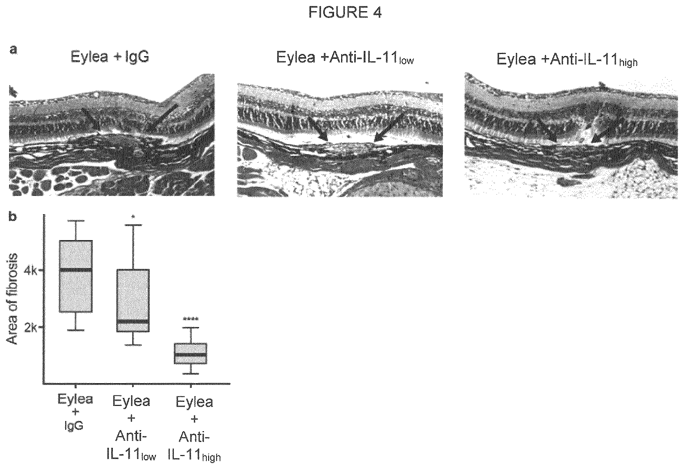

**Figure 4****.** Histological images of fibrosis on day 28 of the experiment. **(a)** Representative histological images of fibrosis, showing that laser injury led to a fibrotic scar that was significantly reduced in size with anti-IL-11 antibody treatment, and that this effect is more pronounced in the combination treatment. **(b)** The area of fibrosis was quantified using ImageJ software; two-tailed Dunnett's test; box- and-whisker plots show median (middle line), 25th-75th percentiles (box) and 10th-90th percentiles (whiskers).

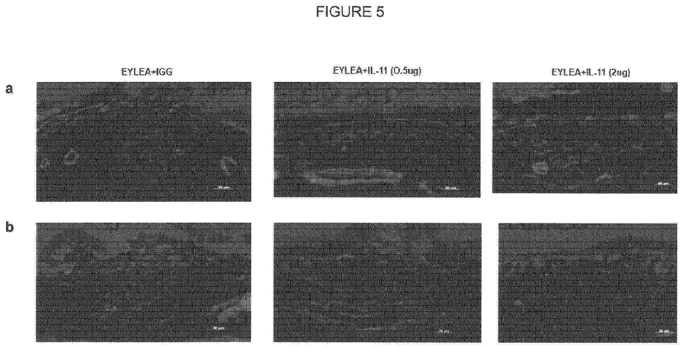

**Figure 5****.** Immunofluorescence (Alpha-SMA, DAPI) stained images (40X) of fibrosis on day 28 of the experiment, showing that laser injury led to a fibrotic scar that was significantly reduced in size with anti-IL-11 antibody treatment, and that this effect is more pronounced in the combination treatment. **(a)** Images from individuals 1-3, receiving Eylea + IgG control, low dosage combination therapy and high dosage combination therapy respectively. **(b)** Images from individuals 4-6, receiving Eylea + IgG control, low dosage combination therapy and high dosage combination therapy respectively.

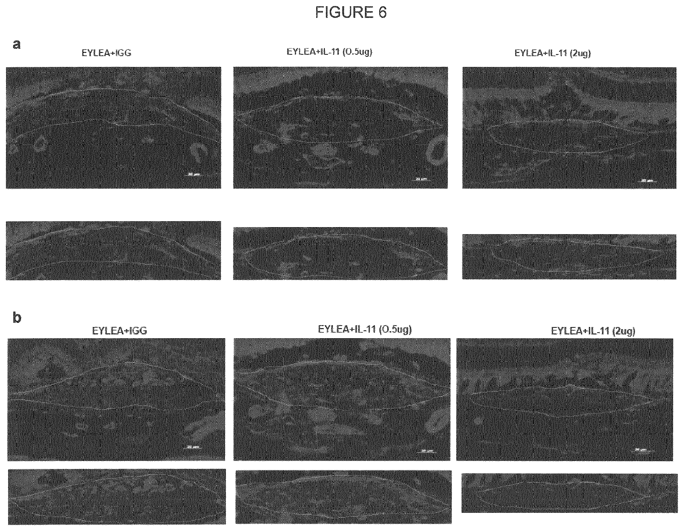

**Figure 6****.** Quantitative Alpha-SMA stained images (40X) of fibrosis on day 28 of the experiment, showing that laser injury led to a fibrotic scar that was significantly reduced in size with anti-IL-11 antibody treatment, and that this effect is more pronounced in the combination treatment; dashed outline marks the area of fibrosis. **(a)** Images from individuals 1-3, receiving Eylea + IgG control, low dosage combination therapy and high dosage combination therapy respectively; lower panels show top panel cropped to show area of fibrosis only. **(b)** Images from individuals 4-6, receiving Eylea + IgG control, low dosage combination therapy and high dosage combination therapy respectively; lower panels show top panel cropped to show area of fibrosis only.

### Examples

### Example 1: Materials and Methods

C57BL/6J wild type (WT) mice were purchased from InVivos (Singapore). Animals were housed on a 12 hour light/ 12 hour dark cycle with food and water provided *ad libitum.* Handling and care of all animals were performed according to the guidelines approved by SingHealth Institutional Animal Care and Use Committee (IACUC], Singapore, and is conducted in accordance with the Association for Research in Vision and Ophthalmology (ARVO) recommendations for animal experimentation.

### Laser induced CNV:

4 laser photocoagulation sites were placed concentrically around the optic disc of both eyes to induce CNVs. A diode laser (810 nm) was used with a relative potency scale of 120mW, an exposure time of 0.05 second, and a spot size of 50 µm. Laser spots were focused with crystal covers to avoid laser beam dispersion. Bubble formation was confirmed the rupture of Bruch's membrane.

### Intravitreal injection (IVT):

After the animal being anesthetized, the eyes were locally anesthetized by putting a drop of 1% xylocaine in the conjunctival sac. A 5% povidone iodine solution was placed in the conjunctival sac. The intravitreal injection was performed using a 32-gauge needle. The tested compounds were administered IVT injection into right eyes. Daily cage-side observations were performed on all animals to monitor for clinical signs of poor health, including any ocular abnormalities.

The following treatments were administered via the IVT route on days 8 and 16 post-laser:
*Eylea* + *IgG*: Anti-VEGF agent Eylea and IgG (isotype control). Total volume per injection = 1µl.
*Eylea* + *anti-IL-11(low):* Combination of Eylea and anti-IL-11 antibody (0.5µg in 1µl). Total volume per injection = 1µl.
*Eylea* + *anti-IL-11(high):* Combination of Eylea and anti-IL-11 antibody (2µg in 1µl). Total volume per injection = 1µl.

Animals was observed twice daily for signs of potential adverse events, and once daily for qualitative assessment of food consumption. The body weights of the animals were recorded at the time of animal selection for laser injury and every week throughout the remainder of the study.

At day 28, animals were euthanized by overdose of pentobarbital for blood and tissue collection.

### Fundus Fluorescein Angiography:

Fundus Fluorescein Angiography (FFA) was imaged using a MICRON IV fundus camera (Phoenix Laboratories USA). For FFA, animals were intraperitoneally injected with 10% sodium fluorescein at a dose of 0.01 ml per 5-6 gm body weight.

Retinal structure was monitored real time by posterior-segment Optical Coherence Tomography (PS-OCT).

### Histopathology and Immunohistochemistry

Whole mouse eye (28 days, n=10) was enucleated and fixed in a mixture of 4% Paraformaldehyde (PFA) (Sigma-Aldrich, St. Louis, MO) for 24 hours. The whole mouse eye were then dehydrated in increasing concentration of ethanol, clearance in xylene, and embedding in paraffin (Leica-Surgipath, Leica Biosystems Richmond, Inc.) Five-micron sections were cut with a rotary microtome (RM2255, Leica Biosystems Nussloch GmbH, Germany) and collected on POLYSINE^{™} microscope glass slides (Gerhard Menzel, Thermo Fisher Scientific, Newington, CT). The sections were dried in an oven of 37°C for at least 24 hour. To prepare the sections for histopathological and immunohistochemical examination, the sections were heated on a 60°C heat plate, deparaffinized in xylene and rehydrated in decreasing concentration of ethanol. The recommended procedure for Masson's trichrome staining (26367-Series, Electron Microscopy Sciences) were also performed to study both connective tissue and muscular pathologies. A light microscope (Axioplan 2; Carl Zeiss Meditec GmbH, Oberkochen, Germany) was used to examine the slides and images were captured.

In parallel, immunofluorescence staining was performed. Heat-induced antigen retrieval was performed by incubating sections in sodium citrate buffer (10 mM Sodium citrate, 0.05% Tween 20, pH 6.0) for 20 minutes at 95-100°C. The sections were then cooled down in sodium citrate buffer for 20 minutes in RT and washed three times for 5 minutes each with 1X PBS. Non-specific sites were blocked with blocking solution of 5% bovine serum albumin (BSA) in 0.1% Triton X-100 and 1XPBS for 1 hour at room temperature in a humidified chamber. The slides were then rinsed briefly with 1X PBS. A specific primary antibody shown in Table 1 was applied and incubated overnight at 4°C in a humidified chamber prepared in blocking solution. After washing twice with 1XPBS and once with 1X PBS with 0.1% tween for 10 minutes each, Alexa Fluro^{®} 594 - conjugated fluorescein-labeled secondary antibody shown in Table 1 (Invitrogen- Molecular Probes, Eugene, OR) was applied at a concentration of 1:1000 in blocking solution and incubated for 90 minutes at RT. The slides were then washed twice with 1XPBS and once with 1X PBS with 0.1% tween for 5 minutes each, the slides were mounted on the slides with Prolong Diamond Anti-fade DAPI5 Mounting Media (Invitrogen- Molecular Probes, Eugene, OR) to visualize cell nucleic. For negative controls, primary antibody was omitted.

A fluorescence microscope (Axioplan 2; Carl Zeiss Meditec GmbH, Oberkochen, Germany) was used to examine the slides and images were captured. Experiments were repeated in duplicates for the antibody.

**Table 1.**

| **Antibody** | **Catalog No.** | **Company** | **Concentration** |
|---|---|---|---|
| Smooth muscle Actin - Alpha (Alpha-SMA) | ab5694 | Abcam (Cambridge, MA, USA) | 1:100 |
| Alexa Fluor 594 goat anti-rabbit IgG (H+L) | A11012 | Invitrogen. Life Technologies (Invitrogen, Eugene, OR) | 1:1000 |

### Example 2: Fundus Fluorescein Angiography (FFA)

Subject animals were assigned to three cohorts (10 animals/cohort). Laser injury was induced at day 0 using four burn spots per eye in 10 animals per group.

The three cohorts were each respectively administered the following treatments injected via intravitreal (IVT) route on days 8 and 16 post-laser as follows:
*Eylea* + *IgG* Anti-VEGF agent Eylea and IgG (isotype control) was injected via intravitreal (IVT) route, 3 injections 1 week, 2 week and 3 week post laser of 1ul volume at 0.5 ug.
*Eylea* + *anti-IL-11(low)* Combination of Eylea and anti-IL-11 antibody (0.5µg in 1µl) was injected via intravitreal (IVT) route, 2 injections Day 8 and Day 16 post laser of 1µl volume.
*Eylea* + *anti-IL-11(high)* Combination of Eylea and anti-IL-11 antibody (2µg in 1µl) was injected via intravitreal (IVT) route, 2 injections Day 8 and Day 16 post laser of 1ul volume.

Choroidal neovascularization (CNV) was assessed using fluorescein fundus angiogram (FFA) after laser injury but before treatment (day 7) and at the end of the therapy (day 28).

Fundus Fluorescein Angiography (FFA) was imaged using a MICRON IV fundus camera (Phoenix Laboratories USA). For FFA, animals were intraperitoneally injected with 10% sodium fluorescein at a dose of 0.01 ml per 5-6 gm body weight.

FFA results can be seen in **Figure 2****.** The area of leakage was lower at day 28 than day 7 in all three treatment groups **(****Figure 2a****),** however this effect was more pronounced for the combination Eylea + anti-IL-11 therapy groups. Furthermore, we observe a dose dependent effect of anti-IL-11 antibody in reducing CNV in combination with Eylea **(****Figure 2b****).** The laser injuries showed reduced leakage by day 28 in all three treatment groups. This effect was more pronounced for Eylea + anti-IL-11(low) group than the IgG control, with Eylea + anti-IL-11(high) showing the highest rate of healing after 28 days. Figures 2a and 2b show a synergistic effect of antagonism of IL-11 mediated signalling and antagonism of an angiogenic factor in the treatment of CNV.

Retinal structure was also monitored real time by posterior-segment Optical Coherence Tomography (PS-OCT) on day 7 following laser injury and day 28. Results can be seen in **Figure 3****,** which demonstrate a decrease in choroidal neovascularization in the combination therapy cohorts in a dose-dependent manner.

### Example 3: Fibrotic Area

Fibrotic Area was measured at the end of treatment (day 28) for the animals above through histopathological examination. Collagen was stained with Masson's Trichrome Staining, and the area of fibrosis quantified, the results of which can be seen in **Figure 4****.** Lower areas of fibrosis were seen for both the low and high combination therapy cohorts compared to the Eylea + IgG control cohort, and these differences were statistically significant. Whilst the low dosage combination therapy cohort showed a smaller area of fibrosis than the Eylea+IgG cohort, this effect was more pronounced for the high dosage cohort. The histopathology data therefore demonstrate that the combination therapy is more effective than monotherapy alone, and that this effect is dose dependent.

Immunofluorescence staining was performed in parallel, the results of which can be seen in **Figures 5** **and** 6, which confirmed the observations of decreased fibrotic area in the combination treatment cohorts.

### Example 4: Comparative investigation

Animals were assigned to four cohorts as follows:
(i) Untreated controls
(ii) Treatment with Eylea
(iii) Treatment with anti-IL-11 antibody
(iv) Treatment with Eylea + anti-IL-11 antibody

Laser injury was induced at day 0 using four burn spots per eye in 10 animals per group.

Animals were administered their assigned treatments, injected via the intravitreal (IVT) route, on days 8 and 16 post-laser.

Choroidal neovascularization (CNV) was assessed using fluorescein fundus angiogram (FFA) after laser injury but before treatment (day 7) and at the end of the therapy on (day 28). Fibrotic area was measured on day 28.

The results demonstrated:
- Lower CNV and fibrotic area at day 28 in all treatment cohorts relative to the untreated control cohort
- Lower CNV and fibrotic area at day 28 in the cohort undergoing treatment with Eylea + anti-IL-11 antibody relative to the monotherapy treatment cohorts
- A synergistic (i.e. super-additive) effect was observed, with the improvements in CNV and fibrotic area in the cohort undergoing treatment with Eylea + anti-IL-11 antibody being greater than the sum of the effect observed in the monotherapy cohorts when combined.

### Summary

The results herein indicate that IL-11 antagonist therapy dramatically reduces retinal fibrosis **(****Figure 3****).** Furthermore, anti-fibrotic therapy with an IL-11 antagonist also appears to have a beneficial effect on the anti-angiogenic properties of Eylea **(****Figure 2****).** It is therefore clear that anti-fibrotic therapy has a synergistic effect with anti-angiogenic therapy, as shown by the marked improvement in both retinal fibrosis and choroidal neovascularization of the combination therapy cohorts compared with those receiving anti-fibrotic monotherapy.

### References

1. Du, X. & Williams, D. A. Interleukin-11: review of molecular, cell biology, and clinical use. Blood 89, 3897-3908 (1997).
2. Hegner, B. et al. Intrinsic Deregulation of Vascular Smooth Muscle and Myofibroblast Differentiation in Mesenchymal Stromal Cells from Patients with Systemic Sclerosis. PLoS One 11, e0153101 (2016).
3. Guo, X. & Chen, S.-Y. Transforming growth factor-β and smooth muscle differentiation. World J. Biol. Chem. 3, 41-52 (2012).
4. Hautmann, M. B., Madsen, C. S. & Owens, G. K. A Transforming Growth Factor β (TGFβ) Control Element Drives TGFβ-induced Stimulation of Smooth Muscle α-Actin Gene Expression in Concert with Two CArG Elements. J. Biol. Chem. 272, 10948-10956 (1997).
5. Ding, R., Darland, D. C., Parmacek, M. S. & D'Amore, P. A. Endothelial-mesenchymal interactions in vitro reveal molecular mechanisms of smooth muscle/pericyte differentiation. Stem Cells Dev. 13, 509-520 (2004).
6. Taki, H. et al. Monokine stimulation of interleukin-11 production by human vascular smooth muscle cells in vitro. Atherosclerosis 144, 375-380 (1999).
7. Zimmerman, M. A. et al. Interleukin-11 attenuates human vascular smooth muscle cell proliferation. Am. J. Physiol. Heart Circ. Physiol. 283, H175-80 (2002).
8. Postlethwaite, Postlethwaite, Pattanaik, D. & Brown, M. Vascular involvement in systemic sclerosis (scleroderma). J. Inflamm. Res. 105 (2011).
9. Matucci-Cerinic, M., Kahaleh, B. & Wigley, F. M. Review: Evidence That Systemic Sclerosis Is a Vascular Disease. Arthritis & Rheumatism 65, 1953-1962 (2013).
10. Tuder, R. M., Marecki, J. C., Richter, A., Fijalkowska, I. & Flores, S. Pathology of pulmonary hypertension. Clin. Chest Med. 28, 23-42, vii (2007).
11. Boin, F. et al. Oxidative stress-dependent activation of collagen synthesis is induced in human pulmonary smooth muscle cells by sera from patients with scleroderma-associated pulmonary hypertension. Orphanet J. Rare Dis. 9, 123 (2014).
12. Rabinovitch, M. Molecular pathogenesis of pulmonary arterial hypertension. J. Clin. Invest. 122, 4306-4313 (2012).
13. Gordon, K. J. & Blobe, G. C. Role of transforming growth factor-beta superfamily signalling pathways in human disease. Biochim. Biophys. Acta 1782, 197-228 (2008).
14. Crosas-Molist, E. et al. Vascular smooth muscle cell phenotypic changes in patients with Marfan syndrome. Arterioscler. Thromb. Vasc. Biol. 35, 960-972 (2015).
15. Matt, P. et al. Circulating transforming growth factor-beta in Marfan syndrome. Circulation 120, 526-532 (2009).
16. Holm, T. M. et al. Noncanonical TGFbeta signalling contributes to aortic aneurysm progression in Marfan syndrome mice. Science 332, 358-361 (2011).
17. Lindsay, M. E. & Dietz, H. C. Lessons on the pathogenesis of aneurysm from heritable conditions. Nature 473, 308-316 (2011).
18. Dietz, H. C. 2006 Curt Stern Award Address. Marfan syndrome: from molecules to medicines. Am. J. Hum. Genet. 81, 662-667 (2007).
19. Goumans, M.-J., Liu, Z. & ten Dijke, P. TGF-beta signalling in vascular biology and dysfunction. Cell Res. 19, 116-127 (2009).
20. Starke, R. M. et al. Vascular smooth muscle cells in cerebral aneurysm pathogenesis. Transl. Stroke Res. 5, 338-346 (2014).
21. Nakajima, N., Nagahiro, S., Sano, T., Satomi, J. & Satoh, K. Phenotypic modulation of smooth muscle cells in human cerebral aneurysmal walls. Acta Neuropathol. 100, 475-480 (2000).
22. Nikol, S. et al. Expression of transforming growth factor-beta 1 is increased in human vascular restenosis lesions. J. Clin. Invest. 90, 1582-1592 (1992).
23. Nabel, E. G. et al. Direct transfer of transforming growth factor beta 1 gene into arteries stimulates fibrocellular hyperplasia. Proc. Natl. Acad. Sci. U. S. A. 90, 10759-10763 (1993).
24. Wolf, Y. G., Rasmussen, L. M. & Ruoslahti, E. Antibodies against transforming growth factor-beta 1 suppress intimal hyperplasia in a rat model. J. Clin. Invest. 93, 1172-1178 (1994).
25. Edlin, R. S. et al. Characterization of primary and restenotic atherosclerotic plaque from the superficial femoral artery: Potential role of Smad3 in regulation of SMC proliferation. J. Vasc. Surg. 49, 1289-1295 (2009).
26. Tsai, S. et al. TGF-beta through Smad3 signalling stimulates vascular smooth muscle cell proliferation and neointimal formation. Am. J. Physiol. Heart Circ. Physiol. 297, H540-9 (2009).
27. Mallawaarachchi, C. M., Weissberg, P. L. & Siow, R. C. M. Smad7 gene transfer attenuates adventitial cell migration and vascular remodeling after balloon injury. Arterioscler. Thromb. Vasc. Biol. 25, 1383-1387 (2005).
28. Louis, S. F. & Zahradka, P. Vascular smooth muscle cell motility: From migration to invasion. Exp. Clin. Cardiol. 15, e75-85 (2010).
29. Suwanabol, P. A., Kent, K. C. & Liu, B. TGF-β and restenosis revisited: a Smad link. J. Surg. Res. 167, 287-297 (2011).
30. McCaffrey, T. A. et al. Genomic instability in the type II TGF-beta1 receptor gene in atherosclerotic and restenotic vascular cells. J. Clin. Invest. 100, 2182-2188 (1997).
31. Mallat, Z. et al. Inhibition of transforming growth factor-beta signalling accelerates atherosclerosis and induces an unstable plaque phenotype in mice. Circ. Res. 89, 930-934 (2001).
32. Robertson, A.-K. L. et al. Disruption of TGF-β signalling in T cells accelerates atherosclerosis. J. Clin. Invest. 112, 1342-1350 (2003).
33. Grainger, D. J., Mosedale, D. E., Metcalfe, J. C. & Böttinger, E. P. Dietary fat and reduced levels of TGFbeta1 act synergistically to promote activation of the vascular endothelium and formation of lipid lesions. J. Cell Sci. 113 ( Pt 13), 2355-2361 (2000).
34. Grainger, D. J., Witchell, C. M. & Metcalfe, J. C. Tamoxifen elevates transforming growth factor-beta and suppresses diet-induced formation of lipid lesions in mouse aorta. Nat. Med. 1, 1067-1073 (1995).
35. O'Connor, S. C. & Gornik, H. L. Recent developments in the understanding and management of fibromuscular dysplasia. J. Am. Heart Assoc. 3, e001259 (2014).
36. Begelman, S. M. & Olin, J. W. Fibromuscular dysplasia. Curr. Opin. Rheumatol. 12, 41-47 (2000).
37. Ganesh, S. K. et al. Clinical and biochemical profiles suggest fibromuscular dysplasia is a systemic disease with altered TGF-β expression and connective tissue features. FASEB J. 28, 3313-3324 (2014).
38. Weber, B. R. & Dieter, R. S. Renal artery stenosis: epidemiology and treatment. Int. J. Nephrol. Renovasc. Dis. 7, 169-181 (2014).
39. El Mabrouk, M., Diep, Q. N., Benkirane, K., Touyz, R. M. & Schiffrin, E. L. SAM68: a downstream target of angiotensin II signalling in vascular smooth muscle cells in genetic hypertension. Am. J. Physiol. Heart Circ. Physiol. 286, H1954-62 (2004).
40. Zhang, L. et al. Impaired peroxisome proliferator-activated receptor-gamma contributes to phenotypic modulation of vascular smooth muscle cells during hypertension. J. Biol. Chem. 285, 13666-13677 (2010).
41. Warren, H. R. et al. Genome-wide association analysis identifies novel blood pressure loci and offers biological insights into cardiovascular risk. Nat. Genet. 49, 403-415 (2017).
42. Cove-Smith, A. & Hendry, B. M. The regulation of mesangial cell proliferation. Nephron Exp. Nephrol. 108, e74-9 (2008).
43. Diamond, J. R. & Karnovsky, M. J. Focal and segmental glomerulosclerosis: analogies to atherosclerosis. Kidney Int. 33, 917-924 (1988).
44. Herrera, G. A. Plasticity of mesangial cells: a basis for understanding pathological alterations. Ultrastruct. Pathol. 30, 471-479 (2006).
45. Loeffler, I. & Wolf, G. Transforming growth factor-β and the progression of renal disease. Nephrol. Dial. Transplant 29 Suppl 1, i37-i45 (2014).
46. Santibanez, J. F., Krstic, J., Quintanilla, M. & Bernabeu, C. TGF-β Signalling and Its Role in Cancer Progression and Metastasis. in eLS 1-9 (2016).
47. Freyer, A. M., Johnson, S. R. & Hall, I. P. Effects of growth factors and extracellular matrix on survival of human airway smooth muscle cells. Am. J. Respir. Cell Mol. Biol. 25, 569-576 (2001).
48. Chung, K. F. The role of airway smooth muscle in the pathogenesis of airway wall remodeling in chronic obstructive pulmonary disease. Proc. Am. Thorac. Soc. 2, 347-54; discussion 371-2 (2005).
49. Chen, G. & Khalil, N. TGF-beta1 increases proliferation of airway smooth muscle cells by phosphorylation of map kinases. Respir. Res. 7, 2 (2006).
50. Ojiaku, C. A., Yoo, E. J. & Panettieri, R. A., Jr. Transforming Growth Factor β1 Function in Airway Remodeling and Hyperresponsiveness. The Missing Link? Am. J. Respir. Cell Mol. Biol. 56, 432-442 (2017).
51. McMillan, S. J., Xanthou, G. & Lloyd, C. M. Manipulation of allergen-induced airway remodeling by treatment with anti-TGF-beta antibody: effect on the Smad signalling pathway. J. Immunol. 174, 5774-5780 (2005).
52. Johnson, P. R. et al. Airway smooth muscle cell proliferation is increased in asthma. Am. J. Respir. Crit. Care Med. 164, 474-477 (2001).
53. Doeing, D. C. & Solway, J. Airway smooth muscle in the pathophysiology and treatment of asthma. J. Appl. Physiol. 114, 834-843 (2013).
54. Takizawa, H. et al. Increased Expression of Transforming Growth Factor- β 1 in Small Airway Epithelium from Tobacco Smokers and Patients with Chronic Obstructive Pulmonary Disease (COPD). Am. J. Respir. Crit. Care Med. 163, 1476-1483 (2001).
55. Stanzel, R. D. P., Lourenssen, S., Nair, D. G. & Blennerhassett, M. G. Mitogenic factors promoting intestinal smooth muscle cell proliferation. Am. J. Physiol. Cell Physiol. 299, C805-17 (2010).
56. Graham, M. F., Bryson, G. R. & Diegelmann, R. F. Transforming growth factor beta 1 selectively augments collagen synthesis by human intestinal smooth muscle cells. Gastroenterology 99, 447-453 (1990).
57. Zhang, H., Xiong, Z.-M. & Cao, K. Mechanisms controlling the smooth muscle cell death in progeria via down-regulation of poly(ADP-ribose) polymerase 1. Proc. Natl. Acad. Sci. U. S. A. 111, E2261-70 (2014).
58. Aliper, A. M. et al. Signalling pathway activation drift during aging: Hutchinson-Gilford Progeria Syndrome fibroblasts are comparable to normal middle-age and old-age cells. Aging 7, 26-37 (2015).
59. Bierie, B. et al. Abrogation of TGF-beta signalling enhances chemokine production and correlates with prognosis in human breast cancer. J. Clin. Invest. 119, 1571-1582 (2009).
60. Shull, M. M. et al. Targeted disruption of the mouse transforming growth factor-beta 1 gene results in multifocal inflammatory disease. Nature 359, 693-699 (1992).
61. GTEx Consortium. The Genotype-Tissue Expression (GTEx) project. Nat. Genet. 45, 580-585 (2013).
62. Andersson, R. et al. An atlas of active enhancers across human cell types and tissues. Nature 507, 455-461 (2014).
63. Love, M. I., Huber, W. & Anders, S. Moderated estimation of fold change and dispersion for RNA-seq data with DESeq2. (2014). doi:10.1101/002832
64. Dams-Kozlowska, H. et al. A designer hyper interleukin 11 (H11) is a biologically active cytokine. BMC Biotechnol. 12, 8 (2012).
65. Alitalo K, Tammela T, Petrova TV. Lymphangiogenesis in development and human disease. Nature. 438:946-953 (2005)
66. Lee S, Jilani SM, Nikolova GV, Carpizo D, Iruela-Arispe ML. Processing of VEGF-A by matrix metalloproteinases regulates bioavailability and vascular patterning in tumors. J Cell Biol. 2005;169:681-691.
67. Leung DW, Cachianes G, Kuang WJ, Goeddel DV, Ferrara N. Vascular endothelial growth factor is a secreted angiogenic mitogen. Science. 1989;246:1306-1309. [PubMed]
68. Alon T, Hemo I, Itin A, Stone J, Keshet E. Vascular endothelial growth factor acts as a survival factor for newly formed retinal vessels and has implications for retinopathy of prematurity. Nat Med. 1995;1:1024-1028. [PubMed]

## Claims

1. A combination of an antagonist of IL-11 mediated signalling and an antagonist of an angiogenic factor for use in a method of treating or preventing fibrosis;
wherein the antagonist of IL-11 mediated signalling is an anti-IL-11 antibody or an antigen-binding fragment thereof, or an anti-IL-Rα antibody or an antigen-binding fragment thereof; and
wherein the antagonist of an angiogenic factor is a decoy VEGF receptor.

2. The combination for use according to claim 1, wherein the fibrosis is fibrosis of the eye, liver, lungs, kidney, heart, blood vessels, skin, pancreas, intestine, brain, or bone marrow.

3. The combination for use according to claim 1 or claim 2, wherein the fibrosis is fibrosis of a disease or condition selected from Grave's opthalmopathy, epiretinal fibrosis, retinal fibrosis, idiopathic premacular fibrosis, subretinal fibrosis, subretinal fibrosis associated with retinal detachment, subretinal fibrosis associated with macular degeneration, subretinal fibrosis associated with wet age-related macular degeneration, diabetic retinopathy, glaucoma, geographic atrophy, corneal fibrosis, post-surgical fibrosis, post-surgical fibrosis of the posterior capsule following cataract surgery, post-surgical fibrosis of the bleb following trabeculectomy for glaucoma, conjunctival fibrosis, subconjunctival fibrosis, pulmonary fibrosis, cystic fibrosis, idiopathic pulmonary fibrosis, progressive massive fibrosis, scleroderma, obliterative bronchiolitis, Hermansky-Pudlak syndrome, asbestosis, silicosis, chronic pulmonary hypertension, AIDS associated pulmonary hypertension, sarcoidosis, tumor stroma in lung disease, asthma, chronic liver disease, primary biliary cirrhosis, schistosomal liver disease, liver cirrhosis, hypertrophic cardiomyopathy, dilated cardiomyopathy, fibrosis of the atrium, atrial fibrillation, fibrosis of the ventricle, ventricular fibrillation, myocardial fibrosis, Brugada syndrome, myocarditis, endomyocardial fibrosis, myocardial infarction, fibrotic vascular disease, hypertensive heart disease, arrhythmogenic right ventricular cardiomyopathy, tubulointerstitial and glomerular fibrosis, atherosclerosis, varicose veins, cerebral infarcts, gliosis, Alzheimer's disease, muscular dystrophy, Duchenne muscular dystrophy, Becker's muscular dystrophy, Crohn's disease, microscopic colitis, primary sclerosing cholangitis, scleroderma, nephrogenic systemic fibrosis, cutis keloid, arthrofibrosis, Dupuytren's contracture, mediastinal fibrosis, retroperitoneal fibrosis, myelofibrosis, Peyronie's disease, adhesive capsulitis, renal fibrosis, nephritic syndrome, Alport's syndrome, HIV associated nephropathy, polycystic kidney disease, Fabry's disease, diabetic nephropathy, chronic glomerulonephritis, nephritis associated with systemic lupus, progressive systemic sclerosis, chronic graft versus host disease, arthritis, fibrotic pre-neoplastic disease, fibrotic neoplastic disease, fibrosis induced by chemical or environmental insult.

4. The combination for use according to any one of claims 1 to 3, wherein the fibrosis is fibrosis of a disease or condition selected from Grave's opthalmopathy, epiretinal fibrosis, retinal fibrosis, idiopathic premacular fibrosis, subretinal fibrosis, subretinal fibrosis associated with retinal detachment, subretinal fibrosis associated with macular degeneration, subretinal fibrosis associated with wet age-related macular degeneration, diabetic retinopathy, glaucoma, geographic atrophy, corneal fibrosis, post-surgical fibrosis, post-surgical fibrosis of the posterior capsule following cataract surgery, post-surgical fibrosis of the bleb following trabeculectomy for glaucoma, conjunctival fibrosis, or subconjunctival fibrosis.

5. The combination for use according to claim 1 or claim 2, wherein the fibrosis is:
in the liver and is associated with chronic liver disease or liver cirrhosis;
in the kidney and is associated with chronic kidney disease; or
in the heart and is associated with dysfunction of the musculature or electrical properties of the heart, or thickening of the walls or valves of the heart.

6. The combination for use according to any one of claims 1 to 5, wherein the antagonist of an angiogenic factor is aflibercept.

7. A combination comprising an antagonist of IL-11 mediated signalling and an antagonist of an angiogenic factor;
wherein the antagonist of IL-11 mediated signalling is an anti-IL-11 antibody or an antigen-binding fragment thereof, or an anti-IL-Rα antibody or an antigen-binding fragment thereof; and
wherein the antagonist of an angiogenic factor is a decoy VEGF receptor.

8. A pharmaceutical composition comprising an antagonist of IL-11 mediated signalling and an antagonist of an angiogenic factor;
wherein the antagonist of IL-11 mediated signalling is an anti-IL-11 antibody or an antigen-binding fragment thereof, or an anti-IL-Rα antibody or an antigen-binding fragment thereof; and
wherein the antagonist of an angiogenic factor is a decoy VEGF receptor.

9. The combination according to claim 7, or the pharmaceutical composition according to claim 8, wherein the antagonist of an angiogenic factor is aflibercept.

## Patentansprüche

1. Kombination aus einem Antagonisten der IL-11-vermittelten Signalgebung und einem Antagonisten eines Angiogenesefaktors zur Verwendung in einem Verfahren zur Behandlung oder Prävention von Fibrose;
wobei der Antagonist der IL-11-vermittelten Signalgebung ein Anti-IL-11-Antikörper oder ein Antigenbindungsfragment davon oder ein Anti-IL-Rα-Antikörper oder ein Antigenbindungsfragment davon ist; und
wobei der Antagonist eines Angiogenesefaktors ein Decoy-VEGF-Rezeptor ist.

2. Kombination zur Verwendung nach Anspruch 1, wobei die Fibrose Fibrose der Augen, der Leber, der Lunge, der Nieren, des Herzens, der Blutgefäße, der Haut, der Bauchspeicheldrüse, des Darms, des Gehirns oder des Knochenmarks ist.

3. Kombination zur Verwendung nach Anspruch 1 oder Anspruch 2, wobei die Fibrose Fibrose einer Erkrankung oder eines Leidens ist, die/das aus Gravescher Ophthalmopathie, epiretinaler Fibrose, Netzhautfibrose, idiopathischer prämakulärer Fibrose, subretinaler Fibrose, subretinaler Fibrose in Verbindung mit Netzhautablösung, subretinaler Fibrose in Verbindung mit Makuladegeneration, subretinaler Fibrose in Verbindung mit feuchter altersbedingter Makuladegeneration, diabetischer Retinopathie, Glaukom, geographischer Atrophie, Hornhautfibrose, postoperativer Fibrose, postoperativer Fibrose der hinteren Kapsel nach einer Kataraktoperation, postoperativer Fibrose des Augapfels nach einer Trabekulektomie wegen eines Glaukoms, Bindehautfibrose, subkonjunktivaler Fibrose, Lungenfibrose, zystischer Fibrose, idiopathischer Lungenfibrose, progressiver massiver Fibrose, Sklerodermie, obliterativer Bronchiolitis, Hermansky-Pudlak-Syndrom, Asbestose, Silikose, chronischer pulmonaler Hypertonie, AIDS-assoziierter pulmonaler Hypertonie, Sarkoidose, Tumorstroma bei Lungenerkrankung, Asthma, chronischer Lebererkrankung, primärer biliärer Zirrhose, schistosomaler Lebererkrankung, Leberzirrhose, hypertropher Kardiomyopathie, dilatativer Kardiomyopathie, Fibrose des Vorhofs, Vorhofflimmern, Fibrose des Ventrikels, Kammerflimmern, Myokardfibrose, Brugada-Syndrom, Myokarditis, Endomyokardfibrose, Myokardinfarkt, fibrotischer Gefäßerkrankung, hypertensiver Herzerkrankung, arrhythmogener rechtsventrikulärer Kardiomyopathie, tubulointerstitieller und glomerulärer Fibrose, Atherosklerose, Krampfadern, Hirninfarkte, Gliose, Alzheimer-Krankheit, Muskeldystrophie, Duchenne-Muskeldystrophie, Becker-Muskeldystrophie, Morbus Crohn, mikroskopischer Kolitis, primär sklerosierender Cholangitis, Sklerodermie, nephrogener systemischer Fibrose, Keloiden der Haut, Arthrofibrose, Dupuytrenscher Kontraktur, Mediastinalfibrose, retroperitonealer Fibrose, Myelofibrose, Peyronie-Krankheit, adhäsiver Kapsulitis, Nierenfibrose, nephritischem Syndrom, Alport-Syndrom, HIV-assoziierter Nephropathie, polyzystischer Nierenerkrankung, Morbus Fabry, diabetischer Nephropathie, chronischer Glomerulonephritis, mit systemischem Lupus assoziierter Nephritis, progressiver systemischer Sklerose, chronischer Transplantat-gegen-Wirt-Krankheit, Arthritis, fibrotischer präneoplastischer Erkrankung, fibrotischer neoplastischer Erkrankung, durch chemische oder umweltbedingte Einflüsse induzierter Fibrose ausgewählt ist.

4. Kombination zur Verwendung nach einem der Ansprüche 1 bis 3, wobei die Fibrose eine Fibrose einer Erkrankung oder eines Leidens ist, die/das aus Gravescher Ophthalmopathie, epiretinaler Fibrose, Netzhautfibrose, idiopathischer prämakulärer Fibrose, subretinaler Fibrose, subretinaler Fibrose in Verbindung mit Netzhautablösung, subretinaler Fibrose in Verbindung mit Makuladegeneration, subretinaler Fibrose in Verbindung mit feuchter altersbedingter Makuladegeneration, diabetischer Retinopathie, Glaukom, geographischer Atrophie, Hornhautfibrose, postoperativer Fibrose, postoperativer Fibrose der hinteren Kapsel nach einer Kataraktoperation, postoperativer Fibrose des Augapfels nach einer Trabekulektomie wegen eines Glaukoms, Bindehautfibrose und subkonjunktivaler Fibrose ausgewählt ist.

5. Kombination zur Verwendung nach Anspruch 1 oder Anspruch 2, wobei die Fibrose:
sich in der Leber befindet und mit chronischer Lebererkrankung oder Leberzirrhose in Zusammenhang steht;
sich in den Nieren befindet und mit chronischer Nierenerkrankung in Zusammenhang steht; oder
sich im Herz befindet und mit einer Dysfunktion der Muskulatur oder der elektrischen Eigenschaften des Herzens oder einer Verdickung der Wände oder Klappen des Herzens in Zusammenhang steht.

6. Kombination zur Verwendung nach einem der Ansprüche 1 bis 5, wobei der Antagonist eines Angiogenesefaktors Aflibercept ist.

7. Kombination, umfassend einen Antagonisten der IL-11-vermittelten Signalgebung und einen Antagonisten eines Angiogenesefaktors;
wobei der Antagonist der IL-11-vermittelten Signalgebung ein Anti-IL-11-Antikörper oder ein Antigenbindungsfragment davon oder ein Anti-IL-Rα-Antikörper oder ein Antigenbindungsfragment davon ist; und
wobei der Antagonist eines Angiogenesefaktors ein Decoy-VEGF-Rezeptor ist.

8. Pharmazeutische Zusammensetzung, umfassend einen Antagonisten der IL-11-vermittelten Signalgebung und einen Antagonisten eines Angiogenesefaktors;
wobei der Antagonist der IL-11-vermittelten Signalgebung ein Anti-IL-11-Antikörper oder ein Antigenbindungsfragment davon oder ein Anti-IL-Rα-Antikörper oder ein Antigenbindungsfragment davon ist; und
wobei der Antagonist eines Angiogenesefaktors ein Decoy-VEGF-Rezeptor ist.

9. Kombination nach Anspruch 7 oder pharmazeutische Zusammensetzung nach Anspruch 8, wobei der Antagonist eines Angiogenesefaktors Aflibercept ist.

## Revendications

1. Combinaison d'un antagoniste de signalisation médiée par IL-11 et d'un antagoniste d'un facteur angiogénique pour utilisation dans un procédé de traitement ou de prévention d'une fibrose ;
dans laquelle l'antagoniste de signalisation médiée par IL-11 est un anticorps anti-IL-11 ou un fragment de liaison à l'antigène de celui-ci, ou un anticorps anti-IL-Rα ou un fragment de liaison à l'antigène de celui-ci ; et
dans laquelle l'antagoniste d'un facteur angiogénique est un récepteur leurre de VEGF.

2. Combinaison pour utilisation selon la revendication 1, dans laquelle la fibrose est une fibrose de l'œil, du foie, des poumons, du rein, du cœur, des vaisseaux sanguins, de la peau, du pancréas, de l'intestin, du cerveau, ou de la moelle osseuse.

3. Combinaison pour utilisation selon la revendication 1 ou la revendication 2, dans laquelle la fibrose est une fibrose d'une maladie ou affection sélectionnée parmi une ophtalmopathie de Grave-Basedow, fibrose épirétinienne, fibrose rétinienne, fibrose prémaculaire idiopathique, fibrose sous-rétinienne, fibrose sous-rétinienne associée à un décollement de rétine, fibrose sous-rétinienne associée à une dégénérescence maculaire, fibrose sous-rétinienne associée à une dégénérescence maculaire humide liée à l'âge, rétinopathie diabétique, glaucome, atrophie géographique, fibrose cornéenne, fibrose post-chirurgicale, fibrose post-chirurgicale de la capsule postérieure après chirurgie de la cataracte, fibrose post-chirurgicale de la vésicule après trabéculectomie pour glaucome, fibrose conjonctivale, fibrose sous-conjonctivale, fibrose pulmonaire, fibrose kystique, fibrose pulmonaire idiopathique, fibrose massive progressive, sclérodermie, bronchiolite oblitérante, syndrome de Hermansky-Pudlak, amiantose, silicose, hypertension pulmonaire chronique, l'hypertension pulmonaire associée au SIDA, sarcoïdose, stroma tumoral lors d'une maladie pulmonaire, asthme, maladie hépatique chronique, cirrhose biliaire primitive, maladie hépatique schistosomale, cirrhose hépatique, cardiomyopathie hypertrophique, cardiomyopathie dilatée, fibrose de l'atrium, fibrillation auriculaire, fibrose du ventricule, fibrillation ventriculaire, fibrose myocardique, syndrome de Brugada, myocardite, fibrose endomyocardique, infarctus myocardique, maladie vasculaire fibrotique, cardiopathie hypertensive, cardiomyopathie arythmogène du ventricule droit, fibrose tubulo-interstitielle et glomérulaire, athérosclérose, varices, infarctus cérébraux, gliose, maladie d'Alzheimer, dystrophie musculaire, dystrophie musculaire de Duchenne, dystrophie musculaire de Becker, maladie de Crohn, colite microscopique, cholangite sclérosante primitive, sclérodermie, fibrose systémique néphrogénique, chéloïde cutanée, arthrofibrose, contracture de Dupuytren, fibrose médiastinale, fibrose rétropéritonéale, myélofibrose, maladie de La Peyronie, capsulite adhésive, fibrose rénale, syndrome néphritique, syndrome d'Alport, néphropathie associée au VIH, polykystose rénale, maladie de Fabry, néphropathie diabétique, glomérulonéphrite chronique, néphrite associée au lupus systémique, sclérose systémique progressive, maladie chronique de rejet de l'hôte, arthrite, maladie pré-néoplasique fibrotique, maladie néoplasique fibrotique, fibrose induite par agression chimique ou environnementale.

4. Combinaison pour utilisation selon l'une quelconque des revendications 1 à 3, dans laquelle la fibrose est une fibrose d'une maladie ou affection sélectionnée parmi une ophtalmopathie de Grave-Basedow, fibrose épirétinienne, fibrose rétinienne, fibrose prémaculaire idiopathique, fibrose sous-rétinienne, fibrose sous-rétinienne associée à un décollement de rétine, fibrose sous-rétinienne associée à une dégénérescence maculaire, fibrose sous-rétinienne associée à une dégénérescence maculaire humide liée à l'âge, rétinopathie diabétique, glaucome, atrophie géographique, fibrose cornéenne, fibrose post-chirurgicale, fibrose post-chirurgicale de la capsule postérieure après chirurgie de la cataracte, fibrose post-chirurgicale de la vésicule après trabéculectomie pour glaucome, fibrose conjonctivale, ou fibrose sous-conjonctivale.

5. Combinaison pour utilisation selon la revendication 1 ou la revendication 2, dans laquelle la fibrose est située :
dans le foie et est associée à une maladie hépatique chronique ou à une cirrhose hépatique ;
dans le rein et est associée à une maladie rénale chronique ; ou
dans le cœur et est associée à un dysfonctionnement de la musculature ou de propriétés électriques du cœur, ou à un épaississement des parois ou des valves du cœur.

6. Combinaison pour utilisation selon l'une quelconque des revendications 1 à 5, dans laquelle l'antagoniste d'un facteur angiogénique est l'aflibercept.

7. Combinaison comprenant un antagoniste de signalisation médiée par IL-11 et un antagoniste d'un facteur angiogénique ;
dans laquelle l'antagoniste de signalisation médiée par IL-11 est un anticorps anti-IL-11 ou un fragment de liaison à l'antigène de celui-ci, ou un anticorps anti-IL-Rα ou un fragment de liaison à l'antigène de celui-ci ; et
dans laquelle l'antagoniste d'un facteur angiogénique est un récepteur leurre de VEGF.

8. Composition pharmaceutique comprenant un antagoniste de signalisation médiée par IL-11 et un antagoniste d'un facteur angiogénique ;
dans laquelle l'antagoniste de signalisation médiée par IL-11 est un anticorps anti-IL-11 ou un fragment de liaison à l'antigène de celui-ci, ou un anticorps anti-IL-Rα ou un fragment de liaison à l'antigène de celui-ci ; et
dans laquelle l'antagoniste d'un facteur angiogénique est un récepteur leurre de VEGF.

9. Combinaison selon la revendication 7, ou composition pharmaceutique selon la revendication 8, dans laquelle l'antagoniste d'un facteur angiogénique est l'aflibercept.
